# EUROPEAN PATENT APPLICATION

(11) **EP 1 712 235 A2**
(43) Date of publication of application: **18.10.2006**
(21) Application number: 06251154.8
(22) Date of filing: 03.03.2006
(51) Int. Cl.: A61K 31/5513, A61P 35/00

(54) **Combinational therapy involving a small molecule inhibitor of the MDM2: P53 interaction**

(30) Priority: 04.03.2005 US 72391
(71) Applicant: Ortho-McNeil Pharmaceutical, Inc., Raritan, NJ 08869 (US)
(72) Inventor: Lu, Tianbao, Churchville, PA 18966 (US); Koblish, Holly K., Exton, PA 19341 (US); Maroney, Anna, Media, PA 19063 (US)
(74) Representative: Mercer, Christopher Paul

(57) **Abstract**

The present invention is directed to a combinational therapy for treating cancer or other cell proliferative diseases. Such a therapy combines the use of radiation therapy or chemotherapy with the use of a small molecule inhibitor of the MDM2: p53 interaction.

### Annex to the application documents - subsequently filed sequences listing

SEQUENCE LISTING

<110> ORTHO-MCNEIL PHARMACEUTICAL, INC.
LU, Tianbao
KOBUSH, Holly K.
MARONEY, Anna

<120> COMBINATION THERAPY INVOLVING A SMALL MOLECULE INHIBITOR OF
THE MDM2:P53 INTERACTION

<130> P043193EP

<140> 06251154.8
<141> 2006-03-03

<150> US 11/072391
<151> 2005-03-04

<160> 3

<170> SeqWin99, version 1.02

<210> 1
<211> 12
<212> PRT
<213> Artificial Sequence

<220>
<223> p53 Analogue

<400> 1
Met Pro Arg Phe Met Asp Tyr Trp Glu Gly Leu Asn
1               5                   10

<210> 2
<211> 37
<212> DNA
<213> Artificial Sequence

<220>
<223> Primer

<400> 2
ctctctcgga tcccagattc cagcttcgga acaagag 37

<210> 3
<211> 41
<212> DNA
<213> Artificial Sequence

<220>
<223> Primer

<400> 3
tatatatctc gagtcagttc tcactcacag atgtacctga g 41

## Description

### CROSS REFERENCE TO RELATED APPLICATIONS

This application is a continuation in part of U.S. application Serial No. 10/829,040, filed on April 21, 2004, and a continuation in part of U.S. Application Serial No. 10/292,876 filed November 13, 2002.

### FIELD OF THE INVENTION

The present invention is in the area of treating cancers or other cell proliferative diseases. Particularly, the invention relates to a combinational therapy involving a small molecule inhibitor of the MDM2:p53 interaction.

HDM2 is the expression product of hdm2, an oncogene that is overexpressed in a variety of cancers, especially soft tissue sarcomas (Momand, J., et al., Nucl. Acids Res. 26:3453-3459 (1998)).

p53 is a transcription factor that plays a pivotal role in the regulation of the balance between cell proliferation and cell growth arrest/apoptosis. Under normal conditions, the half-life of p53 is very short, and consequently the level of p53 in cells is low. However, in response to cellular DNA damage, cellular stress, or other factors, levels of p53 increase. This increase in p53 levels in turn increases the transcription of a number of genes which induces the cell to either arrest growth or undergo apoptosis (i.e., controlled cell death). The function of p53 is to prevent the uncontrolled proliferation of cells and thus protect the organism from the development of cancer (for a review, see Levine, A.J., Cell 88:323-331 (1997)).

p53 is a latent and short-lived transcription factor which is induced by, and is an integration point for, a range of cellular stresses including DNA damage, UV damage, spindle damage, hypoxia, inflammatory cytokines, viral infection, activated oncogenes, and ribonucleotide depletion. Activation of p53 mediates a change in the balance of gene expression such that expression of many genes involved in proliferation is repressed while a range of genes involved in growth arrest (such as p21 WAF1 and GADD45), repair (such as p53RE) and apoptosis (such as Bax, Killer/DR5 and PIGs) is activated. The biological outcome of p53 activation (whether permanent or transient growth arrest or apoptosis) is dependent on several factors including the type and strength of the inducing stress, and the type of cell or tissue.

p53 and MDM2 exist in a negative regulatory feedback loop in which p53 stimulates transcription of the mdm2 gene while MDM2 binds to p53 and targets it for degradation by the 26S proteasome. The key element in the p53 induction process is disruption of the p53-MDM2 complex which permits p53 to accumulate in the nucleus. This mechanism appears to be common to all of the pathways by which p53 becomes activated, although recent evidence has indicated that there is considerable variation in the molecular events by which this is actually achieved.

Inactivation of the p53 tumor suppressor is a frequent event in human neoplasia. The inactivation can occur by mutation of the p53 gene or through binding to viral or cellular oncogene proteins, such as the SV40 large T antigen and MDM2. While the mechanism through which wild-type p53 suppresses tumor cell growth is as yet poorly defined, it is clear that one key feature of the growth suppression is the property of p53 to act as a transcription factor (Farmer, G., et al., Nature 358:83-86 (1992); Funk, W. D. et al., Mol. Cell. Biol. 12: 2866- 2871 (1992); Kern, S. E., et al., Science 256:827-830 (1992)). Currently, considerable effort is being made to identify growth control genes that are regulated by p53 binding to sequence elements near or within these genes. A number of such genes have been identified. In cases such as the muscle creatine kinase gene (Weintraub, H., et al., Proc. Natl. Acad. Sci. U.S.A., 88:4570-4571 (1991); Zambetti, G. P., et al., Genes Dev. 6:1143-1152 (1992)) and a GLN retroviral element (Zauberman, A., et al., EMBO J. 12:2799-2808 (1993)), the role these genes might play in the suppression of growth control is unclear. Yet there are other examples, namely mdm2 (Barak, Y., et al. EMBO J. 12:461-468 (1993); Wu, X., et al., Genes Dev. 7:1126-1132(1993)) GADD 45 (Kastan, M. B., et al., Cell 71:587-597(1992)) and WAF1 or CIP1 (El-Beiry, W. S., et al., Cell 75:817-825 (1993); Harper, J. W., et al., Cell 75:805-816 (1993)), where their involvement in the regulation of cell growth is better understood.

mdm2, a known oncogene, was originally found on mouse double minute chromosomes (Cahilly-Snyder., L., et al., Somatic Cell Mol. Genet. 13:235-244 (1987)). Its protein product was subsequently found to form a complex with p53, which was first observed in a rat fibroblast cell line (Clone 6) previously transfected with a temperature sensitive mouse p53 gene (Michalovitz, D., et al., Cell 62:671-680 (1990)). The rat cell line grew well at 37°C but exhibited a G1 arrest when shifted down to 32°C, which was entirely consistent with an observed temperature dependent switch in p53 conformation and activity. However, the p53-MDM2 complex was only observed in abundance at 32°C, at which temperature p53 was predominantly in a functional or "wild-type" form (Barak, Y. et al., EMBO J. 11:2115-2121 (1992) and Momand, J., et al., Cell 69:1237-1245 (1992)). By shifting the rat cell line down to 32°C and blocking de novo protein synthesis it was shown that only "wild-type" p53 induced expression of the mdm2 gene, thereby accounting for the differential abundance of the complex in terms of p53 transcriptional activity (Barak, Y., et al., EMBO J. 12:461-468 (1993)). The explanation was further developed by the identification of a DNA binding site for wild-type p53 within the first intron of the mdm2 gene (Wu, X., et al., Genes Dev. 7:1126-1132 (1993)). Reporter constructs employing this p53 DNA binding site revealed that they were inactivated when wild-type p53 was co-expressed with MDM2.

This inhibition of the transcriptional activity of p53 may be caused by MDM2 blocking the activation domain of p53 and/or the DNA binding site. Consequently, it was proposed that mdm2 expression is autoregulated, via the inhibitory effect of MDM2 protein on the transcriptional activity of wild-type p53. This p53-mdm2 autoregulatory feedback loop provided a novel insight as to how cell growth might be regulated by p53. Up to a third of human sarcomas are considered to overcome p53-regulated growth control by amplification of the hdm2 gene (the human homologue of mdm2) (Oliner, J.D., et al., Nature 358:80-83 (1992)). Hence, the interaction between p53 and HDM2 represents a key potential therapeutic target. One mechanism by which MDM2 can promote tumorogenesis is by its inhibitory action on p53. The tumor suppressor functions of p53 control a pivotal checkpoint in the control of cell cycling (reviewed in Levine, A.J., Cell 88:323- 331 (1997)). p53 is a transcription factor for a number of proteins that cause cell cycle arrest or cell death by apoptosis. The level and transcriptional activity of p53 are increased by damage to cellular DNA. The MDM2 protein inhibits p53 function by binding to an amphipathic N-terminal helix of p53, abrogating the interaction of p53 with other proteins and its transactivation activity. The interaction with MDM2 also targets p53 for ubiquitin dependent protein degradation. MDM2 exhibits p53 independent effects on cell cycling as well, possibly by direct interaction with some of the downstream effectors such as pRB and EF2 (Reviewed in Zhang, R. and Wang, H., Cur. Pharm. Des. 6:393-416 (2000)).

Blocking HDM2 from binding p53 would be therapeutically useful in restoring cell cycle control to cells that overexpress HDM2 as a front line cancer treatment. More generally, inhibition of HDM2 may increase the effectiveness of chemotherapy and radiation in p53 normal cancers by enhancing apoptosis and growth arrest signaling pathways.

A need continues to exist for developing new therapeutic strategies of using small molecules that inhibit the interactions between HDM2 and p53.

### BACKGROUND OF THE INVENTION

### RELATED ART

Combinational use of chemotherapeutics or radiation with a p53 activating agent may result in enhanced antitumor activity. For example, clinical trials indicated that patients with advanced cancers responded well to combined treatment using chemotherapeutics and irradiation with antineoplaston A10 and AS2-1 (Tsuda et al., 2002, *Oncology Reports,* 9(1): 65-68). Antineoplastons regulate expression of genes p53 and p21 through demethylation of promoter sequences and acetylation of histones (Burzynski, 2004, *Integr Cancer Ther.,* 3(1):47-58). It was suggested that the combination of p53 gene therapy and chemotherapy may increase the therapeutic efficacy in the treatment of patients with soft tissue sarcomas (STS) (Zhan et al., 2001, *Cancer,* 92(6): 1556-1566). In addition, chemosensitization and radiosensitization was detected when the antisense anti-HDM2 oligonucleotides were used in combination with cancer chemotherapeutics and radiation therapy (Wang et al., 2003, *Annals of the New York Academy of Sciences,* 1002 (Therapeutic Oligonucleotides): 217-235).

Although the effectiveness of the combined therapy has been demonstrated with various gene therapies that either increase gene expression of p53 or decrease gene expression of mdm2, it remains uncertain whether a small molecule inhibitor of the HDM2: p53 interaction is equally effective in such a combination therapy.

### SUMMARY OF THE INVENTION

The present invention is directed to combination therapy employing a first form of chemotherapy with a small molecule that inhibits the interactions between HDM2 and p53, so that the antitumor activity of the first form of chemotherapy is enhanced.

A general aspect of the invention is a method of inhibiting the growth of a cell, comprising the steps of: a) administering to the cell an antineoplastic agent; and b) administering to the cell a small molecule that inhibits the binding between proteins MDM2 and p53.

Another general aspect of the invention is a method of inhibiting the growth of a cell, comprising the steps of: a) exposing the cell to a radiation treatment; and b) administering to the cell a small molecule that inhibits the binding between proteins MDM2 and p53.

In one embodiment, the antineoplastic agent is doxorubicin.

In another embodiment, the compound that inhibits the binding between proteins MDM2 and p53 is a Benzodiazapinedione compound that has been described in W02003041715 or WO2004096134. Descriptions in both W02003041715 and WO2004096134 are herein incorporated by reference.

The present invention further provides a method of monitoring the effect of a small molecule that inhibits the binding between proteins MDM2 and p53, comprising the steps of: a) obtaining a biological sample from a subjected who has been administered the small molecule that inhibits the binding between proteins MDM2 and p53; and b) measuring the level of gene expression of a gene whose transcription is regulated by p53.

In one embodiment, the gene whose transcription is regulated by p53 is the p21 ^{waf1/cip1} gene.

### BRIEF DESCRIPTION OF THE FIGURES

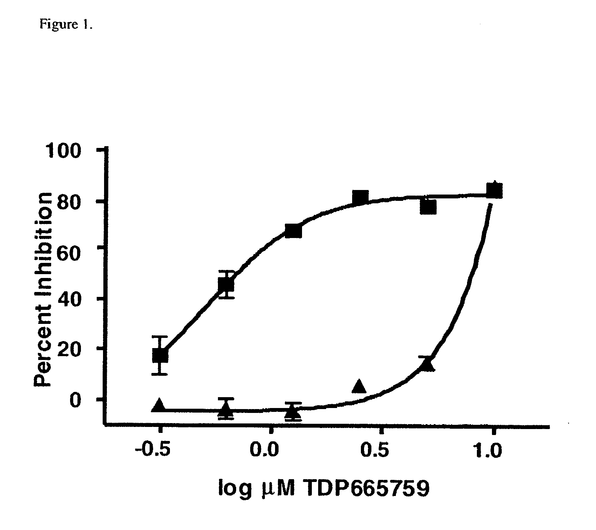

Figure 1. TDP665759 suppresses cell proliferation in a wild-type p53-dependent fashion. Mammary carcinoma cells, MCF7 (wt p53) and MDA-MB-231 (mutant p53), were allowed to adhere overnight before being treated with TDP665759 for 72 hours. The amount of BrdU incorporation was measured. Results were expressed as percent inhibition relative to cells treated with DMSO vehicle control. Shown here is a representative experiment of 4 independent studies where the error bars represent the standard deviation of triplicate samples. MCF7: square, IC₅₀ = 0.5 µM; MDA-MB-231: triangle, IC₅₀ > 5 µM.

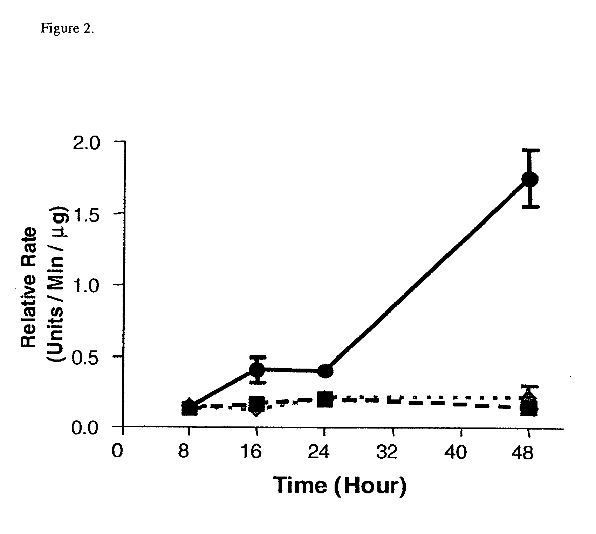

Figure 2. TDP521252 induces caspase-3 and -7 activity in HepG2 cells. HepG2 cells were treated with TDP521252, TDP536356 or vehicle control for 8-48 hours as indicated. At the end of the time points, cells were lysed, caspase substrate was added and activity was measured every 20 minutes for 2 hours. Specific activity is reported as the rate of activity per minute per µg of total protein for the time frame between 20 and 40 minutes post substrate addition. TDP521252: circle; TDP536356: square; and DMSO: diamond.

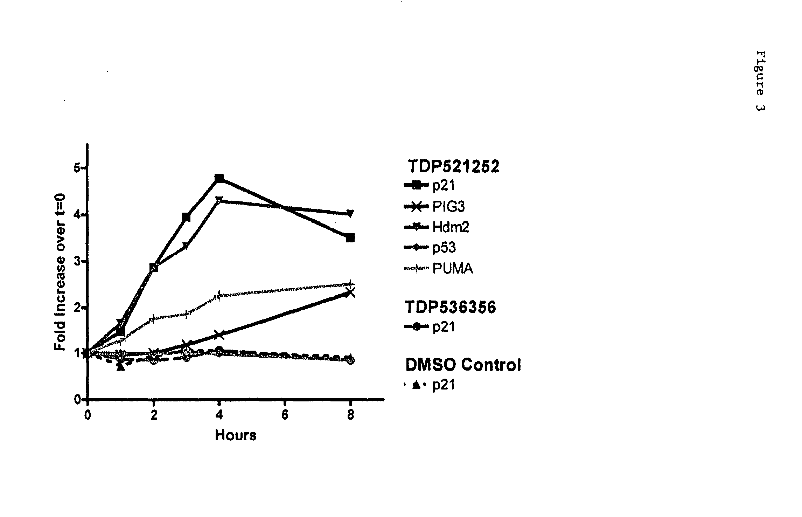

Figure 3. TDP521252 regulates the p53 pathway. HepG2 hepatocellular carcinoma cells were subjected to TDP52152 or TDP536356 for 1, 2, 4 or 8 hours and total cellular RNA was harvested. Quantitative RT-PCR was performed to determine the upregulation of the p53 target genes indicated in the graph. Results were normalized to GAPDH and are expressed as fold increase relative to time zero. Results shown are representative of two separate experiments. TDP521252: p21 (filled circle); PIG3 (filled square); Hdm2 (filled triangle); p53 (asteroid); PUMA (filled diamond). TDP536356: p21 (open circle). DMSO: p21 (open triangle).

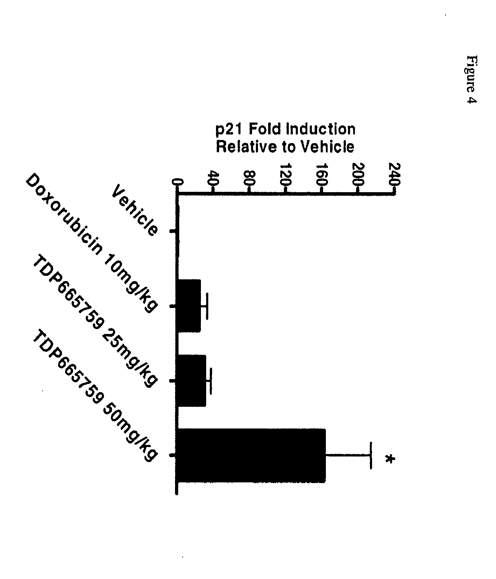

Figure 4. TDP665759 induces p21^{waf1/cip1} *in vivo.* Nude mice were injected with 25 or 50 mg/kg of TDP665759 intraperitoneally b.i.d. for 4 days or with 10 mg/kg doxorubicin once intravenously on day 3. Six hours after the first dose on day four, mice were sacrificed and livers were harvested for total RNA extraction. Quantitative PCR was performed to determine the relative ratios of p21^{wafl/cipl} in liver tissue. Data are presented as the mean ± S.D. of three individual animals per group. * indicates statistical difference (p<0.05) from vehicle control as determined by ANOVA and differences between treatments were determined by post hoc Newman-Keul's test.

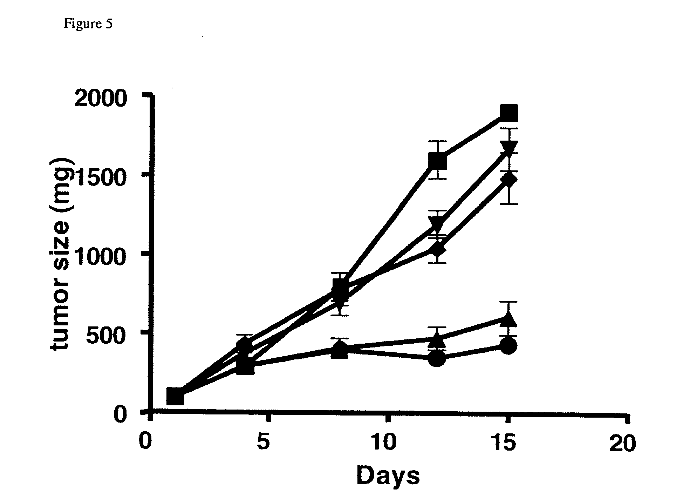

Figure 5. TDP665759 synergizes with doxorubicin to suppress A375 tumor growth. A375 melanoma xenografts were established in female nude mice and allowed to reach 80-120 mg in size. At that time, mice were randomized into treatment groups and treatment with vehicle (20.25% hydroxypropyl-β-cyclodextran, bid x 10), 100 mg/kg TDP665759 (bid x 10), 1.5 or 3 mg/kg doxorubicin (Qd x 5) or a combination of 100 mg/kg TDP665759 (bid x 10) + 1.5 mg/kg doxorubicin (Qd x 5) was initiated. Mice were monitored daily for general health and tumor size was measured every third or fourth day. Mice were euthanized when tumor volume exceeded 2 grams. Data presented here are the mean ± S.D. of the tumor mass. Vehicle: square; upward triangle: 3 mg/kg Doxorubicin; downward triangle: 1.5 mg/kg Doxorubicin; diamond: 100 mg/kg TDP665759; circle: 100 mg/kg TDP665759 + 1.5 mg/kg Doxorubicin.

### DETAILED DESCRIPTION OF THE PREFERRED EMBODIMENTS

The present invention is directed to the use of a novel class of small molecules that bind to HDM2 and/or MDM2 in combination therapy. By interfering with HDM2-p53 or MDM2-p53 interactions, these compounds increase the intracellular concentration of p53. These small molecules, therefore, have therapeutic utility in sensitizing tumor cells for chemotherapy. In tumor types particularly sensitive to an increase in functional p53, compounds of this type will be sufficient to induce apoptosis. Compounds of the present invention are also useful in treating tumor types in which HDM2 or MDM2 is overexpressed.

Compounds of the present invention include compounds of Formula ***I***: 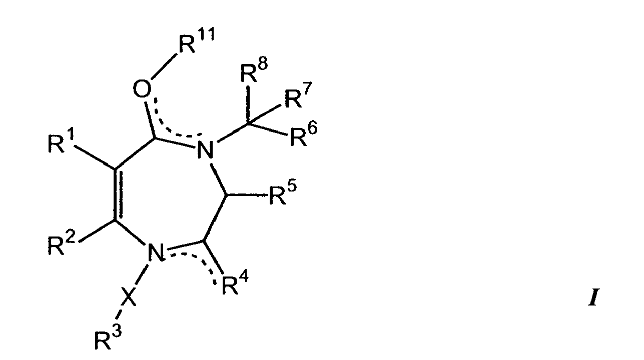 or a solvate, hydrate or pharmaceutically acceptable salt thereof,
wherein:
R¹ and R² are independently hydrogen, alkyl, alkenyl, alkynyl, optionally-substituted cycloalkyl, optionally-substituted cycloalkylalkyl, optionally-substituted aryl, optionally-substituted aralkyl, optionally-substituted heteroaryl, optionally-substituted heteroaralkyl, alkoxy, optionally-substituted aryloxy, optionally-substituted heteroaryloxy, cyano, amino, alkanoylamino, nitro, hydroxy, carboxy, or alkoxycarbonyl; or R¹ and R², together with the two carbon atoms to which they are attached, form a 5- to 10-membered non-aromatic or a 6- to 10-membered aromatic carbocyclic ring, or a 5- to 10-membered aromatic or non-aromatic ring wherein 1 or 2 of the ring atoms are heteroatoms, wherein each of the rings is optionally substituted 1-4 times with R^{a}, wherein each occurrence of R^{a} is independently hydrogen, halo, optionally-substituted alkyl, optionally-substituted alkenyl, optionally-substituted alkynyl, optionally-substituted cycloalkyl, optionally-substituted aryl, optionally-substituted aralkyl, optionally-substituted heteroaryl, optionally-substituted heteroaralkyl, alkoxy, optionally-substituted aryloxy, optionally-substituted heteroaryloxy, cyano, optionally-substituted amino, carbamoyl, alkylaminocarbonyl, dialkylaminocarbonyl, alkanoylamino, nitro, hydroxy, carboxy, alkoxycarbonyl or acyl, optionally-substituted alkyl thiol, optionally-substituted trifluorothio, optionally-substituted hydroxy imine alkyl;
X is a bivalent radical of: an optionally-substituted alkane, a optionally-substituted cycloalkane, an optionally-substituted arene, an optionally-substituted heteroarene, an optionally-substituted arylalkane or an optionally-substituted heteroarylalkane, optionally-substituted amine; and
R³ is -CO₂R^{d}, -CO₂M, -OH, -OR^{d}, -NHR^{d}, -NR^{d}R^{d}, -SO₂R^{d}, -NHCONHR^{d}, -NHCONR^{d}R^{d}, -CH₂R^{d}, optionally-substituted amidino or optionally-substituted guanidino, where R^{d} is hydrogen, alkyl, optionally-substituted alkoxy, optionally-substituted cycloalkyl or optionally-substituted, saturated or partially unsaturated heterocycle, and M is a cation or H;
or R³-X- is hydrogen or an electron pair;
R⁴ is oxygen, two hydrogen groups, or -NR⁹R¹⁰ where R⁹ and R¹⁰ are independently hydrogen, optionally-substituted alkyl, cycloalkyl, optionally-substituted aryl, optionally-substituted heteroaryl, optionally-substituted aralkyl, optionally-substituted heteroaralkyl, carboxyalkyl, alkoxycarbonyl, alkoxycarbonylalkyl, aminocarbonyl, aminocarbonylalkyl, alkylaminocarbonyl or alkylaminocarbonylalkyl, alkoxy;
or R³ and R⁴, together with the two carbon atoms to which they are attached, form a 5- to 10-membered non-aromatic or a 6- to 10-membered aromatic carbocyclic ring, or a 5- to 10-membered aromatic or non-aromatic ring wherein 1 or 2 of the ring atoms are heteroatoms, wherein each of the rings is optionally substituted 1-4 times with R^{a}, where R^{a} is defined as above;
R⁵ is cycloalkyl, aryl, heteroaryl, cycloalkylalkyl, aralkyl, heteroarylalkyl, or a saturated or partially unsaturated heterocycle, each of which is optionally substituted;
R⁶, R⁷ and R⁸ are independently hydrogen, alkyl, cycloalkyl, aryl, heteroaryl, a saturated or partially unsaturated heterocycle, cycloalkylalkyl, aralkyl or heteroarylalkyl, each of which is optionally substituted; or R⁶ and R⁷, together with the carbon atom to which they are attached form a 3- to 7-membered carbocyclic ring optionally substituted 1 to 3 times with R^{a}, where R^{a} is defined as above;
R¹¹ is alkyl, cycloalkyl, aryl, heteroaryl, a saturated or partially unsaturated heterocycle, cycloalkylalkyl, aralkyl or heteroarylalkyl, each of which is optionally substituted.

Useful values of R¹ and R² include hydrogen, C₁₋₆ alkyl, C₂₋₆ alkenyl, C₂₋₆ alkynyl, optionally-substituted C₃₋₈ cycloalkyl, optionally-substituted C₃₋₈ cycloalkyl(C₁₋₄) alkyl, optionally-substituted phenyl, optionally-substituted naphthyl, optionally-substituted phenyl(C₁₋₆) alkyl, optionally-substituted naphthyl(C₁₋₆) alkyl, optionally-substituted heteroaryl having 5 to 7 ring atoms of which 1 or 2 are heteroatoms, optionally-substituted heteroaryl(C₁₋₆) alkyl in which the heteroaryl portion has 5 to 7 ring atoms of which 1 or 2 are heteroatoms, C₁₋₆ alkoxy, optionally-substituted phenoxy, optionally-substituted benzyloxy, optionally-substituted naphthoxy, optionally-substituted heteroaryloxy in which the heteroaryl portion has 5 to 7 ring atoms of which 1 or 2 are heteroatoms, cyano, amino, (C₁₋₆ alkanoyl)amino, nitro, hydroxy, carboxy or (C₁₋₆ alkoxy)carbonyl. Particularly useful values of R¹ and R² include hydrogen, optionally-substituted phenyl, optionally-substituted benzyl, optionally-substituted cyclopentyl, optionally-substituted cyclohexyl, optionally-substituted C₃₋₈ cycloalkyl(C₁₋₄) alkyl and C₁₋₆ alkyl.

Also useful is when R¹ and R², together with the two carbon atoms to which they are attached, form an optionally-substituted 5- to 7-membered ring, aromatic or non-aromatic, wherein 0, 1 or 2 of the ring atoms are heteroatoms. Particularly useful rings formed in this fashion include optionally-substituted benzene and optionally-substituted pyridine.

When R¹ and R², together with the two carbon atoms to which they are attached, form a ring, the ring is optionally substituted one or more times, preferably 1 or 2 times, with R^{a}.

Useful values of R^{a} include hydrogen, halo, C₁₋₆ alkyl, C₂₋₆ alkenyl, C₂₋₆ alkynyl, C₃₋₈ cycloalkyl, optionally-substituted C₆₋₁₀ aryl, optionally-substituted (C₆₋₁₀ aryl)C₁₋₆ alkyl, optionally-substituted thio, optionally-substituted 5- to 7-membered heteroaryl, optionally-substituted heteroaryl(C₁₋₆) alkyl in which the heteroaryl portion has 5 to 7 ring atoms of which 1 or 2 are heteroatoms, C₁₋₆ alkoxy, optionally-substituted (C₆₋₁₀ aryl)oxy, optionally-substituted heteroaryloxy in which the heteroaryl portion has 5 to 7 ring atoms of which 1 or 2 are heteroatoms, cyano, amino, carbamoyl, (C₁₋₆ alkyl)aminocarbonyl, di-(C₁₋₆ alkyl)aminocarbonyl, (C₁₋₆ alkanoyl)amino, nitro, hydroxy, carboxy, (C₁₋₆ alkoxy)carbonyl and C₁₋₆ acyl.

Particularly useful values of X include a bivalent radical of: a C₁₋₆ alkane, an optionally-substituted C₆₋₁₀ arene, an optionally-substituted 5- to 7-membered heteroarene wherein 1 or 2 ring atoms are heteroatoms, an optionally-substituted (C₆₋₁₀ aryl)C₁₋₆ alkane, and an optionally-substituted heteroaryl(C₁₋₆) alkane in which the heteroaryl portion contains 5 to 7 ring atoms and wherein 1 or 2 of the ring atoms are heteroatoms. Preferred values of X include a bivalent radical of: a C₁₋₆ alkane, optionally-substituted benzene, optionally-substituted furan, optionally-substituted thiophene, optionally-substituted pyrrole and optionally-substituted pyridine. More preferred X include a bivalent radical of: methane, ethane, n-propane, n-butane, n-pentane, n-hexane, benzene and furan. When X is a bivalent radical of an alkane: preferably the alkane has at least 3 carbon atoms; preferably the alkane is unbranched; and preferably X is an α,ω-bivalent radical.

Particularly useful values of R³ include -CO₂R^{d} and -CO₂M, where R^{d} is hydrogen, C₁₋₆ alkyl or optionally-substituted C₃₋₈ cycloalkyl, and M is a cation. Preferred values of R³ include -CO₂R^{d} and -CO₂M, where R^{d} is hydrogen or C₁₋₆ alkyl, and M is a cation. More preferred R³ includes -CO₂R^{d}, where R^{d} is hydrogen or C₁₋₄ alkyl. Most preferred is -COOH.

Particularly useful values of R⁵ include C₃₋₈ cycloalkyl, C₆₋₁₀ aryl, 5- to 7-membered heteroaryl wherein I or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, and 5- to 7-membered saturated or partially unsaturated heterocycle wherein 1 or 2 of the ring atoms are heteroatoms, in which each of the preceding groups is optionally substituted. A preferred value of R⁵ is optionally-substituted phenyl. When R⁵ is phenyl it is substituted preferably once in the 4-position or twice in the 3- and 4-positions, and preferably by halo, trifluoromethyl, trifluoromethoxy, nitro and/or amino. More preferred R⁵ include 4-chlorophenyl, 4-trifluoromethylphenyl, 4-trifluoromethoxyphenyl, 4-chloro-3-nitrophenyl, 3-amino-4-chlorophenyl and 3-bromophenyl.

Particularly useful values of R⁶ include C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, and 5- to 7-membered saturated or partially unsaturated heterocycle wherein 1 or 2 of the ring atoms are heteroatoms, in which each of the preceding groups is optionally substituted. Preferred values of R⁶ include optionally-substituted phenyl, optionally-substituted benzyl, optionally-substituted pyridyl and optionally-substituted naphthyl. More preferred is optionally-substituted phenyl. When R⁶ is substituted phenyl or substituted benzyl it is substituted preferably once in the *p*-position or twice in the m- and *p*-positions, or twice in the o- and *p*-position, and preferably by halo, nitro and/or amino. More preferred R⁶ include phenyl, 4-chlorophenyl, 3,4-dichlorophenyl, 2-amino-4-chlorophenyl, 2-amino-4-chloro-5-fluorophenyl, 3-amino-4-chlorophenyl and 4-chloro-3-nitrophenyl. Preferred R⁶ also include *p*-chlorobenzyl and 4-methyl-1-naphthyl.

Particularly useful values of R⁷ include hydrogen, C₁₋₆ alkyl, C₃₋₈ cycloalkyl and (C₃₋₈ cycloalkyl)alkyl. Preferred values of R⁷ include hydrogen, C₁₋₆ alkyl and C₃₋₆ cycloalkyl. More preferred R⁷ include hydrogen, methyl and cyclopropyl.

Particularly useful values of R⁸ include hydrogen and C₁₋₆ alkyl. Preferred values of R⁸ include hydrogen, methyl and ethyl. More preferred is hydrogen.

Particularly useful values of R⁹ include hydrogen, C₁₋₆ alkyl, C₃₋₆ cycloalkyl, hydroxy(C₁₋₆) alkyl, amino(C₁₋₆) alkyl, carboxy(C₁₋₆) alkyl, (C₁₋₆ alkoxy)carbonyl, (C₁₋₆ alkoxy)carbonyl(C₁₋₆) alkyl, carbamoyl, carbamoyl(C₁₋₆) alkyl, (C₁₋₆ alkylamino)carbonyl and (C₁₋₆ alkylamino)carbonyl(C₁₋₆) alkyl. Preferred values of R⁹ include hydrogen, C₁₋₆ alkyl, hydroxy(C₁₋₆) alkyl, amino(C₁₋₆) alkyl and carbamoyl(C₁₋₆) alkyl. More preferred values of R⁹ include hydrogen, methyl, 2-hydroxyethyl, 3-hydroxypropyl, 2-aminoethyl, carbamoylmethyl and carbamoylethyl.

Particularly useful values of R¹⁰ include hydrogen and C₁₋₆ alkyl. Preferred values of R¹⁰ include hydrogen, methyl and ethyl. More preferred is hydrogen.

In one preferred embodiment, compounds of the present invention are compounds of Formula *II:* 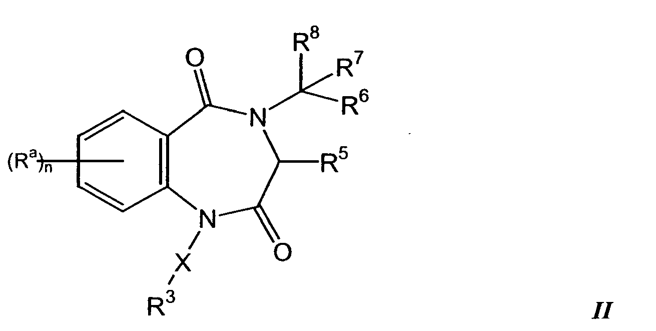 or a solvate, hydrate or pharmaceutically acceptable salt thereof,
wherein R^{a}, R³, X, R⁵, R⁶, R⁷ and R⁸ are defined as for compounds of Formula I, and n is 0, 1, 2, 3 or 4.

In this embodiment, particularly useful and preferred values of R³, X, R⁵, R⁶, R⁷ and R⁸ are as described for compounds of Formula ***I***.

In this embodiment, particularly useful values of R^{a} include halo, C₁₋₆ alkyl, C₂₋₆ alkenyl, C₂₋₆ alkynyl, cyano, C₃₋₈ cycloalkyl, hydroxy, C₁₋₆ alkoxy, carboxy, (C₁₋₆ alkoxy)carbonyl, C₁₋₆ acyl, carbamoyl, (C₁₋₆ alkyl)aminocarbonyl, amino and nitro. Preferred values of R^{a} include halo, C₂₋₆ alkynyl, carboxy, (C₁₋₆ alkoxy)carbonyl, C₁₋₆ acyl and carbamoyl. More preferred values of R^{a} include iodo, bromo, chloro, ethynyl, acetyl, methoxycarbonyl, carboxy and carbamoyl.

In this embodiment, particularly useful values of n include 0, 1 and 2. Preferred values of n include 1 and 2. More preferred is 1. When n is 1, R^{a} occurs preferably at the 7- or the 8-position, more preferably at the 7-position. When n is 2, R^{a} occurs preferably at the 7- and 8-positions.

A preferred group of compounds are compounds of Formula ***II***, wherein:
each instance of R^{a} is independently halo, C₁₋₆ alkyl, C₂₋₆ alkenyl, C₂₋₆ alkynyl, cyano, C₃₋₈ cycloalkyl, hydroxy, C₁₋₆ alkoxy, carboxy, (C₁₋₆ alkoxy)carbonyl, C₁₋₆ acyl, carbamoyl, (C₁₋₆ alkyl)aminocarbonyl, amino or nitro, alkylthio;
n is 0, 1 or 2; when n is 1, R^{a} occurs at the 7- or 8-position; when n is 2, R^{a} occurs at the 7- and 8-positions;
X is a bivalent radical of: a C₁₋₆ alkane, an optionally-substituted C₆₋₁₀ arene, an optionally-substituted 5- to 7-membered heteroarene wherein 1 or 2 ring atoms are heteroatoms, an optionally-substituted (C₆₋₁₀ aryl)C₁₋₆ alkane, or an optionally-substituted heteroaryl(C₁₋₆) alkane in which the heteroaryl portion contains 5 to 7 ring atoms and wherein 1 or 2 of the ring atoms are heteroatoms;
R³ is -CO₂R^{d} or -CO₂M, where R^{d} is hydrogen, C₁₋₆ alkyl or optionally-substituted C₃₋₈ cycloalkyl, and M is a cation;
R⁵ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein I or 2 of the ring atoms are heteroatoms, in which each of the preceding groups is optionally substituted;
R⁶ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁷ is hydrogen, C₁₋₆ alkyl, C₃₋₈ cycloalkyl or (C₃₋₈ cycloalkyl)alkyl; and
R⁸ is hydrogen or C₁₋₆ alkyl.

A more preferred group of compounds are compounds of Formula II, wherein:
each instance of R^{a} is independently halo, C₂₋₆ alkynyl, carboxy, (C₁₋₆ alkoxy)carbonyl, C₁₋₆ acyl or carbamoyl;
n is 1 or 2; when n is 1, R^{a} occurs at the 7-position; when n is 2, R^{a} occurs at the 7- and 8-positions;
X is a bivalent radical of a C₁₋₆ alkane, optionally-substituted benzene, optionally-substituted furan, optionally-substituted thiophene, optionally-substituted pyrrole or optionally-substituted pyridine;
R³ is -CO₂R^{d} or -CO₂M, where R^{d} is hydrogen or C₁₋₆ alkyl, and M is a cation;
R⁵ is optionally-substituted phenyl;
R⁶ is optionally-substituted phenyl, optionally-substituted benzyl, optionally-substituted pyridyl or optionally-substituted naphthyl;
R⁷ is hydrogen, C₁₋₆ alkyl or C₃₋₆ cycloalkyl; and
R⁸ is hydrogen.

In one preferred embodiment, compounds of the present invention are compounds of Formula ***III*:** 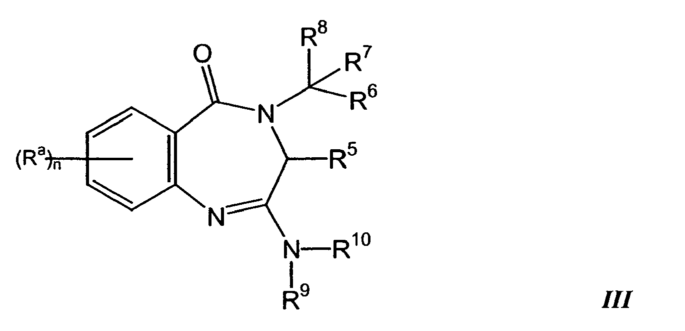 or a solvate, hydrate or pharmaceutically acceptable salt thereof,
wherein R^{a}, R⁵, R⁶, R⁷, R⁸, R⁹ and R¹⁰ are defined as for compounds of Formula I, and n is 0, 1, 2, 3 or 4.

Those skilled in the art will recognize that compounds of Formula *I* when R⁴ is -NR⁹R¹⁰ are amidines which exist in an equilibrium between tautomeric forms when at least one of R⁹ and R¹⁰ is hydrogen. While Formula III illustrates only one form, the invention is intended to encompass both tautomers.

In this embodiment, particularly useful and preferred values of R⁵, R⁶, R⁷, R⁸ , R⁹ and R¹⁰ are as described for compounds of Formula ***I***.

In this embodiment, particularly useful values of R^{a} include halo, C₁₋₆ alkyl, C₂₋₆ alkenyl, C₂₋₆ alkynyl, cyano, C₃₋₈ cycloalkyl, hydroxy, C₁₋₆ alkoxy, carboxy, (C₁₋₆ alkoxy)carbonyl, C₁₋₆ acyl, carbamoyl, (C₁₋₆ alkyl)aminocarbonyl, amino, alkylthio, and nitro. Preferred values of R^{a} include halo, C₂₋₆ alkynyl, carboxy, (C₁₋₆ alkoxy)carbonyl, C₁₋₆ acyl and carbamoyl. More preferred values of R^{a} include iodo, chloro, ethynyl, acetyl, methoxycarbonyl, carboxy and carbamoyl.

In this embodiment, particularly useful values of n include 0, 1 and 2. Preferred values of n include 1 and 2. More preferred is 1. When n is 1, R^{a} occurs preferably at the 7- or the 8-position, more preferably at the 7-position. When n is 2, R^{a} occurs preferably at the 7- and 8-positions.

A preferred group of compounds are compounds of Formula *III,* wherein:
each instance of R^{a} is independently halo, C₁₋₆ alkyl, C₂₋₆ alkenyl, C₂₋₆ alkynyl, cyano, C₃₋₈ cycloalkyl, hydroxy, C₁₋₆ alkoxy, carboxy, (C₁₋₆ alkoxy)carbonyl, C₁₋₆ acyl, carbamoyl, (C₁₋₆ alkyl)aminocarbonyl, amino, alkylthio, or nitro;
n is 0, 1 or 2; when n is 1, R^{a} occurs at the 7- or 8-position; when n is 2, R^{a} occurs at the 7- and 8-positions;
R⁵ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁶ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁷ is hydrogen, C₁₋₆ alkyl, C₃₋₈ cycloalkyl or (C₃₋₈ cycloalkyl)alkyl;
R⁸ is hydrogen or C₁₋₆ alkyl;
R⁹ is hydrogen, C₁₋₆ alkyl, C₃₋₆ cycloalkyl, hydroxy(C₁₋₆) alkyl, amino(C₁₋₆) alkyl, carboxy(C₁₋₆) alkyl, (C₁₋₆ alkoxy)carbonyl, (C₁₋₆ alkoxy)carbonyl(C₁₋₆) alkyl, carbamoyl, carbamoyl(C₁₋₆) alkyl, (C₁₋₆ alkylamino)carbonyl or (C₁₋₆ alkylamino)carbonyl(C₁₋₆) alkyl; and
R¹⁰ is hydrogen or C₁₋₆ alkyl.

A more preferred group of compounds are compounds of Formula III, wherein:
each instance of R^{a} is independently halo, C₂₋₆ alkynyl, carboxy, (C₁₋₆ alkoxy)carbonyl, C₁₋₆ acyl or carbamoyl;
n is 1 or 2; when n is 1, R^{a} occurs at the 7-position; when n is 2, R^{a} occurs at the 7- and 8-positions;
R⁵ is optionally-substituted phenyl;
R⁶ is optionally-substituted phenyl, optionally-substituted pyridyl, optionally-substituted benzyl or optionally-substituted naphthyl;
R⁷ is hydrogen, C₁₋₆ alkyl or C₃₋₆ cycloalkyl;
R⁸ is hydrogen;
R⁹ is hydrogen, C₁₋₆ alkyl, hydroxy(C₁₋₆) alkyl, amino(C₁₋₆) alkyl or carbamoyl(C₁₋₆) alkyl; and
R¹⁰ is hydrogen.

A second aspect of the present invention is directed to pharmaceutical compositions comprising
a) at least one compound of Formula I or a pharmaceutically acceptable salt thereof; and
b) one or more pharmaceutically-acceptable excipients.

Preferably, the pharmaceutical composition is sterile.

A third aspect of the present invention is directed to a method of inhibiting the binding of a protein encoded by mdm2 to p53 protein, comprising contacting p53 or one or more proteins encoded by mdm2 with one or more compounds of Formula *I*, wherein R¹-R⁸ and X are defined as above.

A fourth aspect of the invention is directed to a method of inducing apoptosis, comprising contacting an animal with a composition comprising a pharmaceutically effective amount of at least one compound of Formula *I*, or a salt thereof, wherein R¹-R⁸ and X are defined as above, and optionally one or more pharmaceutically-acceptable excipients.

A fifth aspect of the present invention is directed to a method of treating cancer, comprising contacting an animal with (a) a pharmaceutically effective amount of an antineoplastic agent, and (b) a pharmaceutically effective amount of at least one compound of Formula I, or a salt thereof, wherein R¹-R¹¹ and X are defined as above, and optionally one or more pharmaceutically-acceptable excipients, in combination with (a), (b), or (a) and (b).

A sixth aspect of the present invention is directed to a method of treating cancer, comprising contacting an animal with a composition comprising (a) a pharmaceutically effective amount of at least one compound of Formula ***I***, or a salt thereof, wherein R¹-R¹¹ and X are defined as above, (b) one or more agents that induce or cause DNA damage, and optionally (c) one or more pharmaceutically-acceptable excipients.

A seventh aspect of the present invention is directed to a method of making compounds of Formula *I*.

Compounds within the scope of the invention are described in the Examples. Examples of preferred compounds include: 5-1(3*S*)-3-(4-Chlorophenyl)-4-[(*R*)-1-(4-chlorophenyl)-ethyl]-7-iodo-2,5-dioxo-2,3,4,5-tetrahydro-benzo[e][1,4]diazepin-1-yl}-pentanoic acid; 5-{(3*S*)-3-(4-Chlorophenyl)-4-[(*R*)-1-(4-chlorophenyl)-ethyl]-7-ethynyl-2,5-dioxo-2,3,4,5-tetrahydro-benzo[e][1,4]diazepin-1-yl}-pentanoic acid; 5-[4-[(*R*)-1-(4-Chlorophenyl)-ethyl]-7-iodo-2,5-dioxo-(3*S*)-3-(4-trifluoromethyl-phenyl)-2,3,4,5-tetrahydro-benzo[e][1,4]diazepin-1-yl]-pentanoic acid; 5-[4-[(*R*)-1-(4-Chlorophenyl)-ethyl]-7-iodo-2,5-dioxo-(3*S*)-3-(4-trifluoromethoxy-phenyl)-2,3,4,5-tetrahydro-benzo[e][1,4]diazepin-1-yl]-pentanoic acid; 6-{(3*S*)-3-(4-Chlorophenyl)-4-[(R)-1-(4-chlorophenyl)-ethyl]-7-iodo-2,5-dioxo-2,3,4,5-tetrahydro-benzo[e][1,4]diazepin-1-yl}-hexanoic acid; 7-{(3*S*)-3-(4-Chlorophenyl)-4-[(*R*)-1-(4-chlorophenyl)-ethyl]-7-iodo-2,5-dioxo-2,3,4,5-tetrahydro-benzo[e][1,4]diazepin-1-yl}-heptanoic acid; 4-{(3*S*)-3-(4-Chlorophenyl)-4-[(*R*)-1-(4-chlorophenyl)-ethyl]-7-iodo-2,5-dioxo-2,3,4,5-tetrahydro-benzo[e][1,4]diazepin-1-ylmethyl}-benzoic acid; 4-{(3*S*)-3-(4-Chlorophenyl)-4-[(*R*)-1-(4-chlorophenyl)-ethyl]-7-iodo-2,5-dioxo-2,3,4,5-tetrahydro-benzo[e][1,4]diazepin-1-yl}-butyric acid; 3-{(3*S*)-3-(4-Chlorophenyl)-4-[(*R*)-1-(4-chlorophenyl)-ethyl]-7-iodo-2,5-dioxo-2,3,4,5-tetrahydro-benzo[e][1,4]diazepin-1-yl}-propionic acid; 5-{(3*S*)-3-(4-Chlorophenyl)-4-[(R)-1-(4-chlorophenyl)-ethyl]-7-iodo-2,5-dioxo-2,3,4,5-tetrahydro-benzo[e][1,4]diazepin-1-ylmethyl}-furan-2-carboxylic acid; 5-{(3*S*)-8-Chloro3-(4-chloro-phenyl)-4-[(R)-1-(4-chlorophenyl)-ethyl]-7-iodo-2,5-dioxo-2,3,4,5-tetrahydro-benzo[e][1,4]diazepin-1-yl}-pentanoic acid; 5-{3-(4-Chlorophenyl)-4-[(4-chlorophenyl)-cyclopropyl-methyl]-7-iodo-2,5-dioxo-2,3,4,5-tetrahydro-benzo[e][1,4]diazepin-1-yl}-pentanoic acid; 5-{3-(4-Chlorophenyl)-4-[1-(3,4-dichlorophenyl)-ethyl]-7-iodo-2,5-dioxo-2,3,4,5-tetrahydro-benzo[e][1,4]diazepin-1-yl}-pentanoic acid; 5-[4-[1-(3-Amino-4-chlorophenyl)-ethyl]-3-(4-chlorophenyl)-7-iodo-2,5-dioxo-2,3,4,5-tetrahydrobenzo[e][1,4]diazepin-1-yl]-pentanoic acid; 5-[4-[1-(4-Chloro-3-nitrophenyl)-ethyl]-3-(4-chlorophenyl)-7-iodo-2,5-dioxo-2,3,4,5-tetrahydrobenzo[e][1,4]diazepin-1-yl]-pentanoic acid; 5-{3-(4-Chlorophenyl)-7-iodo-4-[1-(4-methyl-naphthalen-1-yl)-ethyl]-2,5-dioxo-2,3,4,5-tetrahydrobenzo[e][1,4]diazepin-1-yl}-pentanoic acid; 5-[4-(4-Chlorobenzyl)-3-(4-chlorophenyl)-7-iodo-2,5-dioxo-2,3,4,5-tetrahydro-benzo[e] 1,4]diazepin-1-yl]-pentanoic acid; 5-{3-(4-Chlorophenyl)-4-[2-(4-chlorophenyl)-1-methylethyl]-7-iodo-2,5-dioxo-2,3,4,5-tetrahydro-benzo[e][1,4]diazepin-1-yl}-pentanoic acid; 5- ((3S)-3-(3-Bromo-phenyl)-4-[(*R*)-1 -(4-chloro-phenyl)-ethyl]-7-iodo-2,5-dioxo-2,3,4,5-tetrahydro-benzo[e] [,4]diazepin-1-yl}-pentanoic acid; [4-Benzyl-3-(4-chlorophenyl)-7-iodo-2,5-dioxo-2,3,4,5-tetrahydro-benzo[e][1,4]diazepin-1-yl]-acetic acid; 5-{(3*S*)-3-(4-Chloro-3-nitro-phenyl)-4-[(*R*)-1-(4-chlorophenyl)-ethyl]-7-iodo-2,5-dioxo-2,3,4,5-tetrahydro-benzo[e][1,4]diazepin-1-yl}-pentanoic acid; 5-{(3*S*)-7-Acetyl-3-(4-chlorophenyl)-4-[(*R*)-1-(4-chlorophenyl)-ethyl]-2,5-dioxo-2,3,4,5-tetrahydrobenzo[e][1,4]diazepin-1-yl}-pentanoic acid; 5-{(3*S*)-3-(3-Amino-4-chlorophenyl)-4-[(R)-1-(4-chloro-phenyl)-ethyl]-7-iodo-2,5-dioxo-2,3,4,5-tetrahydro-benzo[e][1,4]diazepin-1-yl}-pentanoic acid; (3*S*)-2-Amino-3-(4-chlorophenyl)-4-[(*R*)-1-(4-chlorophenyl)-ethyl]-7-iodo-3,4-dihydrobenzo[e] [1,4]diazepin-5-one; 2-{(3*S*)-3-(4-Chlorophenyl)-4-[(*R*)-1-(4-chlorophenyl)-ethyl]-7-iodo-5-oxo-4,5-dihydro-3*H*-benzo[e][1,4]diazepin-2-ylamino}-acetamide; (3*S*)-3-(4-Chloro-phenyl)-4-[(*R*)-1-(4-chloro-phenyl)-ethyl]-2-(2-hydroxy-ethylamino)-7-iodo-3,4-dihydro-benzo[e][1,4]diazepin-5one; (3*S*)-3-(4-Chloro-phenyl)-4-[(*R*)-1-(4-chloro-phenyl)-ethyl]-2-(3-hydroxy-propylamino)-7-iodo-3,4-dihydro-benzo[e][1,4]diazepin-5-one; N-(2-{(3*S*)-3-(4-Chloro-phenyl)-4-[(*R*)-1-(4-chloro-phenyl)-ethyl]-7-iodo-5-oxo-4,5-dihydro-3H-benzo[e][1,4]diazepin-2-ylamino}-ethyl)-acetamide; (3*S*)*-3-*(4-Chloro-phenyl)-4-[(*R*)-1-(4-chloro-phenyl)-ethyl]-7-iodo-2-methylamino-3,4-dihydro-benzo[e][1,4]diazepin-5-one; 2-(2-Amino-ethylamino)-(3*S*)-3-(4-chloro-phenyl)-4-[(R)-1-(4-chloro-phenyl)-ethyl]-7-iodo-3,4-dihydrobenzo[e][1,4]diazepin-5-one; 2-[*N*-(3-amino-3-oxopropyl)amino]-(3s)-3-(4-chlorophenyl)-4-[(*R*)-1-(4-chlorophenyl)ethyl]-7-iodo-3,4-dihydro-1,4-benzodiazepin-5-one; 5-[(3*S*)-3-(4-chlorophenyl)-4-[(*R*)-1-(4-chlorophenyl)ethyl]-7-methoxycarbonyl-2,5-dioxo-3,4-dihydro-1*H*-1,4-benzodiazepin-1-yl]valeric acid tert-butyl ester; (3*S*)-*1-(*4*-tert-*Butoxycarbonyl-butyl)-3-(4-chloro-phenyl)-4-[(R)-1-(4-chloro-phenyl)-ethyl]-2,5-dioxo-2,3,4,5-tetrahydro-1H-benzo[e][1,4]diazepine-7-carboxylic acid; 7-aminocarbonyl-5-[(3*S*)-3-(4-chlorophenyl)-4-[(*R*)-1-(4-chlorophenyl)ethyl]-2,5-dioxo-3,4-dihydro-1*H*-1,4-benzodiazepin-1-yl]valeric acid; 5-[4-(4-Chloro-2-methyl-benzyl)-3-(R,*S*)-(4-chloro-phenyl)-7-iodo-2,5-dioxo-2,3,4,5-tetrahydro-benzo[e][1,4]diazepin-1-yl]-valeric acid; Sodium; 5-{(3R)-3-(4-chloro-phenyl)-4-[(*R*)-1-(4-chloro-phenyl)-ethyl]-7-iodo-2,5-dioxo-2,3,4,5-tetrahydro-benzo[e][1,4]diazepin-1-yl}-valerate; 5-[(3*S*)-3-(4-Chlorophenyl)-4-[(*R*)-1-(4-chlorophenyl)-ethyl]-7-[(*R,S*)-1-hydroxyethyl]-2,5-dioxo-2,3,4,5-tetrahydro-benzo[e][1,4]diazepin-1-yl]-valeric acid; 5-[(*R,S*)-3-(4-Chlorophenyl)-4-[(*R*)-1-(4-chloro-phenyl)-ethyl]-7-(1-(*R,S*)-hydroxyethyl)-2,5-dioxo-2,3,4,5-tetrahydro-benzo[e][1,4]diazepin-1-yl]-valeric acid; 5-[(3*S*)-4-[(*R*)-1-(4-Chlorophenyl)ethyl]-7-iodo-2,5-dioxo-3-(4-trifluoromethyl-phenyl)-2,3,4,5-tetrahydro-benzo[e][1,4]diazepin-1-yl]-valeric acid; Sodium; 5-{(*S*)*-3-*(4-chloro-phenyl)-4-[(*R*)-1-(4-chloro-phenyl)-ethyl]-7-ethynyl-2,5-dioxo-2,3,4,5-tetrahydro-benzo[e][1,4]diazepin-1-yl}-valerate; 5-{(3*R*,*S*)-3-(4-Chlorophenyl)-4-[(*R*,*S*)-1-(4-chlorophenyl)-2-hydroxyethyl]-7-iodo-2,5-dioxo-2,3,4,5-tetrahydro-benzo[e][1,4]diazepin-1-yl}-valeric acid; Sodium; 5-{(3*S*)-3-(4-chlorophenyl)-4-[(*R*)-1-(4-chlorophenyl)ethyl]-7-iodo-2,5-dioxo-2,3,4,5-tetrahydro-benzo[e][1,4]diazepin-1-yl}-valerate; 5-[(3*R*,*S*)-4-(4-Chloro-2-methyl-benzyl)-3-(4-chloro-phenyl)-7-iodo-2,5-dioxo-2,3,4,5-tetrahydrobenzo[e][1,4]diazepin-1-yl]-valeric acid; Sodium; 5-{(3*R*)-3-(4-chlorophenyl)-4-[(*R*)-1-(4-chlorophenyl)ethyl]-7-iodo-2,5-dioxo-2,3,4,5-tetrahydrobenzo[e][1,4]diazepin-1-yl}-valerate; 5-[(3*S*)-3-(4-Chlorophenyl)-4-[(*R*)-1-(4-chlorophenyl)-ethyl]-7-(1-(*R,S*)-hydroxyethyl)-2,5-dioxo-2,3,4,5-tetrahydrobenzo[e][1,4]diazepin-1-yl]-valeric acid; 5-[(3R,S)-3-(4-Chlorophenyl)-4-[(R)-1-(4-chlorophenyl)ethyl]-7-[(*R,S*)-1-hydroxy-ethyl]-2,5-dioxo-2,3,4,5-tetrahydro-benzo[e][1,4]diazepin-1-yl]-valeric acid; 5-[(3*S*)-4-[(*R*)-1-(4-Chlorophenyl)-ethyl]-7-iodo-2,5-dioxo-3-(4-trifluoromethyl-phenyl)-2,3,4,5-tetrahydro-benzo[e][1,4]diazepin-1-yl]-valeric acid; Sodium; 5-{*(3S)*-3-(4-chlorophenyl)-4-[(*R*)-1-4-chlorophenyl)-ethyl]-7-ethynyl-2,5-dioxo-2,3,4,5-tetrahydro-benzo[e][1,4]diazepin-1-yl}-valerate; 5-{3-(4-Chlorophenyl)-4-[1-(4-chlorophenyl)-2-hydroxy-ethyl]-7-iodo-2,5-dioxo-2,3,4,5-tetrahydrobenzo[e][1,4]diazepin-1-yl}-valeric acid; Sodium; 5-{*(3S)*-3-(4-chlorophenyl)-4-[(*R*)-1-(4-chlorophenyl)-ethyl]-7-iodo-2,5-dioxo-2,3,4,5-tetrahydro-benzo[e][1,4]diazepin-1-yl}-valerate; 5-[(3*S*)-3-(4-Chlorophenyl)-4-[(*R*)-1-(4-chlorophenyl)ethyl]-7-(methylthio)-2,5-dioxo-1,4-benzodiazepin-1-yl]valeric acid; 5-[(3*R*)-3-(4-Chlorophenyl)-4-[(*R*)-1-(4-chlorophenyl)ethyl]-7-(methylthio)-2,5-dioxo-1,4-benzodiazepin-1-yl]valeric acid; 5-[(3*S*)-3-(4-Chlorophenyl)-4-[(*R*)-1-(4-chlorophenyl)ethyl]-7-(trifluoromethylthio)-2,5-dioxo-1,4-benzodiazepin-1-yl]valeric acid; 5-[(3*S*)-3-(2-Allyloxy-4-chlorophenyl)-4-[(*R*)-1-(4-chlorophenyl)ethyl]-7-iodo-2,5-dioxo-1,4-benzodiazepin-1-yl]valeric acid; 5-[(3*S*)-3-(4-chloro-2-hydroxyphenyl)-4-[(*R*)-1-(4-chlorophenyl)ethyl]-7-iodo-2,5-dioxo-1,4-benzodiazepin-1-yl]valeric acid; 5-[(3*S*)-3-(4-Chlorophenyl)-4-[(*R*)-1-(4-chlorophenyl)ethyl]-2,5-dioxo-7-phenyl-1,4-diazepin-1-yl]valeric acid sodium salt; 5-[(3*S*)-7-(2-Bromophenyl)-3-(4-chlorophenyl)-4-[(*R*)-1-(4-chlorophenyl)ethyl]-2,5-dioxo-1,4-diazepin-1-yl]valeric acid sodium salt; 5-[(3*S*)-3-(4-Chlorophenyl)-4-[(*R*)-1-(4-chlorophenyl)ethyl]-7-(2,5-dimethylphenyl)-2,5-dioxo-1,4-diazepin-1-yl]valeric acid sodium salt; 5-[(3*S*)-3-(4-Chlorophenyl)-4-[(*R*)-1-(4-chlorophenyl)ethyl]-7-(2-methylphenyl)-2,5-dioxo-1,4-benzodiazepine-1-yl]valeric acid sodium salt; 5-[4-[1-(2-Amino-4-chlorophenyl)ethyl]-3-(4-chlorophenyl)-7-iodo-2,5-dioxo-1,4-benzodiazoin-1-yl]valeric acid sodium salt; 5-[4-[(2-Amino-4-chlorobenzyl]-7-bromo-3-(4-chlorophenyl)-2,5-dioxo-1,4-benzodiazepin-1-yl]valeric acid sodium salt; 4-(4-Chloro-benzyl)-3-(4-chloro-phenyl)-7-iodo-3,4-dihydro-1H-benzo[e][1,4]diazepine-2,5-dione; 4-(R)-[1-(2-Amino-4-chloro-5-fluoro-phenyl)-ethyl]-3(S)-(4-chloro-phenyl)-7-iodo-1-(2-morpholin-4-yl-ethyl)-3,4-dihydro-1H-benzo[e][1,4]diazepine-2,5-dione; 4(R,S)-[1-(2-Amino-4-chloro-5-hydroxy-phenyl)-ethyl]-3(S,R)-(4-chloro-phenyl)-7-iodo-1-(2-morpholin-4-yl-ethyl)-3,4-dihydro-1H-benzo[e][1,4]diazepine-2,5-dione; 3(S)-(4-Chloro-2-hydroxy-phenyl)-4(R)-[1-(4-chloro-phenyl)-ethyl]-7-iodo-3,4-dihydro-1H-benzo[e][1,4]diazepine-2,5-dione; 4-[1-(2-Amino-4-chloro-phenyl)-ethyl]-3-(4-chloro-phenyl)-7-iodo-1-[3-(4-methyl-piperazin-1-yl)-propyl]-3,4-dihydro-1H-benzo[e][1,4]diazepine-2,5-dione; 4-(2-Amino-4-chloro-benzyl)-3-(4-chloro-phenyl)-1-(4-dimethylamino-butyl)-7-iodo-3,4-dihydro-1H-benzo[e][1,4]diazepine-2,5-dione; 4-(2-Amino-4-chloro-benzyl)-3-(4-chloro-phenyl)-7-iodo-1-(4-morpholin-4-yl-butyl)-3,4-dihydro-1H-benzo[e][1,4]diazepine-2,5-dione; 4-(2-Amino-4-chloro-benzyl)-3-(4-chloro-phenyl)-7-iodo-1-[4-(4-methyl-piperazin-1-yl)-butyl]-3,4-dihydro-1H-benzo[e][1,4]diazepine-2,5-dione; 4-(2-Amino-4-chloro-benzyl)-3-(4-chloro-phenyl)-1-(3-dimethylamino-propyl)-7-iodo-3,4-dihydro-1H-benzo[e][1,4]diazepine-2,5-dione; 4-(2-Amino-4-chlorobenzyl)-3-(4-chloro-phenyl)-7-iodo-1-(3-morpholin-4-yl-propyl)-3,4-dihydro-1H-benzo[e][1,4]diazepine-2,5-dione; 4-(4-Chloro-2-hydroxybenzyl)-3-(4-chlorophenyl)-7-iodo-1-[2-(2-methoxy-ethoxy)ethyl]-3,4-dihydro-1*H-*benzo[e][1,4]diazepine-2,5-dione; 4-[(*S*)-1-(2-Amino-4-chlorophenyl)ethyl]-(3*R*)-3-(4-chlorophenyl)-7-iodo-1-(2-morpholin-4-ylethyl)-3,4-dihydro-1*H-*benzo[e][1,4]diazepine-2,5-dione; 4-(2-Amino-4-chlorobenzyl)-3-(4-chlorophenyl)-7-iodo-1-[3-(4-methyl-piperazin-1-yl)propyl]-3,4-dihydro-1*H-*benzo[e][1,4]diazepine-2,5-dione; 5-(2-Allyloxy-4-chlorobenzyloxy)-3-(4-chlorophenyl)-7-iodo-1-[2-(2-methoxyethoxy)ethyl]-1,3-dihydrobenzo[e][1,4]diazepin-2-one; 4-(2-Amino-4-chlorobenzyl)-3-(4-chlorophenyl)-7-iodo-3,4-dihydro-1*H*-benzo[e][1,4]diazepine-2,5-dione; 4-[1-(3-Amino-4-chlorophenyl)ethyl]-3-(4-chlorophenyl)-7-iodo-3,4-dihydro-1H benzo[e][1,4]diazepine-2,5-dione; 4-Benzyl-7-bromo-3-(4-chlorophenyl)-1-methyl-1,4-benzodiazepine-2,5-dione; 7-Bromo-3-(4-chlorophenyl)-1-methyl-4-(1-phenethyl)-1,4-benzodiazepine-2,5-dione; 1,3-Dihydro-4-[1-(2-amino-4-chlorophenyl)ethyl]-3-(4-chlorophenyl)-7-iodo-1,4-benzodiazepine-2,5-dione; 3-(4-Chlorophenyl)-4-[1-(4-chloro-2-nitrophenyl)ethyl]-7-iodo-1-[2-(4-morpholino)ethyl]-1,4-benzodiazepine-2,5-dione; 5-[(3S)-3-(4-Chlorophenyl)-4-[(*R*)-1-(4-chlorophenyl)ethyl]-2,5-dioxo-7-(propyn-1-yl)-1,4-benzodiazepin-1-yl]valeric acid sodium salt; 4-[1-(2-Amino-4-chlorophenyl)ethyl]-3-(4-chlorophenyl)-7-iodo-1-[2-(2-methoxyethoxy)ethyl]-1,4-benzodiazepine-2,5-dione; 4-[(R)-1-(2-amino-4-chlorophenyl)ethyl]-(3S)-3-(4-chlorophenyl)-7-iodo-1-[2-(2-methoxyethoxy)ethyl]-1,4-benzodiazepine-2,5-dione; (3R)-4-[(S)-1-(2-amino-4-chlorophenyl)ethyl]-3-(4-chlorophenyl)-7-iodo-1-[2-(2-methoxyethoxy)ethyl]-1,4-benzodiazepine-2,5-dione; 4-[1-(2-Amino-4-chlorophenyl)ethyl]-3-(4-chlorophenyl)-7-iodo-1-[2-(4-morpholino)ethyl]-1,4-benzodiazepine-2,5-dione; (3S)-4-[(R)-1-(2-Amino-4-chlorophenyl)ethyl]-3-(4-chlorophenyl)-7-iodo-1-[2-(4-morpholino)ethyl]-1,4-benzodiazepine-2,5-dione; (3R)-4-[(S)-1-(2-amino-4-chlorophenyl)ethyl]-3-(4-chlorophenyl)-7-iodo-1-[2-(4-morpholino)ethyl]-1,4-benzodiazepine-2,5-dione; 3-(4-Chlorophenyl)-4-[1-(2,6-dichloro-3-pyridyl)ethyl]-7-iodo-1,4-benzodiazepine-2,5-dione; 1,3-Dihydro-4-[1-(2-amino-4-chlorophenyl)ethyl]-3-(4-chlorophenyl)-7-iodo-1-methyl-1,4-benzodiazepine-2,5-dione; 1,3-Dihydro-4-[1-(2-acetylamino-4-chlorophenyl)ethyl]-3-(4-chlorophenyl)-7-iodo-1,4-benzodiazepine-2,5-dione; 1,3-Dihydro-4-[1-(2-azido-3-pyridyl)ethyl]-3-(4-chlorophenyl)-7-iodo-1,4-benzodiazepine-2,5-dione; 4-[1-(2-Amino-4-chlorophenyl)ethyl]-3-(4-chlorophenyl)-1-[2-(2-methoxyethoxy)ethyl]- 7-(propyn-1-yl)-1,4-benzodiazepine-2,5-dione hydrochloride; (3S)-4-[(R)-1-(2-Amino-4-chlorophenyl)ethyl]-3-(4-chlorophenyl)-1-[2-(2-methoxyethoxy)ethyl]- 7-(propyn-1-yl)-1,4-benzodiazepine-2,5-dione hydrochloride; 4-[1-(2-Amino-4-chlorophenyl)ethyl]-3-(4-chlorophenyl)-1-[2-(4-morpholino)ethyl]-7-(propyn-1-yl)-1,4-benzodiazepine-2,5-dione hydrochloride; (3S)-4-[(R)-1-(2-Amino-4-chlorophenyl)ethyl]-3-(4-chlorophenyl)-1-[2-(4-morpholino)ethyl]-7-(propyn-1-yl)-1,4-benzodiazepine-2,5-dione hydrochloride; 4-[1-(2-Amino-4-chlorophenyl)ethyl]-3-(4-chlorophenyl)-7-iodo-2,3-dihydro-1H-1,4-benzodiazepin-5-one; 4-[1-(2-amino-6-chloro-3-pyridyl)methyl]-3-(4-chlorophenyl)-7-iodo-1-[2-(2-methoxyethoxy)ethyl]-1,4-benzodiazepine-2,5-dione hydrochloride; 4-[1-(3-Amino-4-chlorophenyl)cyclopropyl]-3-(4-chlorophenyl)-1-[2-(4-morpholino)ethyl]-7-iodo-1,4-benzodiazepine-2,5-dione methanesulfonate; (3S)-4-[1-(2-Amino-4-chlorophenyl)ethyl]-3-(4-chlorophenyl)-7-iodo-1-[(R)-2-(1-piperazinyl)-2-oxoethyl]-1,4-benzodiazepine-2,5-dione hydrochloride; 4-(4-Chloro-2-methyl-benzyl)-3-(4-chloro-phenyl)-7-iodo-1-(2-morpholin-4-yl-ethyl)-3,4-dihydro-1H- benzo[e][1,4]diazepine-2,5-dione; 3-(S)-(4-Chlorophenyl)-4-[1-(R)-(4-chloro-phenyl)-ethyl]-7-iodo-1-(2-morpholin-4-yl-2-oxoethyl)-3,4-dihydro-1H-benzo[e][1,4]diazepine-2,5-dione; 3-(S)-(4-Chlorophenyl)-4-[1-(R)-(4-chloro-phenyl)-ethyl]-7-iodo-1-(2-morpholin-4-yl-ethyl)-3,4-dihydro-1H-benzo[e][1,4]diazepine-2,5-dione; 3-(R)-(4-Chloro-phenyl)-4-[1-(R)-(4-chloro-phenyl)-ethyl]-7-iodo-1-(2-morpholin-4-yl-ethyl)-3,4-dihydro-1H-benzo[e][1,4]diazepine-2,5-dione; 3-(S)-(4-Chloro-phenyl)-4-[1-(R)-(4-chloro-phenyl)-ethyl]-7-iodo-1-[2-(2-methoxy-ethoxy)-ethyl]-3,4-dihydro-1H-benzo[e][1,4]diazepine-2,5-dione; 3-(S)-(4-Chloro-phenyl)-4-[1-(R)-(4-chloro-phenyl)-ethyl]-7-(1-hydroxyimino-ethyl)-3,4-dihydro-1H-benzo[e][1,4]diazepine-2,5-dione; 7-Iodo-4-naphthalen-1-ylmethyl-3-(4-trifluoromethoxy-phenyl)-3,4-dihydro-1H-benzo[e][1,4]diazepine-2,5-dione; 4-Benzo[1,3]dioxol-5-ylmethyl-3-(4-chloro-phenyl)-7-iodo-3,4-dihydro-1H-benzo[e][1,4]diazepine-2,5-dione; 4-Benzo[1,3]dioxol-5-ylmethyl-7-iodo-3-(4-trifluoromethoxy-phenyl)-3,4-dihydro-1H-benzo[e][1,4]diazepine-2,5-dione; 7-Iodo-4-(2-pyridin-2-yl-ethyl)-3-(4-trifluoromethoxy-phenyl)-3,4-dihydro-1H-benzo[e][1,4]diazepine-2,5-dione; 4-Benzyl-3-(4-chloro-phenyl)-7-iodo-3,4-dihydro-1H-benzo[e][1,4]diazepine-2,5-dione; 3-(4-Chlorophenyl)-7-iodo-4-phenethyl-3,4-dihydro-1H-benzo[e][1,4]diazepine-2,5-dione; 4-Benzyl-7-iodo-3-(4-trifluoromethoxy-phenyl)-3,4-dihydro-1H-benzo[e][1,4]diazepine-2,5-dione; 7-Iodo-4-phenethyl-3-(4-trifluoromethoxyphenyl)-3,4-dihydro-1H-benzo[e][1,4]diazepine-2,5-dione.

The invention disclosed herein is also meant to encompass the *in vivo* metabolic products of the disclosed compounds. Such products may result for example from the oxidation, reduction, hydrolysis, amidation, esterification and the like of the administered compound, primarily due to enzymatic processes. Accordingly, the invention includes compounds produced by a process comprising contacting a compound of this invention with a mammal for a period of time sufficient to yield a metabolic product thereof. Such products typically are identified by preparing a radiolabeled compound of the invention, administering it parenterally in a detectable dose to an animal such as rat, mouse, guinea pig, monkey, or to man, allowing sufficient time for metabolism to occur and isolating its conversion products from the urine, blood or other biological samples.

Some of the compounds disclosed herein may contain one or more asymmetric centers and thus give rise to enantiomers, diastereomers, and other stereoisomeric forms. The present invention is also meant to encompass all such possible forms as well as their racemic and resolved forms and mixtures thereof When the compounds described herein contain olefinic double bonds or other centers of geometric asymmetry, and unless specified otherwise, it is intended to include both E and Z geometric isomers. All tautomers are intended to be encompassed by the present invention as well.

As used herein, the term "stereoisomers" is a general term for all isomers of individual molecules that differ only in the orientation of their atoms in space. It includes enantiomers and isomers of compounds with more than one chiral center that are not mirror images of one another (diastereomers).

The term "chiral center" refers to a carbon atom to which four different groups are attached, or a sulfur atom to which three different groups are attached, where the sulfur atom and its attached groups form a sulfoxide, sulfinic ester, sulfonium salt or sulfite.

The term "enantiomer" or "enantiomeric" refers to a molecule that is nonsuperimposable on its mirror image and hence optically active wherein the enantiomer rotates the plane of polarized light in one direction and its mirror image rotates the plane of polarized light in the opposite direction.

The term "racemic" refers to a mixture of equal parts of enantiomers and which is optically inactive.

The term "resolution" refers to the separation or concentration or depletion of one of the two enantiomeric forms of a molecule. The phrase "enantiomeric excess" refers to a mixture wherein one enantiomer is present is a greater concentration than its mirror image molecule.

The compounds of Formula *I* may also be solvated, especially hydrated. Hydration may occur during manufacturing of the compounds or compositions comprising the compounds, or the hydration may occur over time due to the hygroscopic nature of the compounds.

Certain compounds within the scope of Formula I are derivatives referred to as "prodrugs." The expression "prodrug" denotes a derivative of a known direct acting drug, which derivative has enhanced delivery characteristics and therapeutic value as compared to the drug, and is transformed into the active drug by an enzymatic or chemical process. Prodrugs are derivatives of the compounds of the invention which have metabolically cleavable groups and become by solvolysis or under physiological conditions the compounds of the invention which are pharmaceutically active *in vivo.* For example, ester derivatives of compounds of this invention are often active *in vivo,* but *not in vitro.* Other derivatives of the compounds of this invention have activity in both their acid and acid derivative forms, but the acid derivative form often offers advantages of solubility, tissue compatibility, or delayed release in the mammalian organism (see, Bundgard, H., Design of Prodrugs, pp. 7-9, 21-24, Elsevier, Amsterdam 1985). Prodrugs include acid derivatives well known to practitioners of the art, such as, for example, esters prepared by reaction of the parent acid with a suitable alcohol, or amides prepared by reaction of the parent acid compound with an amine. Simple aliphatic or aromatic esters derived from acidic groups pendent on the compounds of this invention are preferred prodrugs. In some cases it is desirable to prepare double ester type prodrugs such as (acyloxy) alkyl esters or ((alkoxycarbonyl)oxy)alkyl esters. Useful prodrugs are those where R^{b} is alkyl, alkenyl, alkynyl, or arylalkyl.

When any variable occurs more than one time in any constituent or in Formula I, its definition on each occurrence is independent of its definition at every other occurrence. Also, combinations of substituents and/or variables are permissible only if such combinations result in stable compounds.

### Definitions

The term "alkyl" as employed herein by itself or as part of another group refers to both straight and branched chain radicals of up to 10 carbons, unless the chain length is otherwise limited, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, or decyl.

The term "alkenyl" is used herein to mean a straight or branched chain radical of 2-10 carbon atoms, unless the chain length is otherwise limited, wherein there is at least one double bond between two of the carbon atoms in the chain, including, but not limited to, ethenyl, 1-propenyl, 2-propenyl, 2-methyl-1-propenyl, 1-butenyl, 2-butenyl, and the like. Preferably, the alkenyl chain is 2 to 8 carbon atoms in length, most preferably from 2 to 4 carbon atoms in length.

The term "alkynyl" is used herein to mean a straight or branched chain radical of 2-10 carbon atoms, unless the chain length is otherwise limited, wherein there is at least one triple bond between two of the carbon atoms in the chain, including, but not limited to, ethynyl, 1-propynyl, 2-propynyl, and the like. Preferably, the alkynyl chain is 2 to 8 carbon atoms in length, most preferably from 2 to 4 carbon atoms in length.

In all instances herein where there is an alkenyl or alkynyl moiety as a substituent group, the unsaturated linkage, i.e., the vinyl or ethenyl linkage, is preferably not directly attached to a nitrogen, oxygen or sulfur moiety.

The term "alkoxy" or "alkyloxy" refers to any of the above alkyl groups linked to an oxygen atom. Typical examples are methoxy, ethoxy, isopropyloxy, *sec-* butyloxy, and *t*-butyloxy.

The term "aryl" as employed herein by itself or as part of another group refers to monocyclic or bicyclic aromatic groups containing from 6 to 12 carbons in the ring portion, preferably 6-10 carbons in the ring portion. Typical examples include phenyl, biphenyl, naphthyl or tetrahydronaphthyl.

The term "aralkyl" or "arylalkyl" as employed herein by itself or as part of another group refers to C₁₋₆ alkyl groups as discussed above having an aryl substituent, such as benzyl, phenylethyl or 2-naphthylmethyl.

The term "heteroaryl" as employed herein refers to groups having 5 to 14 ring atoms; 6, 10 or 14 pi electrons shared in a cyclic array; and containing carbon atoms and 1, 2, 3, or 4 oxygen, nitrogen or sulfur heteroatoms (where examples of heteroaryl groups are: thienyl, benzo[b]thienyl, naphtho[2,3-b]thienyl, thianthrenyl, furyl, pyranyl, isobenzofuranyl, benzoxazolyl, chromenyl, xanthenyl, phenoxathiinyl, 2*H*-pyrrolyl, pyrrolyl, imidazolyl, pyrazolyl, pyridyl, pyrazinyl, pyrimidinyl, pyridazinyl, indolizinyl, isoindolyl, 3*H*-indolyl, indolyl, indazolyl, purinyl, 4*H*-quinolizinyl, isoquinolyl, quinolyl, phthalazinyl, naphthyridinyl, quinazolinyl, cinnolinyl, pteridinyl, 4_{α}*H-*carbazolyl, carbazolyl, β-carbolinyl, phenanthridinyl, acridinyl, perimidinyl, phenanthrolinyl, phenazinyl, isothiazolyl, phenothiazinyl, isoxazolyl, furazanyl, phenoxazinyl, and tetrazolyl groups).

The phrase "saturated or partially unsaturated heterocycle" as employed herein, by itself or as part of another group, refers to a saturated or partially unsaturated ring system having 5 to 14 ring atoms selected from carbon atoms and 1, 2, 3, or 4 oxygen, nitrogen, or sulfur heteroatoms. Typical saturated examples include pyrrolidinyl, imidazolidinyl, pyrazolidinyl, tetrahydrofuranyl, tetrahydropyranyl, piperidyl, piperazinyl, quinuclidinyl, morpholinyl, and dioxacyclohexyl. Typical partially unsaturated examples include pyrrolinyl, imidazolinyl, pyrazolinyl, dihydropyridinyl, tetrahydropyridinyl, and dihydropyranyl. Either of these systems can be optionally fused to a benzene ring.

The terms "heteroarylalkyl" or "heteroaralkyl" as employed herein both refer to a heteroaryl group attached to an alkyl group. Typical examples include 2-(3-pyridyl)ethyl, 3-(2-furyl)-*n*-propyl, 3-(3-thienyl)-*n*-propyl, and 4-(1-isoquinolinyl)-*n*-butyl.

The term "cycloalkyl" as employed herein by itself or as part of another group refers to cycloalkyl groups containing 3 to 9 carbon atoms. Typical examples are cyclopropyl, cyclobutyl, cyclopentyl, cyclohexyl, cycloheptyl, cyclooctyl and cyclononyl.

The term "cycloalkylalkyl" or "cycloalkyl(alkyl)" as employed herein, by itself or as part of another group, refers to a cycloalkyl group attached to an alkyl group. Typical examples are 2-cyclopentylethyl, cyclohexylmethyl, cyclopentylmethyl, 3-cyclohexyl-*n*-propyl, and 5-cyclobutyl-*n*-pentyl.

The term "cycloalkenyl" as employed herein, by itself or as part of another group, refers to cycloalkenyl groups containing 3 to 9 carbon atoms and 1 to 3 carbon-carbon double bonds. Typical examples include cyclopropenyl, cyclobutenyl, cyclopentenyl, cyclohexenyl, cyclohexadienyl, cycloheptenyl, cycloheptadienyl, cyclooctenyl, cyclooctadienyl, cyclooctatrienyl, cyclononenyl, and cyclononadienyl.

The term "halogen" or "halo" as employed herein by itself or as part of another group refers to chlorine, bromine, fluorine or iodine.

The term "monoalkylamine" or "monoalkylamino" as employed herein by itself or as part of another group refers to the group NH₂ wherein one hydrogen has been replaced by an alkyl group, as defined above.

The term "dialkylamine" or "dialkylamino" as employed herein by itself or as part of another group refers to the group NH₂ wherein both hydrogens have been replaced by alkyl groups, as defined above.

The term "hydroxyalkyl" as employed herein refers to any of the above alkyl groups wherein one or more hydrogens thereof are substituted by one or more hydroxyl moieties.

The term "haloalkyl" as employed herein refers to any of the above alkyl groups wherein one or more hydrogens thereof are substituted by one or more halo moieties. Typical examples include fluoromethyl, difluoromethyl, trifluoromethyl, trichloroethyl, trifluoroethyl, fluoropropyl, and bromobutyl.

The term "carboxyalkyl" as employed herein refers to any of the above alkyl groups wherein one or more hydrogens thereof are substituted by one or more carboxylic acid moieties.

The term "heteroatom" is used herein to mean an oxygen atom ("O"), a sulfur atom ("S") or a nitrogen atom ("N"). It will be recognized that when the heteroatom is nitrogen, it may form an NR^{a}R^{b} moiety, wherein R^{a} and R^{b} are, independently from one another, hydrogen or C₁ to C₈ alkyl, or together with the nitrogen to which they are bound form a saturated or unsaturated 5-, 6-, or 7-membered ring.

The phrase "optionally-substituted" when not explicitly defined refers to a group or groups being optionally substituted with one or more substituents independently selected from the group consisting of hydroxy, nitro, trifluoromethyl, halogen, C₁₋₆ alkyl, C₁₋₆ haloalkyl, C₁₋₆ alkoxy, C₁₋₆ alkylenedioxy, C₁₋₆ aminoalkyl, C₁₋₆ hydroxyalkyl, C₂₋₄ alkenyl, C₂₋₄ alkynyl, C₆₋₁₀ aryl, phenoxy, benzyloxy, 5-10 membered heteroaryl, C₁₋₆ aminoalkoxy, amino, mono(C₁₋₄)alkylamino, di(C₁₋₄)alkylamino, C₂₋₆ alkylcarbonylamino, C₂₋₆ alkoxycarbonylamino, C₂₋₆ alkoxycarbonyl, C₂₋₆ alkoxycarbonylalkyl, carboxy, C₂₋₆ hydroxyalkoxy, (C₁₋₆)alkoxy(C₂₋₆)alkoxy, mono(C₁₋₄)alkylamino(C₂₋₆)alkoxy, di(C₁₋₄)alkylamino(C₂₋₆)alkoxy C₂₋₁₀ mono(carboxyalkyl)amino, bis(C₂₋₁₀ carboxyalkyl)amino, C₂₋₆ carboxyalkoxy, C₂₋₆ carboxyalkyl, carboxyalkylamino, guanidinoalkyl, hydroxyguanidinoalkyl, cyano, trifluoromethoxy, or perfluoroethoxy.

Preferred optional substituents include one or more substituents independently selected from the group consisting of nitro, hydroxy, carboxy, C₁₋₄ alkoxy, C₁₋₄ alkyl, halo, C₁₋₄ haloalkyl, C₁₋₄ alkylthio, thio, amino, mono(C₁₋₄)alkylamino, and di(C₁₋₄)alkylamino.

A "biological sample" as used herein refers to a sample containing or consisting of cell or tissue matter, such as cells or biological fluids isolated from a subject.

The "subject" can be a mammal, such as a rat, a mouse, a monkey, or a human, that has been the object of treatment, observation or experiment. Examples of biological samples include, for example, sputum, blood, blood cells (e.g., white blood cells), amniotic fluid, plasma, semen, bone marrow, tissue or fine-needle biopsy samples, urine, peritoneal fluid, pleural fluid, and cell cultures. Biological samples may also include sections of tissues such as frozen sections taken for histological purposes. A test biological sample is the biological sample that has been the object of analysis, monitoring, or observation. A control biological sample can be either a positive or a negative control for the test biological sample. Often, the control biological sample contains the same type of tissues, cells and/or biological fluids of interest as that of the test biological sample.

A "cell" refers to at least one cell or a plurality of cells appropriate for the sensitivity of the detection method. Cells suitable for the present invention may be bacterial, but are preferably eukaryotic, and are most preferably mammalian.

A "mdm2" or "hdm2" gene or oncogene is used herein to mean the murine double minute 2 gene, and homologous genes found in other animals. This gene is a target gene of the transcription factor tumor protein p53. It encodes a protein MDM2 or HDM2. Overexpression of this gene can result in excessive inactivation of tumor protein p53, diminishing its tumor suppressor function. More than 40 different alternatively spliced transcript variants have been isolated from both tumor and normal tissues.

"MDM2" or "HDM2" is used herein to mean a protein obtained as a result of expression of the mdm2 oncogene. Within the meaning of this term, it will be understood that MDM2 encompasses all proteins encoded by mdm2, mutants thereof, alternative splice proteins thereof, and phosphorylated proteins thereof. Additionally, as used herein, it will be understood that the term "MDM2" includes MDM2 homologues of other animals (e.g., HDM2).

A "MDM2" or "HDM2" protein is a nuclear phosphoprotein that binds and inhibits transactivation by tumor protein p53, as part of an autoregulatory negative feedback loop. This protein has E3 ubiquitin ligase activity, which targets tumor protein p53 for proteasomal degradation. This protein also affects the cell cycle, apoptosis, and tumorigenesis through interactions with other proteins, including retinoblastoma 1 and ribosomal protein L5. Exemplary "MDM2" includes the human MDM2 isoforms, such as those listed by GenBank protein ID: NP_002383, NP_006869, NP_006870, NP_006871, NP_006872, NP_006873, and their structural and functional polymorphisms. "Polymorphism" refers to a set of genetic variants at a particular genetic locus among individuals in a population. MDM2 also includes orthologs of the human MDM2 in other animals including rat, mouse, pig, dog and monkey.

A p21^{wafl/cipl} gene refers to the gene of cyclin-dependent kinase inhibitor 1A. This gene encodes a potent cyclin-dependent kinase inhibitor. The encoded protein binds to and inhibits the activity of cyclin-CDK2 or - CDK4 complexes, and thus functions as a regulator of cell cycle progression at G1. The expression of this gene is tightly controlled by the tumor suppressor protein p53, through which this protein mediates the p53-dependent cell cycle G 1 phase arrest in response to a variety of stress stimuli. The protein encoded by the p21^{wafl/cipl} gene can interact with proliferating cell nuclear antigen (PCNA), a DNA polymerase accessory factor, and plays a regulatory role in S phase DNA replication and DNA damage repair. Examples of the gene include the two alternatively spliced variants, GenBank cDNA accession number NM_000389, and NM_078467, which encode an identical protein in human. "p21^{wafl/cipl} gene" also includes orthologs of the human p21^{waf1/cip1} gene in other animals including rat, mouse, pig, dog and monkey.

A "p53", or "TP53", or "tumor protein p53" all refers to a nuclear protein that plays an essential role in the regulation of cell cycle, specifically in the transition from G0 to G1. It is found in very low levels in normal cells, however, in a variety of transformed cell lines, it is expressed in high amounts, and believed to contribute to transformation and malignancy. p53 is a DNA-binding protein containing DNA-binding, oligomerization and transcription activation domains. It is postulated to bind as a tetramer to a p53-binding site and activate expression of downstream genes that inhibit growth and/or invasion, and thus function as a tumor suppressor. Mutants of p53 that frequently occur in a number of different human cancers fail to bind the consensus DNA binding site, and hence cause the loss of tumor suppressor activity. Alterations of the TP53 gene occur not only as somatic mutations in human malignancies, but also as germline mutations in some cancer-prone families with Li-Fraumeni syndrome. Exemplary "p53" includes the human p53, such as that listed by GenBank protein ID: NP_000537, and its structural and functional polymorphisms. P53 also includes orthologs of the human p53 in other animals including rat, mouse, pig, dog and monkey.

The phrase "antineoplastic agent" is used herein to mean any agent that is used to treat or prevent cancer or other conditions comprising uncontrolled proliferation and growth of cells. Antineoplastic agents include anticancer agents.

The phrase "contacting one or more proteins" is used herein to mean placing a compound of the present invention in a solution with one or more proteins of interest. A compound of Formula I and one or more proteins of interest may be in solution together in an aqueous solution, non-aqueous solution, or combination of an aqueous solution and non-aqueous solution. Other proteins may be present in solution along with the compound of Formula I and the protein of interest. Other inorganic or organic molecules may be present in the solution. Such inorganic and organic molecules include, but are not limited to, NaCl, HEPES, and octyl glucoside. The solution may be within an animal cell or outside of an animal cell.

The phrase "inhibiting the binding" is used herein to mean preventing or reducing the direct or indirect association of one or more molecules, peptides, proteins, enzymes, or receptors; or preventing or reducing the normal activity of one or more molecules, peptides, proteins, enzymes, or receptors.

The phrase "inducing apoptosis" is used herein to mean causing directly or indirectly a cell of animal origin to undergo apoptosis, a process of controlled, or programmed, cellular death.

The phrase "HDM2 inhibitor" is used herein to describe an agent which inhibits the function of HDM2 in the assay described in Example 35.

The phrase "a small molecule that inhibits the binding between proteins MDM2 and p53" refers to a small organic compound, i.e., having a molecular weight of more than 50 yet less than about 3000, that inhibits the binding between the two proteins p53 and MDM2. Examples of "small molecule that inhibits the binding between proteins MDM2 and p53" include, but are not limited to Benzodiazapinediones that have been described in WO200304175 or WO2004096134, synthetic chalcones, norbornane derivatives, cis-imidazoline derivatives (Nutlins), a pyrazolidinedione sulfonamide and 1,4-benzodiazepine-2,5-diones, as well as tryptophan derivatives (Buolamwini et al., 2005, *Curr Cancer Drug Targets.* 5(1):57-68). As used herein, "radiation therapy" or "radiation treatment" refers to a therapy that comprises exposing the subject or cell to radiation. Such therapy or treatment is known to those skilled in the art. The appropriate scheme of radiation therapy will be similar to those already employed in clinical therapies wherein the radiation therapy is used alone or in combination with other chemotherapeutics.

The pharmaceutically-acceptable salts of the compounds of Formula *I* (in the form of water- or oil-soluble or dispersible products) include the conventional non-toxic salts or the quaternary ammonium salts which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, nitrate, oxalate, palmoate, pectinate, persulfate, 3-phenylpropionate, picrate, pivalate, propionate, succinate, sulfate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quatemized with such agents as lower alkyl halides, such as methyl, ethyl, propyl and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; and aralkyl halides like benzyl and phenethyl bromides and others. Preferred acids for forming acid addition salts include HCl, acetic acid, trifluoroacetic acid and fumaric acid.

### Compositions and Methods of Use

Compositions of the present invention include pharmaceutical compositions comprising a compound of Formula ***I,*** wherein R¹-R¹¹ and X are defined above, and one or more pharmaceutically acceptable excipients. Preferred compositions of the present invention are pharmaceutical compositions comprising a compound selected from a preferred group of compounds of Formula *I* as defined above, and one or more pharmaceutically acceptable excipients.

The pharmaceutical compositions of the invention can be administered to any animal that can experience the beneficial effects of the compounds of the invention. Foremost among such animals are humans, although the invention is not intended to be so limited.

The pharmaceutical compositions of the present invention can be administered by any means that achieve their intended purpose. For example, administration can be by subcutaneous, intravenous, intramuscular, intraperitoneal, buccal, or ocular routes, rectally, parenterally, intrasystemically, intravaginally, topically (as by powders, ointments, drops or transdermal patch), or as an oral or nasal spray. Alternatively, or concurrently, administration can be by the oral route. The dosage administered will be dependent upon the age, health, and weight of the recipient, kind of concurrent treatment, if any, frequency of treatment, and the nature of the effect desired.

In addition to the pharmacologically active compounds, the new pharmaceutical preparations can contain suitable pharmaceutically acceptable carriers comprising excipients and auxiliaries that facilitate processing of the active compounds into preparations that can be used pharmaceutically.

The pharmaceutical preparations of the present invention are manufactured in a manner that is, itself, known, for example, by means of conventional mixing, granulating, dragée-making, dissolving, or lyophilizing processes. Thus, pharmaceutical preparations for oral use can be obtained by combining the active compounds with solid excipients, optionally grinding the resulting mixture and processing the mixture of granules, after adding suitable auxiliaries, if desired or necessary, to obtain tablets or dragée cores.

Suitable excipients are, in particular, fillers such as saccharides, for example, lactose or sucrose, mannitol or sorbitol, cellulose preparations and/or calcium phosphates, for example, tricalcium phosphate or calcium hydrogen phosphate, as well as binders, such as, starch paste, using, for example, maize starch, wheat starch, rice starch, potato starch, gelatin, tragacanth, methyl cellulose, hydroxypropyl methylcellulose, sodium carboxymethylcellulose, and/or polyvinyl pyrrolidone. If desired, disintegrating agents can be added, such as, the above-mentioned starches and also carboxymethyl-starch, crosslinked polyvinyl pyrrolidone, agar, or alginic acid or a salt thereof, such as, sodium alginate. Auxiliaries are, above all, flow-regulating agents and lubricants, for example, silica, talc, stearic acid or salts thereof, such as, magnesium stearate or calcium stearate, and/or polyethylene glycol. Dragée cores are provided with suitable coatings that, if desired, are resistant to gastric juices. For this purpose, concentrated saccharide solutions can be used, which can contain gum arabic, talc, polyvinyl pyrrolidone, polyethylene glycol, and/or titanium dioxide, lacquer solutions and suitable organic solvents or solvent mixtures. In order to produce coatings resistant to gastric juices, solutions of suitable cellulose preparations, such as, acetylcellulose phthalate or hydroxypropylmethyl-cellulose phthalate, are used. Dye stuffs or pigments can be added to the tablets or dragée coatings, for example, for identification or in order to characterize combinations of active compound doses.

Other pharmaceutical preparations which can be used orally include push-fit capsules made of gelatin, as well as soft, sealed capsules made of gelatin and a plasticizer, such as, glycerol or sorbitol. The push-fit capsules can contain the active compounds in the form of granules that may be mixed with fillers such as lactose, binders such as starches, and/or lubricants such as talc or magnesium stearate and, optionally, stabilizers. In soft capsules, the active compounds are preferably dissolved or suspended in suitable liquids, such as, fatty oils or liquid paraffin. In addition, stabilizers may be added.

Suitable formulations for parenteral administration include aqueous solutions of the active compounds in water-soluble form, for example, water-soluble salts, alkaline solutions and cyclodextrin inclusion complexes. Especially preferred alkaline salts are ammonium salts prepared, for example, with Tris, choline hydroxide, Bis-Tris propane, *N*-methylglucamine, or arginine. One or more modified or unmodified cyclodextrins can be employed to stabilize and increase the water solubility of compounds of the present invention. Useful cyclodextrins for this purpose are disclosed in U.S. Patent Nos. 4,727,064, 4,764,604, and 5,024,998.

In addition, suspensions of the active compounds as appropriate oily injection suspensions can be administered. Suitable lipophilic solvents or vehicles include fatty oils, for example, sesame oil, or synthetic fatty acid esters, for example, ethyl oleate or triglycerides or polyethylene glycol-400 (the compounds are soluble in PEG-400). Aqueous injection suspensions can contain substances that increase the viscosity of the suspension, for example, sodium carboxymethyl cellulose, sorbitol, and/or dextran. Optionally, the suspension may also contain stabilizers.

Liquid dosage forms for oral administration include pharmaceutically acceptable emulsions, solutions, suspensions, syrups, and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

Topical administration includes administration to the skin or mucosa, including surfaces of the lung and eye. Compositions for topical administration, including those for inhalation, may be prepared as a dry powder which may be pressurized or non-pressurized. In nonpressurized powder compositions, the active ingredients in finely divided form may be used in admixture with a larger-sized pharmaceutically acceptable inert carrier comprising particles having a size, for example, of up to 100 micrometers in diameter. Suitable inert carriers include sugars such as lactose. Desirably, at least 95% by weight of the particles of the active ingredient have an effective particle size in the range of 0.01 to 10 micrometers.

Alternatively, the composition may be pressurized and contain a compressed gas, such as nitrogen or a liquefied gas propellant. The liquefied propellant medium and indeed the total composition are preferably such that the active ingredients do not dissolve therein to any substantial extent. The pressurized composition may also contain a surface-active agent. The surface-active agent may be a liquid or solid non-ionic surface-active agent or may be a solid anionic surface-active agent. It is preferred to use the solid anionic surface-active agent in the form of a sodium salt.

A further form of topical administration is to the eye. The compounds and compositions of the present invention are delivered in a pharmaceutically acceptable ophthalmic vehicle, such that the compounds are maintained in contact with the ocular surface for a sufficient time period to allow the compounds to penetrate the corneal and internal regions of the eye, as for example the anterior chamber, posterior chamber, vitreous body, aqueous humor, vitreous humor, cornea, iris/ciliary, lens, choroid/retina and sclera. The pharmaceutically acceptable ophthalmic vehicle may, for example, be an ointment, vegetable oil or an encapsulating material.

Compositions for rectal or vaginal administration are preferably suppositories which can be prepared by mixing the compounds of the present invention with suitable non-irritating excipients or carriers such as cocoa butter, polyethylene glycol or a suppository wax which are solid at room temperature but liquid at body temperature and therefore melt in the rectum or vaginal cavity and release the drugs.

The compositions of the present invention can also be administered in the form of liposomes. As is known in the art, liposomes are generally derived from phospholipids or other lipid substances. Liposomes are formed by mono-or multi-lamellar hydrated liquid crystals that are dispersed in an aqueous medium. Any non-toxic, physiologically acceptable and metabolizable lipid capable of forming liposomes can be used. The present compositions in liposome form can contain, in addition to the compounds of the present invention, stabilizers, preservatives, excipients, and the like. The preferred lipids are the phospholipids and the phosphatidyl cholines (lecithins), both natural and synthetic. Methods to form liposomes are known in the art (see, for example, Prescott, Ed., *Meth. Cell Biol. 14*:33 (1976)).

Compounds of the present invention may be used in combination with one or more additional antineoplastic agents. The compound of the present invention may be formulated with the other antineoplastic agent or agents so that a pharmaceutical composition comprising a compound of Formula I and one or more additional antineoplastic agents is administered to an animal. Alternatively, the compound of Formula I can be administered as a separate pharmaceutical composition from the composition comprising the one or more additional antineoplastic agents. Antineoplastic agents that may be used in combination with the compounds of the present invention include compounds selected from the following compounds and classes of antineoplastic agents:
1. fluoropyrimidines, such as 5-FU (5-fluorouracil), Fluorodeoxyuridine, Ftorafur, 5'-deoxyfluorouridine, UFT, and S-1 Capecitabine;
2. pyrimidine nucleosides, such as Deoxycytidine, Cytosine Arabinoside, Cytarabine, Azacitidine, 5-Azacytosine, Gencitabine, and 5-Azacytosine-Arabinoside;
3. purines, such as 6-Mercaptopurine, Thioguanine, Azathioprine, Allopurinol, Cladribine, Fludarabine, Pentostatin, and 2-Chloroadenosine;
4. platinum analogues, such as Cisplatin, Carboplatin, Oxaliplatin, Tetraplatin, Platinum-DACH, Ormaplatin, and CI-973, JM-216;
5. anthracyclines/anthracenediones, such as Doxorubicin, Daunorubicin, Epirubicin, Idarubicin, and Mitoxantrone;
6. epipodophyllotoxins, such as Etoposide, and Teniposide;
7. camptothecins, such as Irinotecan, Topotecan, 9-Amino Camptothecin, 10,11-Methylenedioxy Camptothecin, 9-Nitro Camptothecin, and TAS 103;
8. hormones and hormonal analogues, such as diethylstilbestrol, Tamoxifen, Toremefine, Tolmudex, Thymitaq, flutamide, fluoxymesterone, bicalutamide, Finasteride, estradiol, Trioxifene, dexamethasone, leuproelin acetate, estramustine, Droloxifene, medroxyprogesterone, megesterol acetate, aminoglutethimide, testolactone, testosterone, diethylstilbestrol, and hydroxyprogesterone;
9. enzymes, proteins and antibodies, such as Asparaginase, Interleukins, Interferons, Leuprolide, and Pegaspargase;
10. vinca alkaloids, such as Vincristine, Vinblastine, Vinorelbine, and Vindesine;
11. taxanes, such as Paclitaxel, Taxotere and Docetaxel.

Antineoplastic agents that may be used in combination with compounds of the invention also include compounds selected from the following Mechanism-Based Classes:
1. Antihormonals-See classification for Hormones and Hormonal Analogs above, Anastrozole, Goserelin, and Aminoglutethimide;
2. Antifolates, such as methotrexate, leucovorin, aminopterin, trimetrexate, Trimethoprim, pyritrexim, pyrimethamine, Edatrexate, and MDAM;
3. Antimicrotubule Agents, such as Taxanes, Vinca Alkaloids, and Vinorelbine;
4. Alkylating Agents (Classical and Non-Classical), such as Nitrogen Mustards (Mechlorethamine, Chlorambucil, Melphalan, Uracil Mustard), Oxazaphosphorines (Ifosfamide, Cyclophosphamide, Perfosfamide, Trophosphamide), Alkylsulfonates (Busulfan), Nitrosoureas (Carmustine, Lomustine, Streptozocin), Thiotepa, and Dacarbazine;
5. Antimetabolites, such as Purines, pyrimidines and nucleoside analogs, listed above;
6. Antibiotics, such as Anthracyclines/Anthracenediones, Bleomycin, Dactinomycin, Mitomycin, Plicamycin, Pentostatin, and Streptozocin;
7. Topoisomerase Inhibitors, such as Camptothecins (Topo I), Epipodophyllotoxins, AMSA, VP-16 and Ellipticines (Topo II);
8. Antivirals, such as AZT, acyclovir, penciclovir, famcyclovir, didehydrodideoxythymidine, dideoxycytidine, -SddC, ganciclovir, dideoxyinosine, and viral-specific protease inhibitors;
9. Miscellaneous Cytotoxic Agents, such as Hydroxyurea, Mitotane, Fusion Toxins, PZA, Bryostatin, Retinoids, Butyric Acid and derivatives, Pentosan, Fumagillin, Mitoxantrone, Bone Marrow Growth Factors, and Procarbazine.

Compounds of the present invention are useful for the treatment of uncontrolled proliferation of cells and/or cancer. The compounds of the present invention may produce beneficial cytostatic and/or cytotoxic effects. The cytostatic effects include the inhibition of further cell growth and/or cell division. The cytotoxic effects include the induction of cell death by mechanisms that include apoptosis and cellular necrosis. Specifically, the compounds of the present invention are useful in treating the following cancers: breast cancer, ovarian cancer, cervical carcinoma, endometrial carcinoma, choriocarcinoma, soft tissue sarcomas, osteosarcomas, rhabdomyosarcomas, leiomyomas, leiomyosarcomas, head and neck cancers, lung and bronchogenic carcinomas, brain tumors, neuroblastomas, esophogeal cancer, colorectal adenocarcinomas, bladder cancer, urothelial cancers, leukemia, lymphoma, malignant melanomas, oral squamous carcinoma, hepatoblastoma, glioblastoma, astrocytoma, medulloblastoma, Ewing's sarcoma, lipoma, liposarcoma, malignant fibroblast histoma, malignant Schwannoma, testicular cancers, thyroid cancers, Wilms' tumor, pancreatic cancers, colorectal adenocarcinoma, tongue carcinoma, gastric carcinoma, and nasopharyngeal cancers. Preferably, the present invention is used to treat the cancers selected from the group consisting of breast cancer, choriocarcinoma, soft tissue sarcomas, osteosarcomas, rhabdomyosarcomas, lipoma and liposarcoma. The cancers and diseases listed above are merely meant to be illustrative and are by no means meant to be a limiting or exhaustive list.

Additionally, the compounds and compositions described herein are useful to treat any undesired or detrimental condition that results from the HDM2 protein or the MDM2 protein inhibiting the function of p53 or other cellular proteins that induce apoptosis, induce cellular death, or regulate the cellular cycle.

The compounds of the present invention are also useful at inhibiting the interaction between p53 and HDMX and/or MDMX. MDMX, also known as MDM4, is a cellular protein involved in the regulation of the cell cycle. For example, see Riemenschneider *et al., Cancer Res. 59(24)*:6091-6 (1999).

Inhibitors of the interaction of HDM2 and/or MDM2 and p53 are also useful for treating cancer, inhibiting cell growth/replication, and inducing cellular apoptosis and necrosis, when administered along with agents that cause or induce DNA damage (see Chen *et al. Proc. Natl. Acad. Sci. USA* 95:195-200 (1998)). Compounds of the present invention may be used to treat cancer, inhibit cell growth/replication, and induce cellular apoptosis and necrosis, by administering a compound of the present invention along with agents that cause or induce DNA damage. Agents that induce DNA damage include radiation and chemical agents. The radiation can be administered either internally or externally. Chemical agents include any compounds or elements that cause or induce damage to DNA.

The compounds of the present invention may be administered in an effective amount within the dosage range of about 0.01 mg/kg to about 300 mg/kg, preferably between 1.0 mg/kg to 100 mg/kg body weight. Compounds of the present invention may be administered in a single daily dose, or the total daily dosage may be administered in divided doses of two, three or four times daily.

### Preparation of Compounds

The compounds of the present invention can be prepared utilizing a modification of Ugi condensation reaction, according to the synthetic pathway shown in Scheme 1 and as detailed in Keating and Armstrong, *J. Am. Chem. Soc., 118:*2574-2583 (1996). Appropriately substituted or unsubstituted amino carboxylic acids 1, amines 3, and aldehydes 2 can be used to prepare the compounds of the present invention, wherein R¹, R², R⁵, R⁶, R⁷, R⁸, R⁹ and R¹⁰ are defined above. When R³X is selected as a group other than hydrogen or an electron pair, R³X can be introduced by using R³X-halogen in the presence of a base, such as NaH, and a solvent, such as THF and/or catalyst such as tetrabutylammonium iodide. When R³X is hydrogen, compound 7 can be converted to amidine 8 through the use of phosphorous oxychloride and the appropriately substituted amine.

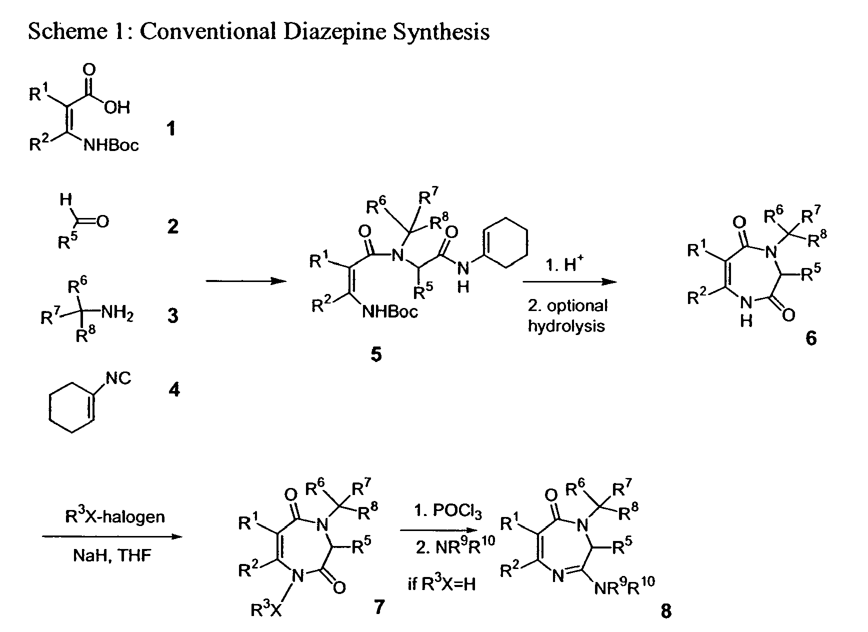

Alternatively, the compounds of the present invention can be prepared utilizing isatoic anhydride chemistry. Optionally-substituted furan-2,5-dione (9) is treated with trimethylsilyl azide to afford isatoic anhydride 10. Compound 10 is then treated with optionally-substituted amino ester in a solvent such as pyridine and heated to 125 °C in a sealed tube for 1.5 h, followed by the treatment with a base such as *t*BuOK, to afford compound 11. Compound 11 is then treated with a base such as sodium hydride followed by an appropriately substituted alkyl halide. The product formed is then treated with lithium *bis*(trimethylsilyl)amide at 0°C in a solvent such as anhydrous DMF. After 10 min., an appropriately substituted alkyl halide is added to afford the final diazepine structure 12. Alternatively, compound 10 is treated with a base, such as NaH, in a solvent, such as DMF, followed by addition of an appropriately substituted alkyl halide. The product 13 formed is then treated with an optionally substituted amino ester in a solvent such as pyridine and heated to 125 °C in a sealed tube for 1.5 h, followed by the treatment with a base such as *t*BuOK, to afford compound 12 and 14.

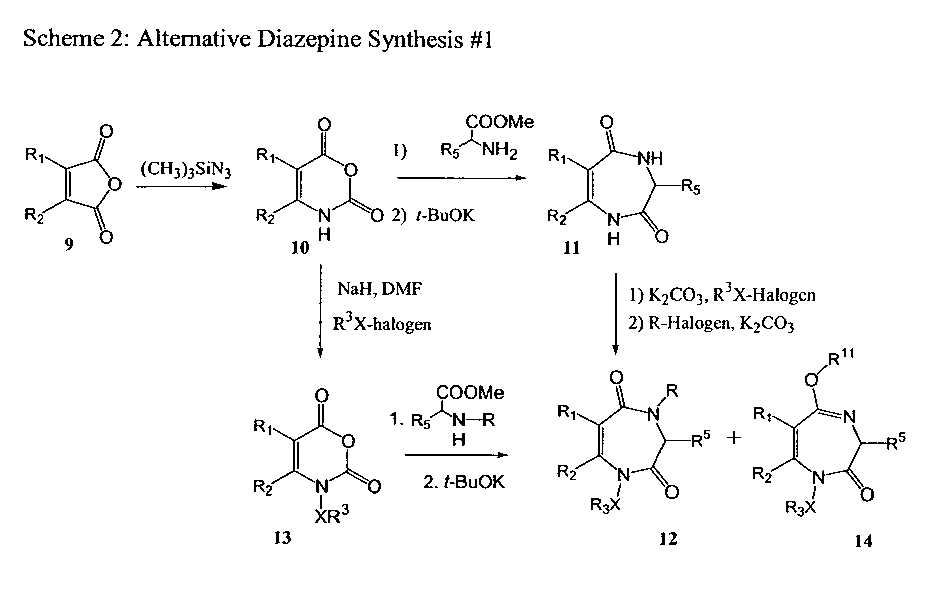

Alternatively, the compounds of the present invention can be prepared starting with an optionally substituted α-haloester. Compound 15, is treated with an optionally substituted amine, in the presence of a base, such as K₂CO₃, in a solvent such as acetonitrile to give compound 16. This can be treated with an optionally substituted α,β-unsaturated acid chloride in the presence of a base, such as diisopropylethylamine, in a solvent such as dichloromethane to give compound 17. This is treated with sodium hydroxide in methanol, followed by coupling with an optionally substituted amine in the presence of coupling reagents such as PyBrOP and HOBt, in a solvent, such as DMF, and a base, such as diisopropylethylamine, to give compound 18. This is subsequently treatment with silver triflate in acetonitrile, followed by addition of benzeneselenyl bromide and DMF. Then, hydrogen peroxide is added in the presence of a solvent, such as THF to give compound **19.** If R³X is hydrogen, the solubilizing group can be introduced using R³X-halogen in the presence of a base, such as NaH, and a solvent, such as DMF.

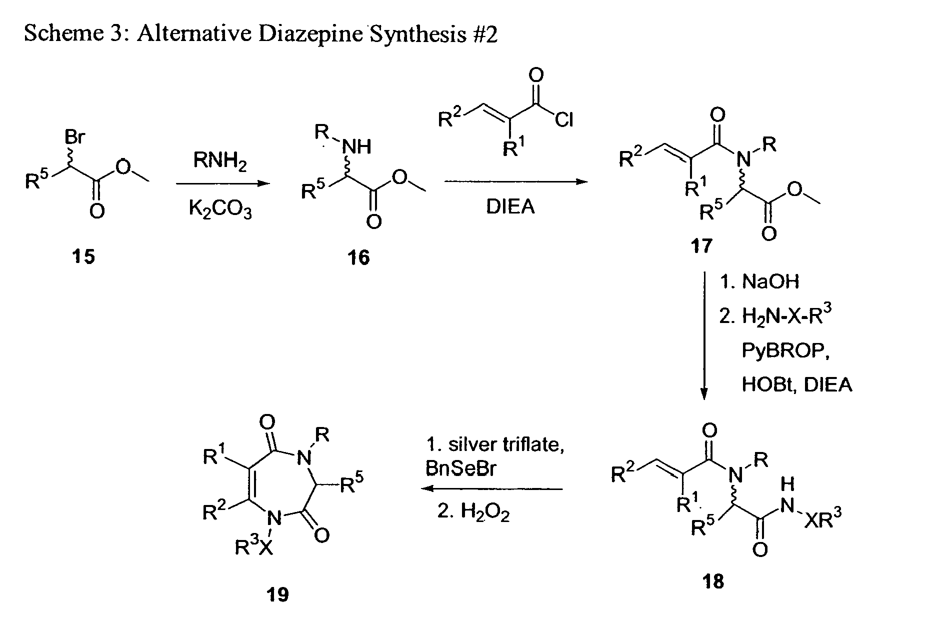

The following examples illustrate, but do not limit, the compounds, methods and compositions of the present invention. Other suitable modifications and adaptations of the variety of conditions and parameters normally encountered in clinical therapy and which are obvious to those skilled in the art are within the spirit and scope of the invention.

### EXAMPLES

The compounds in the examples below were synthesized by the following general procedures.

### General procedure for the conventional synthesis of diazepine compounds

A solution of the aldehyde (0.20 mmol) and amine (0.20 mmol) in methanol (2.0 mL) were stirred at ambient temperature for 30 min. To this solution was added a solution of cyclohexene-1-isonitrile (0.21 mmol) in hexanes, followed by the aryl or heteroaryl amino acid (0.20 mmol). The solution was then stirred for 3 days at ambient temperature. Acetyl chloride (0.2 mL) was added slowly. The solution was then shaken for an additional 3 h and concentrated in vacuo. The residue was purified using pre-packed silica cartridges (methylene chloride to 10 % ethyl acetate in methylene chloride). The residue was purified using pre-packed silica cartridges (8 % ethyl acetate in methylene chloride to 10 % methanol in methylene chloride) to give the title compounds.

### General procedure for the alkylation of diazepines at position 1

Alkylating agent (1.5 equiv) was added to oven dried 2-dram vial equipped with a stir bar under nitrogen. The diazepine to be alkylated (1.0 equiv) was added, and the resulting mixture was dissolved in a solvent, such as DMF. Potassium carbonate (2.0 equiv.) was added, followed by addition of tetrebutylammonium iodide (catalytic). The reaction mixture was tightly capped and heated to 80 °C overnight. The reaction was filtered, concentrated down, and loaded onto a 1000 µm TLC plate. After elution twice with 10% EtOAc/hexanes, the lower band was isolated to give the 1-alkylated compounds.

### General procedure for the synthesis of amidines

A solution of 1,4-benzodiazepine-2,5-dione (0.181 mmol), *4-(N,N* dimethylamino)pyridine (122 mg, 1.0 mmol), and phosphorus oxychloride (350 µL, 1.4 mmol) in dichloroethane (4 mL) was stirred at 135 °C in a sealed tube for 2 h. After the solution was cooled to room temperature, the excess of phosphorus oxychloride was removed under vacuum. Then, the residue was dissolved in dry CH₂Cl₂ (6 mL), and amine (2-10 equiv) was added dropwise with stirring at 0 °C under argon. After 10 min, the reaction mixture was allowed to warm to room temperature. Then, the solvent was evaporated and the residue was chromatographed on silica (EtOAc/hexanes, 1: 1) to give the title compounds as a colorless solid.

### General procedure for the alternative diazepine synthesis #1

A solution of isatoic anhydride (1 mmol) and amino acid ester (1.3 mmol) in pyridine (3 mL) was stirred at 125 °C in a sealed tube for 1.5 h. After cooling to room temperature, the solvent was removed under vacuum. Chromatography on silica (EtOAc/hexanes, 1:1) afforded the appropriately substituted *N*-(2-aminobenzoyl)-2-(aryl)glycine methyl ester, which was dissolved in THF (10 mL). Then, potassium *tert*-butoxide (1M in THF, 1.1 mL) was slowly added, at -78°C under argon atmosphere. The reaction mixture was allowed to warm up to room temperature. After 14 h., the reaction was quenched with acetic acid (150 µL), then the solvent was evaporated to dryness and the residue was diluted with ethyl acetate, washed with water, dried (Na₂SO₄), and concentrated to dryness under reduced pressure. Chromatography on silica (AcOEt/Hexane, 1: 1) afforded the diazepine compound as colorless prisms. Alternatively, isatoic anhydride (10) was treated with a base such as NaH, in a solvent such as DMF at 0°C to room temperature, followed by addition of a substituted halogen derivative to give (13). This was treated with an amino ester (1.5 equiv) at 80-120°C, followed by addition of *t*-BuOK at -20°C to room temperature to give the title compound. General procedure for the alternative diazepine synthesis #2

A solution of α-haloester (10 mmol), an optionally-substituted amine (10 mmol), K₂CO₃ (20 mmol) and TBAI (1.35 mmol) were stirred in acetonitrile at 45°C for 12 hours. The crude mixture was extracted from water with ethyl acetate, followed by chromatography on silica (EtOAc:Hexane 5:95). The product (3.0 mmol) was treated with DIEA (11.5 mmol) in DCM (30 mL), followed by addition of an optionally-substituted α,β-unsaturated amine (3.25 mmol) at 0°C. The crude reaction was extracted from water with EtOAc, followed by chromatography on silica (Hexane: EtOAc 8:1) to give the corresponding product. This product (1.0 mmol) was saponified with NaOH (15.0 mmol) in THF and methanol at room temperature, followed by extraction from 1N HCl with EtOAc. The acid (0.88 mmol) was then treated with ammonium chloride (1.76 mmol), PyBrOP (1.32 mmol), HOBt (1.32 mmol) and DIEA (3.52 mmol) in DMF (3 mL) at room temperature and stirred for 2 hours. The crude mixture was extracted from water with EtOAc and purified by chromatography on silica (AcOEt: Hexane 1:3). The resulting amide (3.3 mmol) was stirred with silver triflate (6.6 mmol) in acetonitrile (120 mL) at room temperature. Benzeneselenyl bromide (6.53 mmol) was added in DMF (5.2 mL) and stirred for 2 hours. The crude residue was extracted from water with EtOAc and purified by chromatography (AcOEt:hexane 1:3). The corresponding product (0.49 mmol) was treated with hydrogen peroxide (500 µL, 30% in water) in THF at room temperature and stirred for 20 minutes. The crude mixture was extracted from 1N sodium hydrogencarbonate with EtOAc, followed by chromatography on silca (AcOEt:hexanes:DCM 1:2:2) to give the title compound.

### General procedure for the dialkylation of diazepines

A base, such as potassium carbonate (0.88 mmol) was added, at 20°C to a stirred solution of diazepine (11) (0.82 mmol) in anhydrous DMF (4 mL). After 10 min., optionally-substituted alkyl halide (0.96 mmol) was added and stirring was continued for 1-12 h. at 25-70°C. Then, the reaction was diluted with ice-cold water (30 mL), and the residue filtered, washed with water, then dried in vacuo. Next, a base, such as K₂CO₃ (1.1 equiv) was added at 0°C to this product in anhydrous solvent, such as DMF (2 mL). After 10 min., optionally-substituted alkyl halide (0.32 mmol) was added and stirring was continued for 5-24 h. at 25-80°C. Then, the reaction was quenched with acetic acid (50 µL). The crude material was purified by column chromatography on silica (EtOAc/hexane, 1.1). Recrystallization from ethanol/ether yielded the compound 12 as colorless crystals.

### EXAMPLE 1

5-{(3*S*)-3-(4-Chlorophenyl)-4-[(*R*)-1-(4-chlorophenyl)-ethyl]-7-iodo-2,5-dioxo-2,3,4,5-tetrahydro-benzo[e] [1,4]diazepin-1-yl} -pentanoic acid 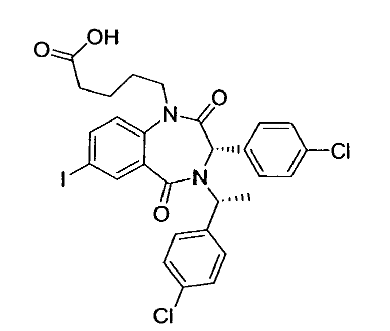
a) 5-{(3*S*)-3-(4-Chlorophenyl)-4-[(*R*)-1-(4-chlorophenyl)-ethyl]-7-iodo-2,5-dioxo-2,3,4,5-tetrahydro-benzo[e][1,4]diazepin-1-yl}-pentanoic acid tert-butyl ester
   The title compound was synthesized following the general procedure for the conventional synthesis of diazepine compounds followed by the general procedure for the alkylation of diazepines at position 1: Mass spectrum (LCMS, ESI pos.): Calcd for C₃₂H₃₃Cl₂IN₂O₄: 706.09; found 707.1 (M+H).
b) 5-{(3*S*)-3-(4-Chlorophenyl)-4-[(*R*)-1-(4-chlorophenyl)-ethyl]-7-iodo-2,5-dioxo-2,3,4,5-tetrahydro-benzo[e][1,4]diazepin-1-yl} -pentanoic acid
   5-{(3*S*)-3-(4-Chloro-phenyl)-4-[(*R*)-1-(4-chloro-phenyl)-ethyl]-7-iodo-2,5-dioxo-2,3,4,5-tetrahydro-benzo[e][1,4]diazepin-1-yl}-pentanoic acid tert-butyl ester (1.0 g, 1.4 mmol) was dissolved in a solution of 20% TFA [10 mL] in dichloromethane at room temperature for 2 hours. The reaction was concentrated and the product was purified by chromatography (hexane: EtOAc 1:1) to give the title compound (0.88 g, 96 %).¹H NMR (400 MHz, DMSO-d₆) δ 7.75 (d, J = 2.3 Hz. 1H), 7.62-7.56 (m, 3H), 7.39 (d, *J =* 8.6 Hz, 2H), 7.00 (d, J =8.6 Hz, 2H), 6.92 (d, J =8.6 Hz, 1H), 6.58-6.56 (m, 2H), 6.21-6.14 (m, 1H), 5.27 (s, 1H), 4.24-4.15 (m, 1 H), 3.69-3.61 (m, 1H), 1.80 (t, *J =* 7.2 Hz, 2H), 1.63 (d, J = 7.2 Hz, 3H), 1.56-1.21 (m, 4H). Mass spectrum (LCMS, ESI pos.): Calcd for C₂₈H₂₅Cl₂IN₂O₄: 650.02; found 650.8(M+H).

### EXAMPLE 2

5-{(3*S*)-3-(4-Chlorophenyl)-4-[(*R*)-1-(4-chlorophenyl)-ethyl]-7-ethynyl-2,5-dioxo-2,3,4,5-tetrahydro-benzo[e][1,4]diazepin-1-yl}-pentanoic acid 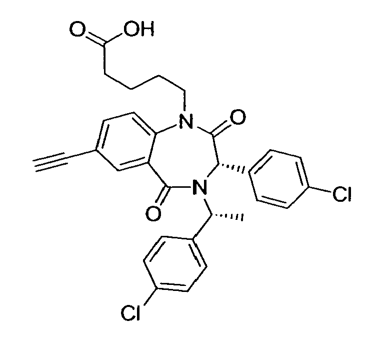 ¹H NMR (400 MHZ, DMSO-D₆) δ 7.60 (D, *J*= 8.6 HZ, 2H), 7.53 (D, J= 2.1 HZ, 1H), 7.42-7.33 (M, 3H), 7.12 (D, *J =* 8.6 HZ, 1H), 6.99 (D, J = 8.4 HZ, 2H), 6.57 (D*, J =* 7.7 HZ, 2H), 6.23-6.15 (M, 1H), 5.29 (S, 1H), 4.31-4.22 (M, 2H), 3.78-3.68 (M, 1H), 2.20-2.12 (M, 2H), 1.63 (D, J= 7.0 HZ, 3H), 1.41-1.38 (M, 2H), 1.27-1.22 (M, 2H). MASS SPECTRUM (LCMS, ESI POS.): CALCD FOR C₃₀H₂₆CL₂N₂O₄: 548.13; FOUND 548.9 (M+H). EXAMPLE 3

5-[4-[(*R*)-1-(4-Chlorophenyl)-ethyl]-7-iodo-2,5-dioxo-(3*S*)-3-(4-trifluoromethyl-phenyl)-2,3,4,5-tetrahydro-benzo[e][1,4]diazepin-1-yl]-pentanoic acid 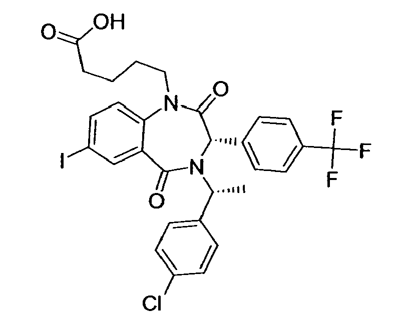 ¹H NMR (400 MHz, CDCl₃) δ 7.94 (d, *J* = 2.1 Hz, 1H), 7.50 (d, *J* = 8.4 Hz, 2H), 7.44 (dd, *J* = 8.6 Hz, 2.1 Hz, 1H), 7.33 (d, *J* = 8.6 Hz, 2H), 7.18 (d, *J* = 8.4 Hz, 2H), 6.68 (d, J = 8.4 Hz, 2H), 6.57 (d, J = 8.6 Hz, 2H), 6.46-6.39 (m, 1H), 5.39 (s, 1H), 4.39-4.31 (m, 1H), 3.70-3.62 (m, 1H), 2.37 (t, J = 7.0 Hz, 2H), 1.75 (d, *J=* 7.2 Hz, 3H), 1.67-1.58 (m, 4H). Mass spectrum (LCMS, ESI pos.): Calcd for C₂₉H₂₅ClF₃IN₂O₄: 684.05; found 684.8 (M+H).

### EXAMPLE 4

5-[4-[(*R*)-1-(4-Chlorophenyl)-ethyl]-7-iodo-2,5-dioxo-(3*S*)-3-(4-trifluoromethoxy-phenyl)-2,3,4,5-tetrahydro-benzo[e][1,4]diazepin-1-yl]-pentanoic acid 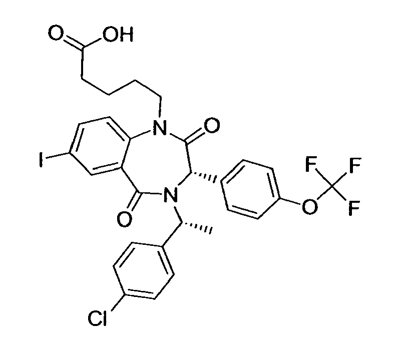 ¹H NMR (400 MHz, CDCl₃) δ 7.89 (d, J = 2.1 Hz, 1H), 7.48 (d, J = 8.4 Hz, 2H), 7.43 (dd, J = 8.6 Hz, 2.1 Hz, 1H), 7.32 (d, J = 8.6 Hz, 2H), 6.75 (d, J = 8.4 Hz, 2H), 6.59-6.53 (m, 3H), 6.42-6.35 (m, 1H), 5.36 (s, 1H), 4.41-4.31 (m, 1H), 3.67-3.57 (m, 1H), 2.37 (t, J = 7.0 Hz, 2H), 1.73 (d, J = 7.2 Hz, 3H), 1.67-1.52 (m, 4H). Mass spectrum (LCMS, ESI pos.): Calcd for C₂₉H₂₅ClF₃IN₂O₅: 700.04; found 700.86 (M+H).

### EXAMPLE 5

6- {(3*S*)-3-(4-Chlorophenyl)-4-[(*R*)-1-(4-chlorophenyl)-ethyl]-7-iodo-2,5-dioxo-2,3,4,5-tetrahydro-benzo[e][1,4]diazepin-1-yl}-hexanoic acid 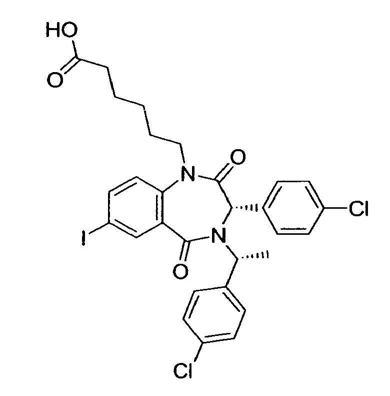 ¹H NMR (400 MHz, DMSO-d₆) δ 7.78-7.74 (m, 1H), 7.62-7.55 (m, 2H), 7.42-7.39 (m, 3H), 7.02-6.99 (m, 2H), 6.93 (d, *J* = 8.6 2H), 6.58 (d, 7.7 Hz, 2H), 6.28-6.14 (m, 1 H), 5.28 (s, 1H), 4.29-4.19 (m, 1H), 3.71-3.62 (m, 1H), 2.15-2.07 (m, 2H), 1.65-1.58 (m, 3H), 1.50-0.96 (m, 6H). Mass spectrum (LCMS, ESI pos.): Calcd for C₂₉H₂₇Cl₂IN₂O₄: 664.04; found 664.8 (M+H).

### EXAMPLE 6

7-{(3*S*)-3-(4-Chlorophenyl)-4-[(*R*)-1-(4-chlorophenyl)-ethyl]-7-iodo-2,5-dioxo-2,3,4,5-tetrahydro-benzo[e][1,4]diazepin-1-yl}-heptanoic acid 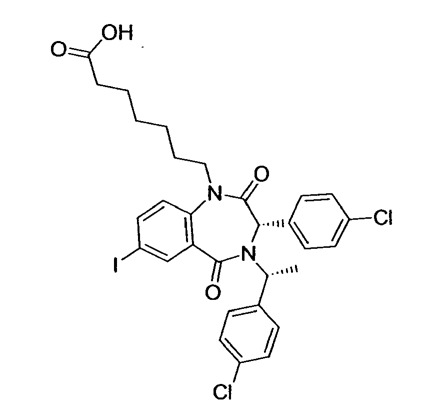 ¹H NMR (400 MHz, DMSO-d₆) δ 7.77-7.75 (m, 1H), 7.63-7.55 (m, 2 H), 7.45-7.37 (m, 3 H), 7.03-6.98 (m, 2H), 6.93-6.83 (m, 1H), 6.58 (d, J= 8.6 Hz, 2H), 6.28-6.13 (m, 1H), 5.28 (s, 1H), 4.30-4.23 (m, 1H), 3.69-3.62 (m, 1H), 2.14 (t, J = 7.44 Hz, 2H), 1.64-1.59 (m, 3H), 1.54-0.97 (m, 8H). Mass spectrum (LCMS, ESI pos.): Calcd for C₃₀H₂₉Cl₂IN₂O₄: 678.05; found 678.9 (M+H).

### EXAMPLE 7

4-{(3*S*)-3-(4-Chlorophenyl)-4-[(R)-1-(4-chlorophenyl)-ethyl]-7-iodo-2,5-dioxo-2,3,4,5-tetrahydro-benzo[e][1,4]diazepin-1-ylmethyl}-benzoic acid 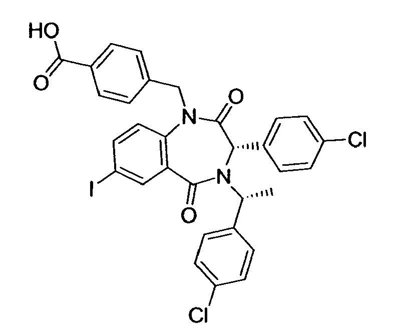 ¹H NMR (400 MHz, DMSO-d₆) δ 8.12-7.91 (m, 3H), 7.47-7.37 (m, 3H), 7.33-7.24 (m, 3H), 7.18-7.02 (m, 1H), 6.97-6.85 (m, 2H), 6.61-6.50 (m, 3H), 6.45-6.33 (m, 1H),5.37(s, 1H), 5.35 (d, *J*=15.6 Hz, 1H), 4.99 (d, *J*=15.8 Hz, 1H), 1.64 (d, *J* = 7.2 Hz, 3H). Mass spectrum (LCMS, ESI pos.): Calcd for C₃₁H₂₃Cl₂IN₂O₄: 684.01; found 684.7 (M+H).

### EXAMPLE 8

4-{(3*S*)-3-(4-Chlorophenyl)-4-[(*R*)-1-(4-chlorophenyl)-ethyl]-7-iodo-2,5-dioxo-2,3,4,5-tetrahydro-benzo[e][1,4]diazepin-1-yl}-butyric acid 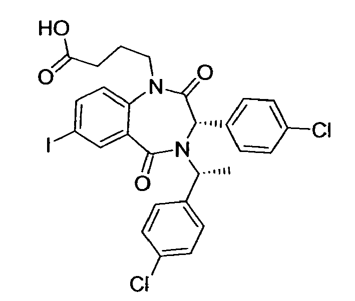 ¹H NMR (400 MHz, DMSO-d₆) δ 7.76, (d, J= 2.1 Hz, 1H), 7.61-7.58 (m, 2H), 7.41-7.39 (m, 2H), 7.00 (d, J = 8.8 Hz, 2H), 6.94-6.86 (m, 1H), 6.60-6.50 (m, 2H), 6.29-6.14 (m, 1H), 5.29 (s, 1H), 4.30-4.20 (m, 1H), 3.75-3.66 (m, 1H), 2.13 (t, *J* = 7.2 Hz, 2H), 1.85-1.70 (m, 1H), 1.65 (d, J = 7.21, 3H), 1.64-1.54 (m, 2H). Mass spectrum (LCMS, ESI pos.): Calcd for C₂₇H₂₃Cl₂IN₂O₄: 636.01; found 636.8 (M+H).

### EXAMPLE 9

3- {(3*S*)-3-(4-Chlorophenyl)-4-[*(R)*-1*-*(4-chlorophenyl)-ethyl]-7-iodo-2,5-dioxo-2,3,4,5-tetrahydro-benzo[e][1,4]diazepin-1-yl}-propionic acid 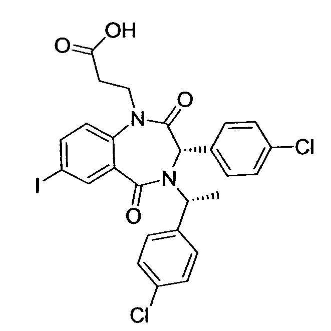 ¹H NMR (400 MHz, DMSO-d₆) δ 7.73 (d, J= 2.1 Hz, 1H), 7.64-7.35 (m, 5H), 7.21-6.96 (m, 2H), 6.97-6.49 (m, 3H), 6.28-6.14 (m, 1H), 5.25 (s, 1H), 4.51-4.31 (m, 1H), 3.94-3.80 (m, 1H), 2.44-2.29 (m, 1H), 1.91 (s, 1H), 1.68-1.57 (m, 3H). Mass spectrum (LCMS, ESI pos.): Calcd for C₂₆H₂₁Cl₂IN₂O₄: 621.99; found 622.8 (M+H).

### EXAMPLE 10

5-{(3,*S*)-3-(4-Chlorophenyl)-4-[(R)-1-(4-chlorophenyl)-ethyl]-7-iodo-2,5-dioxo-2,3,4,5-tetrahydro-benzo[e] [1,4]diazepin-1-ylmethyl }-furan-2-carboxylic acid 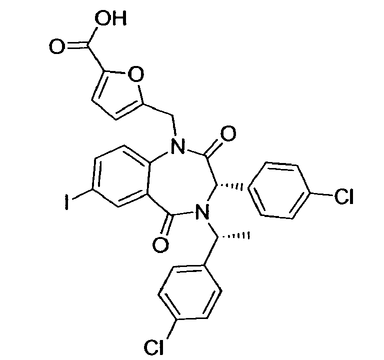 ¹H NMR (400 MHz, CDCl₃) δ 8.04-7.85 (m, 1H), 7.58 (d, *J* = 8.4 Hz, 1H), 7.70-7.44 (m, 2H), 7.39-7.30 (m, 3H), 7.10 (d, *J =* 7.7 Hz, 1H), 6.91 (d, *J* = 8.1 Hz, 2H), 6.74 (d, *J* = 8.6 Hz, 1H), 6.54 (d, *J* = 3.2 Hz, 1H), 6.47 (d, *J* = 8.1 Hz, 2H), 5.55 (s, 1H), 5.27-4.90 (m, 2H), 1.82-1.66 (m, 3H). Mass spectrum (LCMS, ESI pos.): Calcd for C₂₉H₂₁Cl₂IN₂O₅: 673.99; found 674.8 (M+H).

### EXAMPLE 11

5-{(3*S*)-8-Chloro3-(4-chloro-phenyl)-4-[(*R*)-1-(4-chlorophenyl)-ethyl]-7-iodo-2,5-dioxo-2,3,4,5-tetrahydro-benzo[e][1,4]diazepin-1-yl}-pentanoic acid 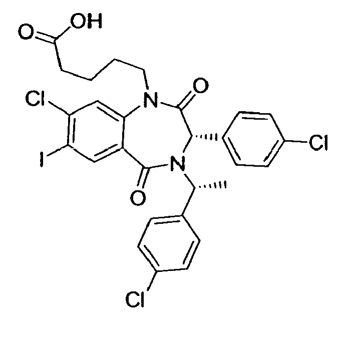 ¹H NMR (400 MHz, CDCl₃) δ 8.08 (s, 1H), 7.46 (d, J = 8.4 Hz, 2H), 7.30 (d, *J* = 8.4 Hz, 2H), 6.95-6.88 (m, 3H), 6.45 (d, *J* = 7.7 Hz, 2H), 6.40-6.34 (m, 1H), 5.33 (s, 1H), 4.39-4.27 (m, 1H), 3.69-3.59 (m, 1H), 2.45-2.34 (m, 2H), 1.70 (d, J = 7.2 Hz, 3H), 1.67-1.52 (m, 4H). Mass spectrum (LCMS, ESI pos.): Calcd for C₂₈H₂₄Cl₃IN₂O₄: 683.98; found 684.8 (M+H).

### EXAMPLE 12

5- {3-(4-Chlorophenyl)-4-[(4-chlorophenyl)-cyclopropyl-methyl]-7-iodo-2,5-dioxo-2,3,4,5-tetrahydro-benzo[e][1,4]diazepin-1-yl}-pentanoic acid 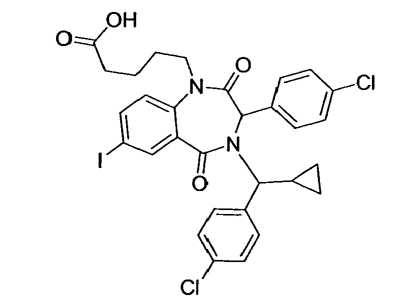 ¹H NMR (400 MHz, DMSO-d₆) δ 7.74 (d, J = 2.1 Hz, 1H), 7.66 (d, J = 8.4 Hz, 2H), 7.59 (dd, J = 8.6 Hz, 2.1 Hz, 1H), 7.40 (d, J= 8.6 Hz, 2H), 7.01 (d, J = 8.8 Hz, 2H), 6.92 (d, J = 8.8 Hz, 1H), 6.59 (dd, *J =* 8.6 Hz, 0.93 Hz, 2H), 5.51 (s, 1H), 5.16 (d, J = 10.7 Hz, 1H), 4.38-4.29 (m, 1H), 3.71-3.62 (m, 1H), 2.19-2.11 (m, 2H), 1.91-1.81 (m, 1H), 1.54-1.21 (m, 4H), 0.81-0.71 (m, 1H), 0.62-0.52 (m, 1H), 0.48-0.34 (m, 2H). Mass spectrum (LCMS, ESI pos.): Calcd for C₃₀H₂₇Cl₂IN₂O₄: 676.04; found 677.0 (M+H).

### EXAMPLE 13

5-{3-(4-Chlorophenyl)-4-[1-(3,4-dichlorophenyl)-ethyl]-7-iodo-2,5-dioxo-2,3,4,5-tetrahydro-benzo[e][1,4]diazepin-1-yl}-pentanoic acid 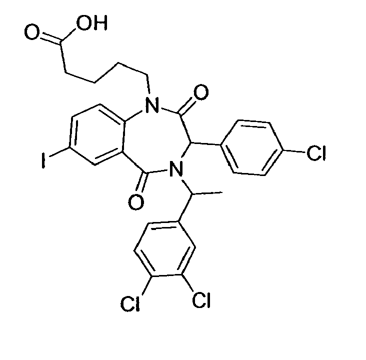 ¹H NMR (400 MHz, DMSO-d₆) δ 7.85-7.76 (m, 2H), 7.63-7.55 (m, 3.0H), 7.02 (d, J = 8.6 Hz, 2H), 6.90 (d, J = 8.6 Hz, 1H), 6.63 (d, J = 8.6 Hz, 2H), 6.13-6.06 (m, 1H), 5.38 (s, 1H), 4.31 (m, 1H), 3.72-3.64 (m, 1H), 2.18-2.11 (m, 2H), 1.65 (d, *J =* 6.9 Hz, 3H), 1.55-1.43 (m, 1H), 1.48-1.28 (m, 3H). Mass spectrum (LCMS, ESI pos.): Calcd for C₂₈H₂₄Cl₃IN₂O₄: 683.98; found 684.8 (M+H).

### EXAMPLE 14

S-[4-[1-(3-Amino-4-chlorophenyl)-ethyl]-3-(4-chlorophenyl)-7-iodo-2,5-dioxo-2,3,4,5-tetrahydro-benzo[e][1,4]diazepin-1-yl]-pentanoic acid 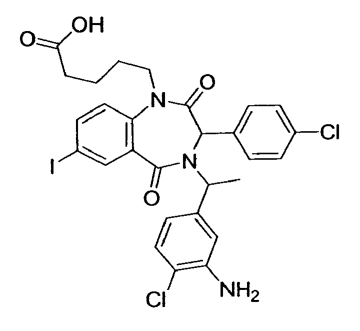 ¹H NMR (400 MHz, DMSO-d₆) δ 7.74 (d, *J =* 2.1 Hz, 1H), 7.59 (dd, *J =* 8.6 Hz, 2.1 Hz, 1H), 7.12 (d, *J* = 8.1 Hz, 1H), 7.03-6.98 (m, 3H), 6.92 (d, *J =* 8.6 Hz, 2H), 6.70 (dd, J = 8.6 Hz, 2.1 Hz, 2H), 6.63 (d, J = 8.6 Hz, 2H), 6.13-6.04 (m, 1H), 5.41 (s, 2H), 5.19 (s, 1H), 4.33-4.22 (m, 1H), 3.73-3.63 (m, 1H), 2.19-2.12 (m, 2H), 1.57-1.45 (m, 3H), 1.44-1.31 (m, 3H). Mass spectrum (LCMS, ESI pos.): Calcd for C₂₈H₂₆Cl₂IN₃O₄: 665.03; found 665.8 (M+H).

### EXAMPLE 15

5-[4-[1-(4-Chloro-3-nitro-phenyl)-ethyl]-3-(4-chlorophenyl)-7-iodo-2,5-dioxo-2,3,4,5-tetrahydro-benzo[e] [,4]diazepin-1-yl]-pentanoic acid 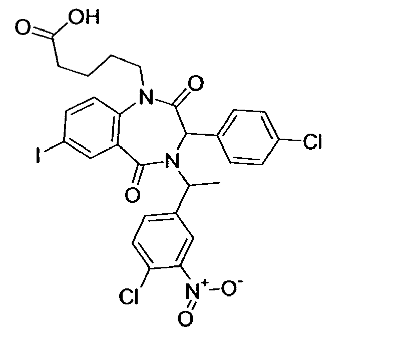 ¹H NMR (400 MHz, DMSO-d₆) δ 8.24 (d, J = 2.1 Hz, 1H), 7.90 (dd, J = 8.6 Hz, 2.3 Hz, 1H), 7.78 (d, *J=* 2.1 Hz, 1H), 7.73 (d, *J=* 8.4 Hz, 1H), 7.58 (dd, *J* = 8.6 Hz, 2.1 Hz, 1H), 7.01 (d,*J*=8.6Hz,2H),6.90(d,*J*=8.8Hz, 1H), 6.66-6.63 (m, 2H), 6.20-6.13 (m, 1H), 5.43 (s, 1H), 4.29-4.20 (m, 1H), 3.72-3.63 (m, 1H), 2.19-2.11 (m, 2H), 1.69 (d, J = 7.2 Hz, 3H), 1.53-1.44 (m, 1H), 1.41-1.32 (m, 3H). Mass spectrum (LCMS, ESI pos.): Calcd for C₂₈H₂₄ChlN₃O₆: 695.01; found 695.8 (M+H).

### EXAMPLE 16

5-{3-(4-Chlorophenyl)-7-iodo-4-[1-(4-methyl-naphthalen-1-yl)-ethyl]-2,5-dioxo-2,3,4,5-tetrahydro-benzo[e][1,4]diazepin-1-yl}-pentanoic acid 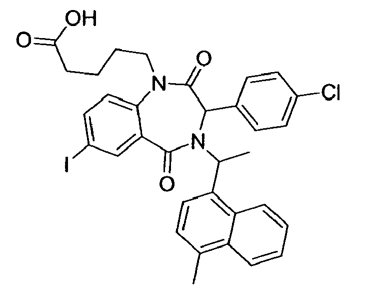 ¹H NMR (400 MHz, DMSO-d₆) δ 8.39 (d, J = 8.8 Hz, 1H), 8.06 (d, J = 8.4 Hz, 1H), 7.87-7.76 (m, 2H), 7.70-7.55 (m, 3H), 7.35 (d, J = 8.1 Hz, 1H), 6.90 (d, J = 8.6 Hz, 2H), 6.64 (d, J= 8.6 Hz, 2H), 6.18-6.13 (m, 2H), 5.18 (s, 1H), 4.37-4.27 (m, 1H), 3.73-3.64 (m, 1H), 2.58 (s, 3H), 2.23-2.15 (m, 2H), 1.75 (d, J = 7.0 Hz, 3H), 1.54-1.30 (m, 4H). Mass spectrum (LCMS, ESI pos.): Calcd for C₃₃H₃₀ClIN₂O₄: 680.09; found 680.8 (M+H).

### EXAMPLE 17

5-[4-(4-Chlorobenzyl)-3-(4-chlorophenyl)-7-iodo-2,5-dioxo-2,3,4,5-tetrahydro-benzo[e][1,4]diazepin-1-yl]-pentanoic acid 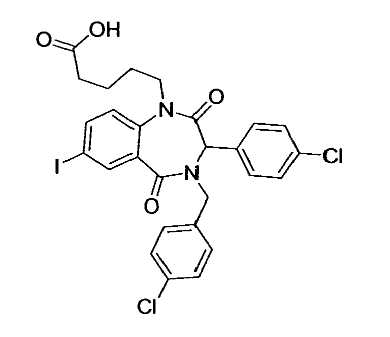 ¹H NMR (400 MHz, DMSO-d₆) δ 12.05 (bs, 1H), 7.71 (d, *J =* 2.1 Hz, 1H), 7.62 (dd, *J =* 8.8 Hz, 2.3 Hz, 1H), 7.43 (m, 4H), 7.17 (d, *J =* 8.6 Hz, 2H), 6.95 (d, *J =* 8.8 Hz, 1H), 6.83 (d, *J =* 7.7 Hz, 2H), 5.70 (s, 1H), 5.27 (d, *J =* 14.6 Hz, 1H), 4.69 (d, *J =* 14.4 Hz, 1H), 4.21-4.13 (m, 1H), 3.67-3.58 (m, 1H), 2.11 (t, J = 7.0 Hz, 2H), 1.32-1.22 (m, 2H), 0.88-0.83 (m, 2H). Mass spectrum (LCMS, ESI pos.): Calcd for C₂₇H₂₃Cl₂IN₂O₄₁ 636.01; found 637.0 (M+H).

### EXAMPLE 18

5-{3-(4-Chlorophenyl)-4-[2-(4-chlorophenyl)-1-methyl-ethyl]-7-iodo-2,5-dioxo-2,3,4,5-tetrahydro-benzo[e][1,4]diazepin-1-yl}-pentanoicacid 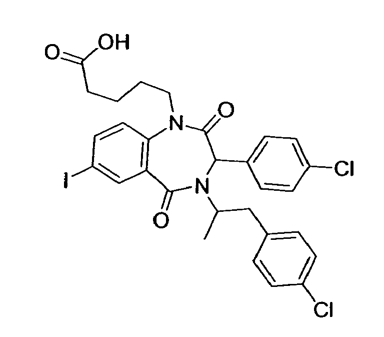 ¹H NMR (400 MHz, DMSO-d₆) δ 7.71-7.65 (m, 1H), 7.60-7.53 (m, 1H), 7.39-7.24 (m, 4H), 7.17-7.04 (m, 2H), 6.92-6.71 (m, 3H), 5.74 (s, 1H), 5.34-5.14 (m, 1H), 4.25-3.99 (m, 1H), 3.70-3.59 (m, 1H), 3.12 (m, 1H), 2.98-2.86 (m, 1H), 2.18-2.09 (m, 2H), 1.52-1.13 (m, 7H). Mass spectrum (LCMS, ESI pos.): Calcd for C₂₉H₂₇Cl₂IN₂O₄: 664.04; found 664.9 (M+H).

### EXAMPLE 19

5-{(3*S*)-3-(3-Bromo-phenyl)-4-[(*R*)-1-(4-chloro-phenyl)-ethyl]-7-iodo-2,5-dioxo-2,3,4,5-tetrahydro-benzo[e][1,4]diazepin-1-yl}-pentanoic acid 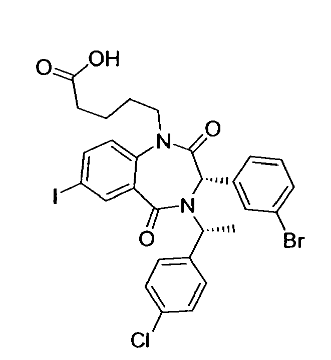 ¹H NMR (400 MHz, DMSO-d₆) δ 7.95 (d, J= 2.1 Hz, 1H), 7.51-7.44 (m, 3H), 7.37-7.29 (m, 2H), 7.08-7.03 (m, 1H), 6.81-6.71 (m, 1H), 6.65-6.61 (m, 1H), 6.59 (d, *J* = 8.8 Hz, 1H), 6.48-6.42 (m, 1H), 6.43-6.35 (m, 1H), 5.33 (s, 1H), 4.41-4.32 (m, 1H), 3.67-3.57 (m, 1H), 2.40-2.34 (m, 2H), 1.72 (d, *J* = 7.2 Hz, 3H), 1.67-1.54 (m, 4H). Mass spectrum (LCMS, ESI pos.): Calcd for C₂₈H₂₅BrClIN₂O₄: 695.78; found 696.8 (M+H).

### EXAMPLE 20

[4-Benzyl-3-(4-chlorophenyl)-7-iodo-2,5-dioxo-2,3,4,5-tetrahydro-benzo[e][1,4]diazepin-1-yl]-acetic acid 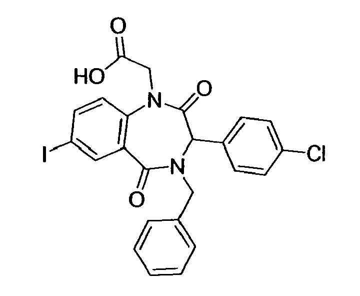 ¹H NMR (400 MHz, CD₃OD) δ 7.80 (d, *J* = 2.1 Hz, 1H), 7.58-7.47 (m, 3H), 7.42-7.28 (m, 3H), 6.99 (s, 4H), 6.89 (d, J = 8.8 Hz, 1H), 5.51 (s, 1H), 5.49 (s, 1H), 5.18 (d, *J =* 14.4 Hz, 1H), 4.57 (d, *J =* 17.0 Hz, 1H), 4.13 (d, J = 17.2 Hz, 1H). Mass spectrum (LCMS, ESI pos.): Calcd for C₂₄H₁₈ClIN₂O₄: 560.00; found 561.1 (M+H).

### EXAMPLE 21

5-(3*S*)-3-(4-Chloro-3-nitro-phenyl)-4-[(R)-1-(4-chlorophenyl)-ethyl]-7-iodo-2,5-dioxo-2,3,4,5-tetrahydro-benzo[e][1,4]diazepin-1-yl} -pentanoic acid 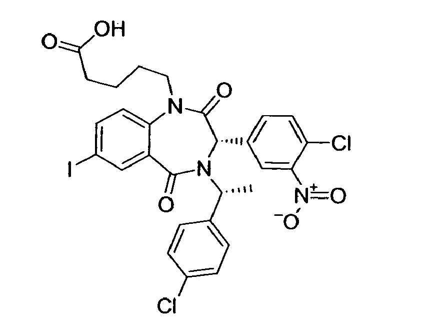 ¹H NMR (400 MHz, CDCl₃) δ 8.06-7.96 (m, 1H), 7.55-7.44 (m, 3H), 7.39-7.28 (m, 3H), 7.13-7.04 (m, 2H), 6.70-6.54 (m, 1H), 6.46-6.36 (m, 1H), 5.30 (s, 1H), 4.40-4.07 (m, 1H), 3.70-3.38 (m, 1H), 2.45-2.24 (m, 2H), 1.98-1.77 (m, 1H), 1.72 (d, *J=* 7.0 Hz, 3H), 1.64-1.54 (m, 3H). Mass spectrum (LCMS, ESI pos.): Calcd for C₂₈H₂₄Cl₂IN₃O₆: 695.01; found 695.7 (M+H).

### EXAMPLE 22

5-{(3*S*)-7-Acetyl-3-(4-chlorophenyl)-4-[(*R*)-1-(4-chlorophenyl)-ethyl]-2,5-dioxo-2,3,4,5-tetrahydro-benzo[e][1,4]diazepin-1-yl}-pentanoic acid 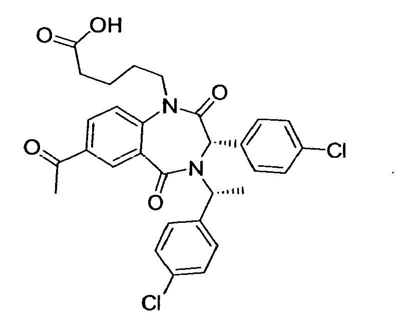 ¹H NMR (400 MHz, DMSO-d₆) δ 8.05 (d, *J* = 2.3 Hz, 1H), 7.79 (dd, *J* = 8.6 Hz, 2.3 Hz, 1H), 7.62 (d, J = 8.6 Hz, 2H), 7.41 (m, 2H), 7.25 (d, J = 8.8 Hz, 1H), 6.96 (d, J = 8.6 Hz, 2H), 6.59 (d, J = 8.6 Hz, 2H), 6.21 (m, 1H), 5.35 (s, 1H), 4.36-4.26 (m, 1H), 3.84-3.74 (m, 1H), 2.21-2.13 (m, 2H), 1.66 (d, *J =* 7.2 Hz, 3H), 1.46-1.33 (m, 3H), 1.30-1.20 (m, 4H). Mass spectrum (LCMS, ESI pos.): Calcd for C₃₀H₂₈Cl₂N₂O₅: 566.14; found 566.9 (M+H).

### EXAMPLE 23

5-{(3*S*)-3-(3-Amino-4-chloro-phenyl)-4-[(*R*)-1-(4-chloro-phenyl)-ethyl]-7-iodo-2,5-dioxo-2,3,4,5-tetrahydro-benzo[e][1,4]diazepin-1-yl}-pentanoic acid

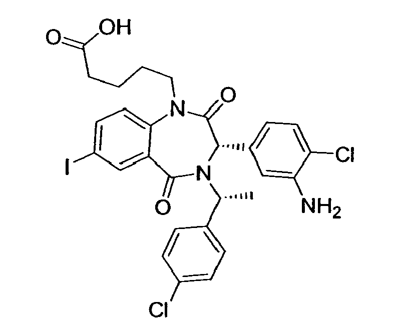

Cl
¹H NMR (400 MHz, CDCl₃) δ 8.13-7.84 (m, 1H), 7.61-7.27 (m, 4H), 7.01-6.81 (m, 1H), 6.76-6.64 (m, 1H), 6.66-5.63 (m, 5H), 5.38-5.10 (m, 2H), 4.41-3.89 (m, 2H), 3.62-3.27 (m, 3H), 1.79-1.32 (m, 6H). Mass spectrum (LCMS, ESI pos.): Calcd for C₂₈H₂₆Cl₂IN₃O₄: 665.03; found 665.8 (M+H).

### EXAMPLE 24

(3*S*)-2-Amino-3-(4-chlorophenyl)-4-[(*R*)-1-(4-chlorophenyl)-ethyl]-7-iodo-3,4-dihydro-benzo[e][1,4]diazepin-5-one 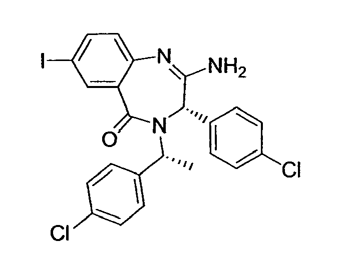 ¹H NMR (400 MHZ, CDCL₃) δ 1.76 (D, *J* = 5.5 HZ, 3H, CH₃), 5.10 (BR S, 1H, CH), 6.46 (Q, *J* = 5.5 HZ, 1H, CH), 6.53 (D, *J* = 6.3 HZ, 1H, ARH), 6.58 (D, *J*= 6.0 HZ, 2H, ARH), 6.91 (D, J= 6.6 HZ, 2H, ARH), 7.33 (D, *J =* 6.6 HZ, 2H, ARH), 7.41 (DD, *J =* 6.3 HZ, *J =* 1.5 HZ, 1H, ARH), 7.53 (D, *J*= 6.6 HZ, 2H, ARH), 8.03 (D, *J* = 1.5 HZ, 1H, ARH). MASS SPECTRUM (LCMS, ESI POS.): CALCD FOR C₂₃H₁₈CL₂IN₃O: 548.99; FOUND 551.0 (M+H).

### EXAMPLE 25

2-{(3*S*)-3-(4-Chlorophenyl)-4-[(*R*)-1-(4-chlorophenyl)-ethyl]-7-iodo-5-oxo-4,5-dihydro-3*H*-benzo[e][1,4]diazepin-2-ylamino}-acetamide 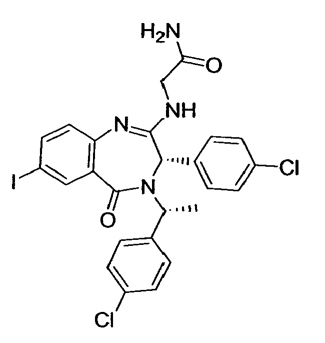 ¹H NMR (400 MHz, CDCl₃) δ 1.76 (d, J = 5.4 Hz, 3H, CH₃), 4.26-4.40 (m, 2H, CH₂), 5.09 (br s, 1H, CH), 5.68 (br s, 1H, NH), 6.30 (br s, 1H, NH), 6.44 (q, *J =* 5.4 Hz, 1H, CH), 6.53-6.58 (m, 3H, ArH), 6.87 (d, J = 6.3 Hz, 2H, CH₂), 7.31-7.40 (m, 3H, ArH), 7.53 (d, J= 6.3 Hz, 2H, ArH), 8.00 (d, J= 1.5 Hz, 1H, ArH). Mass spectrum (LCMS, ESI pos.): Calcd for C₂₅H₂₁Cl₂IN₄O₂: 606.01; found 607.0 (M+H).

### EXAMPLE 26

(3,*S*')-3-(4-Chloro-phenyl)-4-[(*R*)-1-(4-chloro-phenyl)-ethyl]-2-(2-hydroxyethylamino)-7-iodo-3,4-dihydro-benzo[e][1,4]diazepin-5-one 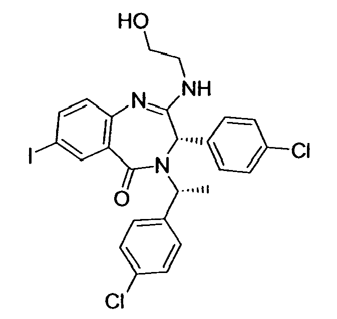 ¹H NMR (400 MHz, CDCl₃) δ 1.71 (d, J = 5.4 Hz, 3H, CH₃), 3.76-3.79 (m, 2H, CH₂), 3.95-3.98 (m, 2H, CH₂), 4.96 (s, 1H, CH), 6.43 (q, *J =* 5.4 Hz, 1H, CH), 6.54-6.56 (m, 3H, ArH), 6.89 (d, *J* = 6.6 Hz, 2H, ArH), 7.31 (d, J = 6.3 Hz, 2H, ArH), 7.38 (dd, J = 6.3 Hz, J = 1.5 Hz, 1H, CH), 7.50 (d, J = 6.3 Hz, 2H, ArH), 7.98 (d, J = 1.5 Hz, 1H, ArH). Mass spectrum (LCMS, ESI pos.): Calcd for C₂₅H₂₂Cl₂IN₃O₂: 593.01; found 594.0 (M+H).

### EXAMPLE 27

(3*S*)-3-(4-Chloro-phenyl)-4-[(*R*)-1-(4-chloro-phenyl)-ethyl]-2-(3-hydroxypropylamino)-7-iodo-3,4-dihydro-benzo[e][1,4]diazepin-5-one 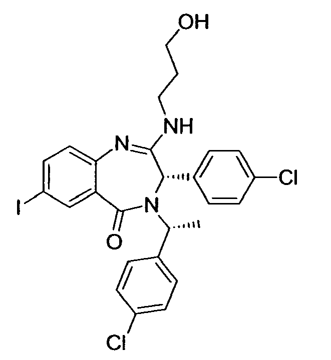 ¹H NMR (400 MHz, CDCl₃) δ 1.72 (d, J = 5.1 Hz, 3H, CH₃), 1.91-1.95 (m, 2H, CH₂), 3.78-3.86 (m, 4H, 2 CH₂), 4.89 (s, 1H, CH), 6.45 (q, J = 5.1 Hz, 1H, CH), 6.54 (d, *J =* 6.0 Hz, 2H, ArH), 6.58 (d, *J =* 6.6 Hz, 1H, ArH), 6.91 (d, *J* = 6.3 Hz, 2H, ArH), 7.32 (d, *J* = 6.0 Hz, 2H, ArH), 7.37-7.39 (m, 1H, ArH), 7.49 (d, J = 6.3 Hz, 2H, ArH), 7.98 (d, J = 1.5 Hz, 1H, ArH). Mass spectrum (LCMS, ESI pos.): Calcd for C₂₆H₂₄Cl₂IN₃O₂: 607.03; found 608.0 (M+H).

### EXAMPLE 28

*N*-(2-{(3*S*)-3-(4-Chloro-phenyl)-4-[(*R*)-1-(4-chloro-phenyl)-ethyl]-7-iodo-5-oxo-4,5-dihydro-3H-benzo[e][1,4]diazepin-2-ylamino}-ethyl)-acetamide 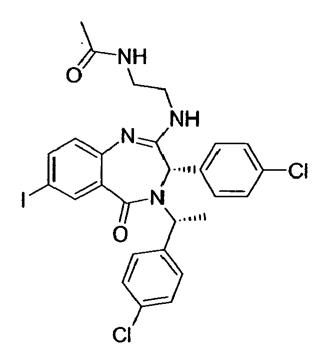 ¹H NMR (400 MHz, CDCl₃) δ 1.72 (d, J = 5.1 Hz, 3H, CH₃), 2.24 (s, 3H, CH₃), 3.47-3.90 (m, 4H, 2 CH₂), 6.08 (s, 1H, CH), 6.54 (q, J = 5.1 Hz, 1H, CH), 6.91 (d, *J* = 6.0 Hz, 2H, CH₃), 7.15-7.18 (m, 3H, ArH), 7.37 (d, *J* = 6.0 Hz, 2H, CH₂), 7.53-7.55 (m, 3H, ArH), 8.13 (d, J = 1.5 Hz, 1H, ArH). Mass spectrum (LCMS, ESI pos.): Calcd forC₂₇H₂₅Cl₂N₄O₂: 634.04; found 635.1 (M+H).

### EXAMPLE 29

(3*S*)-3-(4-Chloro-phenyl)-4-[(*R*)-1-(4-chloro-phenyl)-ethyl]-7-iodo-2-methylamino-3,4-dihydro-benzo[e][1,4]diazepin-5-one 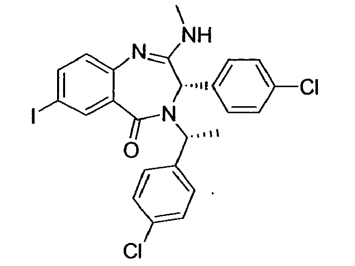 ¹H NMR (400 MHz, CDCl₃) δ 1.67 (d, J = 5.1 Hz, 3H, CH₃), 3.17 (s, 3H, CH₃), 4.85 (br s, 1H, CH), 6.42 (q, *J =* 5.1 Hz, 1H, CH), 6.53 (d, *J =* 6.3 Hz, 2H, ArH), 6.60 (d, *J =* 6.3 Hz, 1H, ArH), 6.89 (d, *J =* 6.3 Hz, 2H, ArH), 7.31 (d, J = 6.3 Hz, 2H, ArH), 7.38 (dd, J = 6.3 Hz, J = 1.5 Hz, 1H, ArH), 7.47 (d, *J =* 6.3 Hz, 2H, ArH), 7.97 (d, *J =* 1.5 Hz, 1H, ArH). Mass spectrum (LCMS, ESI pos.): Calcd for C₂₄H₂₀Cl₂IN₃O: 563.00; found 563.9 (M+H).

### EXAMPLE 30

2-(2-Amino-ethylamino)-(3*S*)-3-(4-chloro-phenyl)-4-[(*R*)-1-(4-chloro-phenyl)-ethyl]-7-iodo-3,4-dihydro-benzo[e][1,4]diazepin-5-one 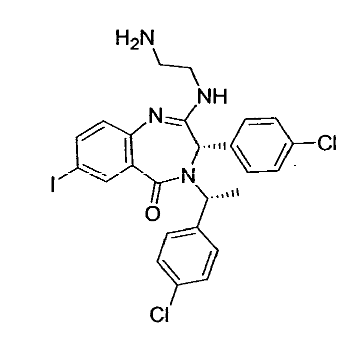 Mass spectrum (LCMS, ESI pos.): Calcd for C₂₅H₂₃Cl₂IN₄O: 592.03; found 593.0 (M+H).

### EXAMPLE 31

2-[*N*-(3-amino-3-oxopropyl)amino]-(3S)-3-(4-chlorophenyl)-4-[(*R*)-1-(4-chlorophenyl)ethyl]-7-iodo-3,4-dihydro-1,4-benzodiazepin-5-one

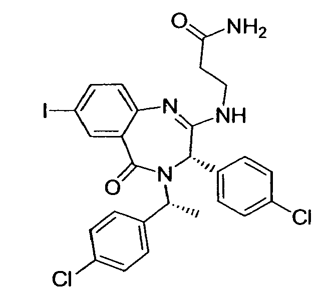

### MASS SPECTRUM (LCMS, ESI POS.): CALCD FOR C₂₆H₂₃CL₂IN₄O₂: 620.02; FOUND 621.0 (M+H). EXAMPLE 32

5-[(3*S*)-3-(4-chlorophenyl)-4-[(*R*)-1-(4-chlorophenyl)ethyl]-7-methoxycarbonyl-2,5-dioxo-3,4-dihydro-1*H*-1,4-benzodiazepin-1-yl]valeric acid tert-butyl ester

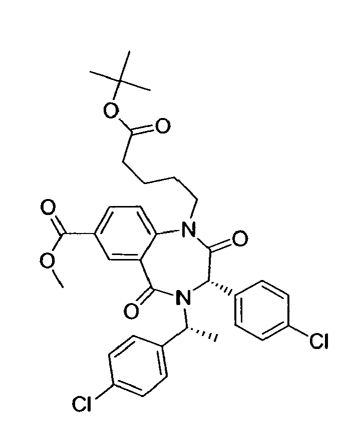

A solution of 5-[(3,*S*)-3-(4-chlorophenyl)-4-[(*R*)-1-(4-chlorophenyl)ethyl]-7-iodo-2,5-dioxo-3,4-dihydro-1*H*-1,4-benzodiazepin-1-yl]valeric acid tert-butyl ester (300 mg, 0.42 mmol), dichloro bis-(triphenylphosphine)Pd(II) (21 mg, 0.030 mmol), and triethylamine (200 µL, 1.43 mmol), in DMF (4 mL) and methanol (1.5 mL) was stirred under a carbon monoxide atmosphere at 80 °C for 24 h. After the solution was cooled to room temperature, the solvent was evaporated under reduced pressure. The crude reaction product was purified by column chromatography on silica gel (EtOAc/hexanes, 1:2) to give the title compound (226 mg, 83 %): ¹H NMR (400 MHz, CDCl₃) δ 1.45 (s, 9H, 3 CH₃), 1.58-1.62 (m, 2H, CH₂), 1.75 (d, J = 5.4 Hz, 3H, CH₃), 2.24 (t, J = 10.5 Hz, 2H, CH₂), 3.62-3.90 (m, 3H, 3 CH), 3.91 (s, 3H, CH₃), 4.23-4.31 (m, 1H, CH), 5.34 (s, 1H, CH), 6.45 (q, J = 5.4 Hz, 1H, CH), 6.50 (d, J = 6.3 Hz, 2H, ArH), 6.84 (d, J = 6.3 Hz, 2H, ArH), 6.94 (d, J = 6.6 Hz, 1H, ArH), 7.32 (d, J = 6.6 Hz, 2H, ArH), 7.50 (d, J = 6.3 Hz, 2H, ArH), 7.84 (dd, J = 6.3 Hz, J = 1.5 Hz, 1H, ArH), 8.33 (d, J = 1.5 Hz, 1H, ArH).

### EXAMPLE 33

(3*S*)-1-(4-*tert*-Butoxycarbonyl-butyl)-3-(4-chloro-phenyl)-4-[(*R*)-1-(4-chlorophenyl)-ethyl]-2,5-dioxo-2,3,4,5-tetrahydro-1H-benzo[e][1,4]diazepine-7-carboxylic acid

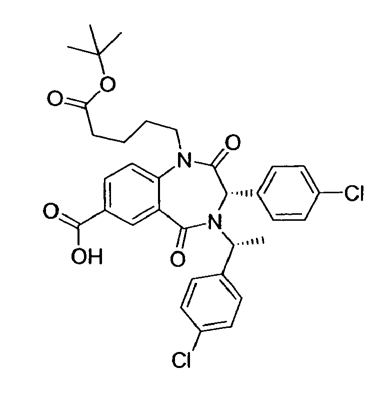

A solution of 5-[(3S)-3-(4-chlorophenyl)-4-[(*R*)-1-(4-chlorophenyl)ethyl]-7-methoxycarbonyl-2,5-dioxo-3,4-dihydro-1*H*-1,4-benzodiazepin-1-yl]valeric acid tert-butyl ester (Example 32) (200 mg, 0.31 mmol) and NaOH (1N in water, 380 µL) in THF/MeOH/H₂O (2 mL/400 µL/100 µL) was stirred at room temperature for 12 h. The mixture was evaporated to dryness. Then, the residue was dissolved in water (10 mL) and the pH was adjusted to 4 with 1N HCl. The solution was extracted with ether (3 x 20 mL), then the organic layer was dried (Na₂SO₄) and concentrated to dryness under reduced pressure. Filtration over silica gel afforded the title compound (150 mg, 77%): Mass spectrum (LCMS, ESI pos.): Calcd for C₃₃H₃₄Cl₂IN₂O₆: 624.18; found 568.9 (M+H - t-bu).

### EXAMPLE 34

7-aminocarbonyl-5-[(3*S*)-3-(4-chlorophenyl)-4-[(*R*)-1-(4-chlorophenyl)ethyl]-2,5-dioxo-3,4-dihydro-1*H*-1,4-benzodiazepin-1-yl]valeric acid

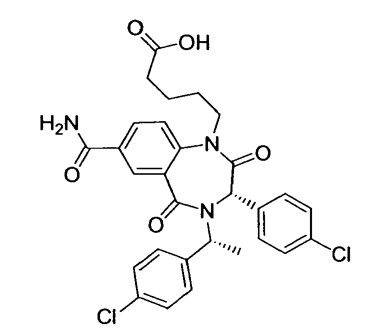

A solution of (3*S*)-1-(4-tert-Butoxycarbonyl-butyl)-3-(4-chloro-phenyl)-4-[(*R*)-1-(4-chloro-phenyl)-ethyl]-2,5-dioxo-2,3,4,5-tetrahydro-1*H*-benzo[e][1,4]-diazepine-7-carboxylic acid (Example 33) (63 mg, 0.10 mmol), O-(7-azabenzotriazol-1-yl)-*N,N,N',N'*-tetramethyluroniumhexafluorophosphate (57 mg, 0.15 mmol), HOBT (20 mg, 0.15 mmol) *N,N-*diisopropylethylamine (70 µL, 0.4 mmol), ammonium chloride (11 mg, 0.2 mmol) in DMF (400 µL) was stirred at room temperature for 1 h. under argon atmosphere. After evaporation of the solvent, the residue was diluted with EtOAc (15 mL), washed with water (10 mL), dried (Na₂SO₄) then concentrated to dryness under reduced pressure. Chromatography on silica (EtOAc/hexanes, 1:1) afforded anoil (58 mg), which was dissolved in dichloromethane (1.5 mL) and TFA (0.5 mL). This solution was stirred at room temperature for 10 h, then evaporated to dryness. The residue was dissolved in ice-cold water (10 mL) and AcONa (100 mg)/AcOH (100 µL) were added. The solution was extracted with ether (3 x 20 mL), then the organic layer was dried (Na₂SO₄) and concentrated to dryness under reduced pressure. Purification by column chromatography (EtOAc/CH₂Cl₂/EtOH, 5:4:1) afforded a colorless oil, which was converted to its sodium salt with NaOH 1N in EtOH. Recrystallization from EtOH/ether yielded title compound (37 mg, 62%) as a white powder: Mass spectrum (LCMS, ESI pos.): Calcd for C₁₉H₁₇Cl₂IN₃O₅: 567.13; found 568.0 (M+H).

### EXAMPLE 35

5-[4-(4-Chloro-2-methyl-benzyl)-3-(*R,S*)-(4-chloro-phenyl)-7-iodo-2,5-dioxo-2,3,4,5-tetrahydro-benzo[e][1,4]diazepin-1-yl]-valeric acid 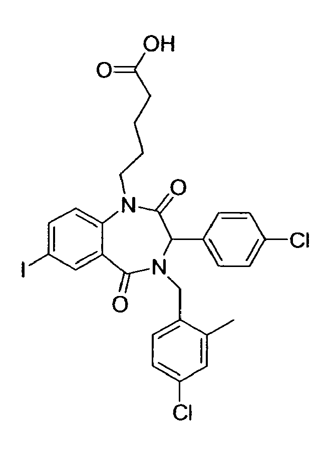 ¹H NMR (400 MHz, DMSO-d₆) δ 7.71 (d, *J*=2.2 Hz, 1H), 7.64-7.68 (m, 1H), 7.32 (m, 1H), 7.16-7.24 (m, 4H), 7.00 (d, *J*=7.0 Hz, 1H), 6.88 (d, *J*=7.6 Hz, 2H), 5.25 (s, 1H), 5.31 (d, *J*=15.6 Hz, 1H), 4.68 (d, *J*=15.4 Hz, 1H), 4.20 (m, 2H), 3.70 (m, 2H), 2.32 (s, 4H), 2.16-2.20 (m, 4H). Mass spectrum (LCMS, ESI pos.): Calcd for C₂₈H₂₅Cl₂IN₂O₄: 650.02; found 650.8 (M + H).

### EXAMPLE 36

Sodium; 5-{(3*R*)-3-(4-chloro-phenyl)-4-[(*R*)-1-(4-chloro-phenyl)-ethyl]-7-iodo-2,5-dioxo-2,3,4,5-tetrahydro-benzo[e] [1,4]diazepin-1-yl}-valerate 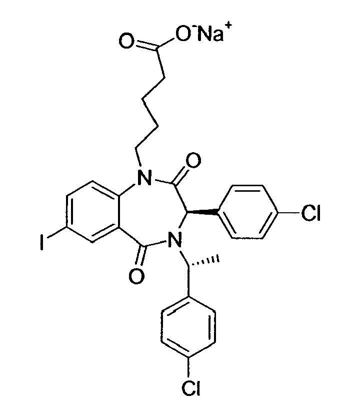 ¹H NMR (400 MHz, DMSO-d₆) δ 7.88 (d, *J*=2.0 Hz, 1H), 7.56-7.60 (m, 1H), 7.40-7.44 (m, 4H), 7.10-7.14 (m, 2H), 6.90-6.95 (m, 2H), 6.80-6.84 (d, J=8.8 Hz, 1H), 6.36-6.40 (m, 1H), 5.32 (s, 1H), 4.04-4.10 (m, 1H), 3.52-3.60 (m, 1H), 2.03-2.12 (m, 1H), 1.64-1.68 (d, J=7.2 Hz, 4H), 1.40-1.52 (m, 4H). Mass spectrum (LCMS, ESI pos.): Calcd for C₁₈H₂₄Cl₂IN₂NaO₄: 672.1; found 673.2 (M + H).

### EXAMPLE 37

5-[(3*S*)-3-(4-Chlorophenyl)-4-[(*R*)-1-(4-chlorophenyl)-ethyl]-7-[(*R,S*)-1-hydroxyethyl]-2,5-dioxo-2,3,4,5-tetrahydro-benzo[e] [1,4]diazepin-1-yl]-valeric acid 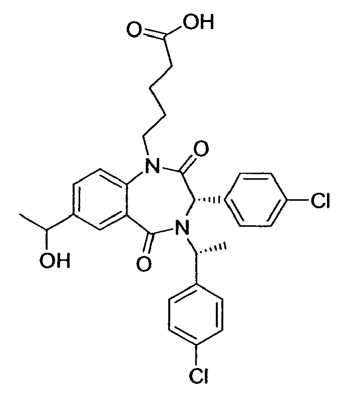
a) 5-[(3*R,S*)-3-(4-Chloro-phenyl)-4-[(*R*)-1-(4-chloro-phenyl)-ethyl]-7-[(*R,S*)-1-hydroxy-ethyl]-2,5-dioxo-2,3,4,5-tetrahydro- benzo[e][1,4]- diazepin-1-yl]-valeric acid tert-butyl ester
   A solution of 5-{(3*R,S*)*-*7-Acetyl-3-(4-chloro-phenyl)-4-[1-(*R*)-(4-chlorophenyl)-ethyl]-2,5-dioxo-2,3,4,5-tetrahydro-benzo[e][1,4]diazepin-1-yl}-valeric acid tert-butyl ester (60 mg, 0.096 mmol) in MeOH (2 mL) was treated with NaBH₄ (5.0 mg, 0.13 mmol). The solution was stirred at RT for 18 h. All of the solution was applied to a prep t1c plate (Analtech Silica Gel GF, 20 X 20 cm, 2000 microns). The desired band was scraped off, extracted with MeOH for 10 min. filtered and concentrated to give the title compound (55 mg, 92%). Mass spectrum (LCMS, ESI pos.): Calcd for C₃₄H₃₈Cl₂N₂O₅: 624.22; found 625.4 (M + H).
b) 5-[(3*S*)-3-(4-Chloro-phenyl)-4-[1-(*R*)-(4-chloro-phenyl)-ethyl]-7-(1-(*R,S*)-hydroxy-ethyl)-2,5-dioxo-2,3,4,5-tetrahydr o-benzo[e][1,4]diazepin-1-yl]-valeric acid
   The title compound was synthesized from 5-[(3*R*,*S*)-3-(4-Chloro-phenyl)-4-[(*R*)-1-(4-chloro-phenyl)-ethyl]-7-[(*R*,*S*)-1-hydroxy-ethyl]-2,5-dioxo-2,3,4,5-tetrahydro-benzo[e][1,4]-diazepin-1-yl]-valeric acid tert-butyl ester following the procedure for example 1, step b. ¹H NMR (400 MHz, DMSO-d₆) δ 7.56-7.61 (m, 3H), 7.40-7.50 (m, 1H), 7.18-7.38 (m, 3H), 7.00-7.08 (t, *J*=8.3 Hz, 1H), 6.83-6.87 (m, 2H), 6.50-6.56 (m, 2H), 6.35-6.40 (m, 1H), 5.35 (s, 1H), 4.68-4.72 (m, 1H), 4.30-4.40 (m, 1H), 3.70-3.79 (m, 1H), 2.10-2.18 (m, 2H), 1.72-1.76 (d, J=7.3 Hz, 4H), 1.50-1.70 (m, 4H), 1.21-1.26 (d, J=6.6 Hz, 3H). Mass spectrum (LCMS, ESI pos.): Calcd for C₃₀H₃₀Cl₂N₂O₅: 568.1; found 569.3 (M + H).

### EXAMPLE 38

5-[(*R,S*)-3-(4-Chloro-phenyl)-4-[(*R*)-1-(4-chloro-phenyl)-ethyl]-7-(1-(*R,S*)-hydroxyethyl)-2,5-dioxo-2,3,4,5-tetrahydro-benzo[e][1,4]diazepin-1-yl]-valeric acid

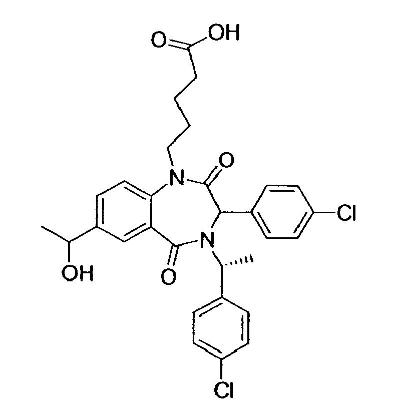

The title compound was prepared following successively the procedures described for example 37: ¹H NMR (400 MHz, DMSO-d₆) δ 7.00-7.61 (m, 9H), 6.84-6.92 (m, 2H), 6.36-6.56 (m, 3H), 5.30-5.36 (m, 1H), 4.08-4.76 (m, 2H), 3.50-3.80 (m, 1H), 2.05-2.20 (m, 2H), 1.72-1.76 (d, *J*=7.3 Hz, 3H), 1.20-1.70 (m, 7H). Mass spectrum (LCMS, ESI pos.): Calcd for C₃₀H₃₀Cl₂N₂O₅: 568.1; found 569.2 (M + H).

### EXAMPLE 39

5-[(3*S*)-4-[(*R*)-1-(4-Chlorophenyl)ethyl]-7-iodo-2,5-dioxo-3-(4-trifluoromethyl-phenyl)-2,3,4,5-tetrahydro-benzo[e][1,4]diazepin-1-yl]-valeric acid 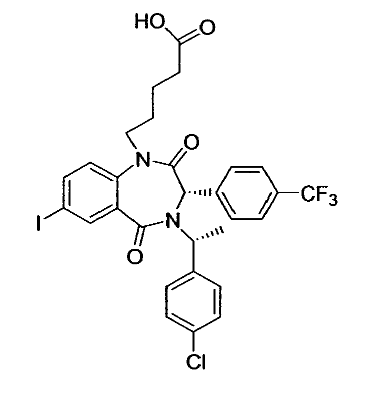 ¹H NMR (400 MHz, DMSO-d₆) δ 7.86-7.91 (d, J=2.0 Hz, 1H), 7.52-7.58 (m, 1H), 7.45-7.51 (m, 4H), 7.12-7.22 (m, 2H), 6.92-7.05 (m, 2H), 6.82-6.94 (d, J=8.8 Hz, 1H), 6.32-6.38 (m, 1H), 5.32 (s, 1H), 4.08-4.11 (m, 1H), 3.52-3.64 (m, 1H), 2.06-2.11 (m, 2H), 1.62-1.70 (d, J=7.2 Hz, 4H), 1.40-1.46 (m, 4H). Mass spectrum (LCMS, ESI pos.): Calcd for C₂₉H₂₅ClF₃IN₂O₄: 684.05; found 685.1 (M + H).

### EXAMPLE 40

Sodium; 5-{(*S*)-3-(4-chloro-phenyl)-4-[(*R*)-1-(4-chloro-phenyl)-ethyl]-7-ethynyl-2,5-dioxo-2,3,4,5-tetrahydro-benzo[e][1,4]diazepin-1-yl}-valerate 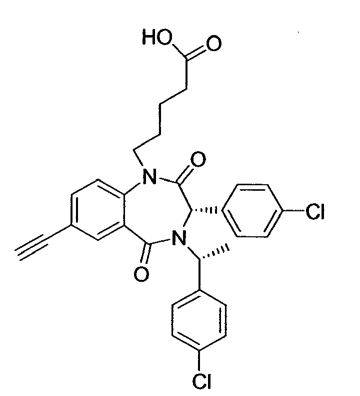
a) 5-{(3*R*,*S*)-3-(4-Chlorophenyl)-4-[(*R*)-1-(4-chlorophenyl)ethyl]-7-ethynyl-2,5-dioxo-2,3,4,5-tetrahydro-benzo[e] [1,4]diazepin-1-yl}-valeric acid tert-butyl ester
   A mixture of 5-{(3*R*,*S*)-3-(4-Chloro-phenyl)-4-[(*R*)-1-(4-chloro-phenyl)-ethyl]-7-iodo-2,5-dioxo-2,3,4,5-tetrahydro-benzo[e][1,4]diazepin-1-yl}-valeric acid tert-butyl ester (3.2 g, 4.5 mmol), trimethylsilylacetylide (1.9 mL, 13.5 mmol), Cul (189 mg, 0.99 mmol) and dichlorobis(triphenylphosphine) palladium (II) (318 mg, 0.45 mmol) in triethylamine (200 mL) was heated to 55°C under an argon atmosphere. After 1 h solution was filtered hot and filtrate was concentrated. A solution of 5-{(3*R*,*S*)-3-(4-Chlorophenyl)-4-[(R)-1-(4-chlorophenyl)ethyl]-2,5-dioxo-7-trimethylsilanylethynyl-2,3,4,5-tetrahydro-benzo[e][1,4]diazepin-1-yl}-valeric acid tert-butyl ester (6.7g, 9.9 mmol) in THF (100 mL) was treated with tetrabutylammonium fluoride (10 mL of 1 M in THF, 10 mmol). After 1 h reaction solution was concentrated. Flash chromatography (silica gel, EtOAc/hexanes 3:7) gave the title compound (5.3g, 89% over two steps).

Sodium; 5-{(3*S*)-3-(4-chloro-phenyl)-4-[(*R*)-1-(4-chloro-phenyl)-ethyl]-7-ethynyl-2,5-dioxo-2,3,4,5-tetrahydro-benzo[e][1,4]diazepin-1-yl}-valerate 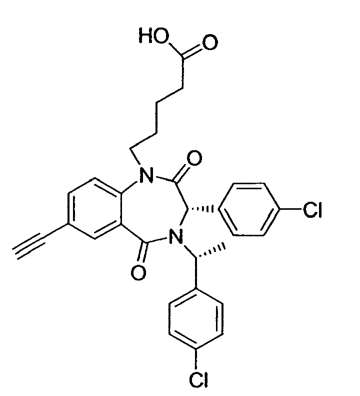 The title compound was synthesized from 5-{(3*R*,*S*)-3-(4-Chlorophenyl)-4-[(*R*)-1-(4-chloro-phenyl)-ethyl]-7-ethynyl-2,5-dioxo-2,3,4,5-tetrahydrobenzo[e][1,4]diazepin-1-yl}-valeric acid tert-butyl ester following the example 1, step b. ¹H NMR (400 MHz, DMSO-d₆) δ 7.59-7.64 (m, 3H), 7.30-7.40 (m, 3H), 7.08-7.10 (d,J=8.5 Hz, 1H), 6.90-6.93 (d,*J*=8.5, 2H), 6.53-6.58 (d,*J*=8.8, 2H), 6.32-6.39 (m, 1H), 5.36 (s, 1H), 4.24-4.32 (m, 1 H), 3.76-3.84 (m, 1H), 3.52 (s, 1H), 2.12-2.20 (m, 2H), 1.72-1.76 (d,*J*=7.1 Hz, 3H), 1.52-1.64 (m, 4H). Mass spectrum (LCMS, ESI pos.): Calcd for C₃₀H₂₅Cl₂N₂NaO₄: 570.11; found 571.2 (M + H).

### EXAMPLE 41

5- {(3*R,S)-*3-(4-Chlorophenyl)-4-[(*R,S*)-1-(4-chlorophenyl)-2-hydroxyethyl]-7-iodo-2,5-dioxo-2,3,4,5-tetrahydro-benzo[e][1,4]diazepin-1-yl}-valeric acid 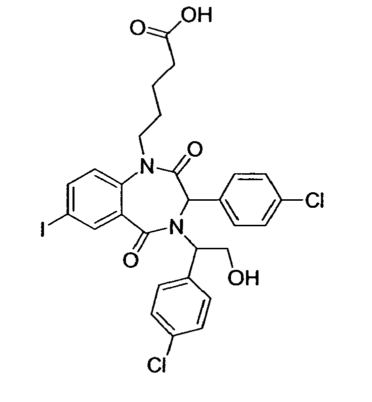 ¹H NMR (400 MHz, DMSO-d₆) δ 7.84-7.87 (d, *J*=2.1 Hz, 1H), 7.55-7.60 (m, 3H), 7.36-7.40 (m, 2H), 6.88-6.96 (m, 3H), 6.56-6.60 (d,*J*=7.7, 2H), 6.20-6.28 (m, 1H), 5.40 (s, 1H), 4.12-4.33 (m, 3H), 3.70-3.80 (s, 1H), 2.12-2.20 (m, 2H), 1.50-1.76 (m, 6H). Mass spectrum (LCMS, ESI pos.): Calcd for C₂₈H₂₅Cl₂IN₂O₅: 666.02; found 667.2 (M + H).

### EXAMPLE 42

Sodium; 5-{(3*S*)-3-(4-chlorophenyl)-4-[(*R*)-1-(4-chlorophenyl)ethyl]-7-iodo-2,5-dioxo-2,3,4,5-tetrahydro-benzo[e][1,4]diazepin-1-yl}-valerate 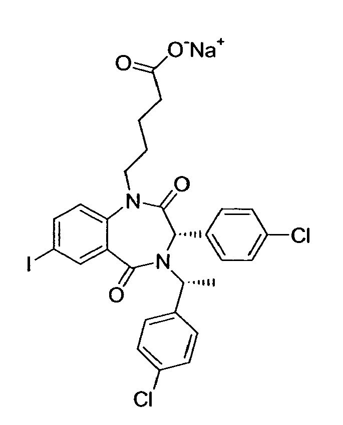 ¹H NMR (400 MHz, DMSO-d₆) δ 7.75-7.77 (d, *J*=2.1 Hz, 1H), 7.56-7.64 (m, 3H), 7.39-7.44 (m, 2H), 6.98-7.03 (m, 2H), 6.90-6.95 (m, 1H), 6.56-6.60 (d, J=7.7 Hz, 1H), 6.16-6.22 (m, 1H), 5.26 (s, 1H), 4.15-4.24 (m, 1H), 3.60-3.70 (m, 1H), 1.76-1.84 (m, 2H), 1.60-1.68 (d, *J*=7.1 Hz, 3H), 1.44-1.56 (m, 1H), 1.28-1.37 (m, 4H). Mass spectrum (LCMS, ESI pos.): Calcd for C₂₈H₂₄Cl₂IN₂NaO₄: 672.01; found 651.2 (M -Na)⁺.

### EXAMPLE 43

5-[(3*R,S*)-4-(4-Chloro-2-methyl-benzyl)-3-(4-chloro-phenyl)-7-iodo-2,5-dioxo-2,3,4,5-tetrahydro-benzo[e][1,4]diazepin-1-yl]-valeric acid 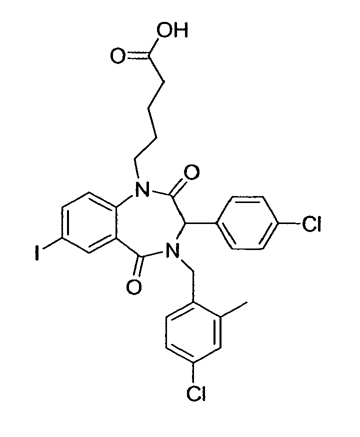 ¹H NMR (400 MHz, DMSO-d₆) δ 7.71 (d, J=2.2 Hz, 1H), 7.64-7.68 (m, 1H), 7.32 (m, 1H), 7.16-7.24 (m, 4H), 7.00 (d, J=7.0 Hz, 1H), 6.88 (d, J=7.6 Hz, 2H), 5.25 (s, 1H), 5.31 (d, J=15.6 Hz, 1H), 4.68 (d, J=15.4 Hz, 1H), 4.20 (m, 2H), 3.70 (m, 2H), 2.32 (s, 4H), 2.16-2.20 (m, 4H). Mass spectrum (LCMS, ESI pos.): Calcd for C₂₈H₂₅Cl₂IN₂O₄: 650.02; found 650.8 (M + H).

### EXAMPLE 44

Sodium; 5-{(3*R*)-3-(4-chlorophenyl)-4-[(*R*)-1-(4-chlorophenyl)ethyl]-7-iodo-2,5-dioxo-2,3,4,5-tetrahydro-benzo[e][1,4]diazepin-1-yl}-valerate 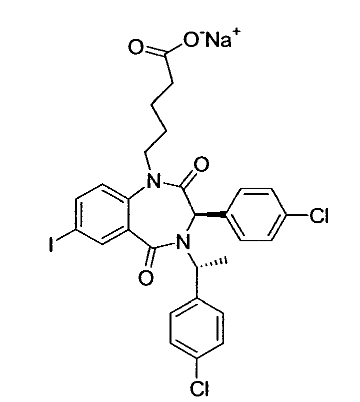 ¹H NMR (400 MHz, DMSO-d₆) δ 7.88 (d, J=2.0 Hz, 1H), 7.56-7.60 (m, 1H), 7.40-7.44 (m, 4H), 7.10-7.14 (m, 2H), 6.90-6.95 (m, 2H), 6.80-6.84 (d, J=8.8 Hz, 1H), 6.36-6.40 (m, 1H), 5.32 (s, 1H), 4.04-4.10 (m, 1H), 3.52-3.60 (m, 1H), 2.03-2.12 (m, 1H), 1.64-1.68 (d, *J*=7.2 Hz, 4H), 1.40-1.52 (m, 4H). Mass spectrum (LCMS, ESI pos.): Calcd for C₂₈H₂₄Cl₂IN₂NaO₄: 672.1; found 673.2 (M + H).

### EXAMPLE 45

5-[(3*S*)-3-(4-Chlorophenyl)-4-[(R)-1-(4-chlorophenyl)-ethyl]-7-(1-(*R,S*)-hydroxyethyl)-2,5-dioxo-2,3,4,5-tetrahydro-benzo[e][1,4]diazepin-1-yl]-valeric acid 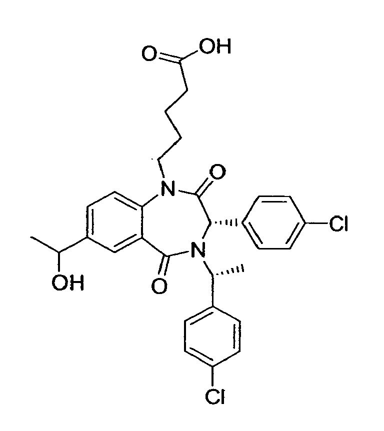 ¹H NMR (400 MHz, DMSO-d₆) δ 7.56-7.61 (m, 3H), 7.40-7.50 (m, 1H), 7.18-7.38 (m, 3H), 7.00-7.08 (t, *J*=8.3 Hz, 1H), 6.83-6.87 (m, 2H), 6.50-6.56 (m, 2H), 6.35-6.40 (m, 1H), 5.35 (s, 1H), 4.68-4.72 (m, 1H), 4.30-4.40 (m, 1H), 3.70-3.79 (m, 1H), 2.10-2.18 (m, 2H), 1.72-1.76 (d, *J=7.3* Hz, 4H), 1.50-1.70 (m, 4H), 1.21-1.26 (d, J=6.6 Hz, 3H). Mass spectrum (LCMS, ESI pos.): Calcd for C₃₀H₃₀Cl₂N₂O₅: 568.1; found 569.3 (M + H).

### EXAMPLE 46

5-[(3*R,S*)-3-(4-Chlorophenyl)-4-[(*R*)-1-(4-chlorophenyl)ethyl]-7-[(*R,S*)-1-hydroxy-ethyl]-2,5-dioxo-2,3,4,5-tetrahydro-benzo[e][1,4]diazepin-1-yl]-valeric acid 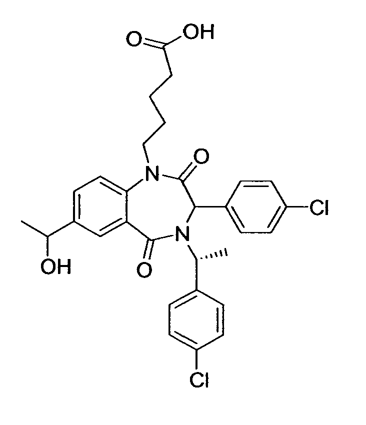 ¹H NMR (400 MHz, DMSO-d₆) δ 7.00-7.61 (m, 9H), 6.84-6.92 (m, 2H), 6.36-6.56 (m, 3H), 5.30-5.36 (m, 1H), 4.08-4.76 (m, 2H), 3.50-3.80 (m, 1H), 2.05-2.20 (m, 2H), 1.72-1.76 (d, *J*=7.3 Hz, 3H), 1.20-1.70 (m, 7H). Mass spectrum (LCMS, ESI pos.): Calcd for C₃₀H₃₀Cl₂N₂O₅: 568.1; found 569.2 (M + H).

### EXAMPLE 47

5-[(3*S*)-4-[(*R*)-1-(4-Chlorophenyl)-ethyl]-7-iodo-2,5-dioxo-3-(4-trifluoromethyl-phenyl)-2,3,4,5-tetrahydro-benzo[e][1,4]diazepin-1-yl]-valeric acid 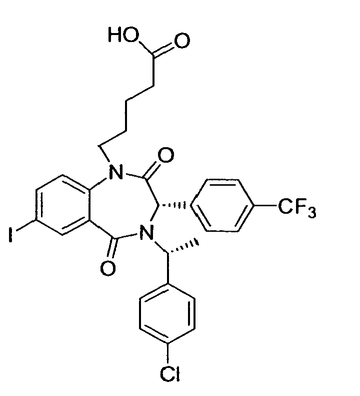 ¹H NMR (400 MHz, DMSO-d₆) δ 7.86-7.91 (d, J=2.0 Hz, 1H), 7.52-7.58 (m, 1H), 7.45-7.51 (m, 4H), 7.12-7.22 (m, 2H), 6.92-7.05 (m, 2H), 6.82-6.94 (d, J=8.8 Hz, 1H), 6.32-6.38 (m, 1H), 5.32 (s, 1H), 4.08-4.11 (m, 1H), 3.52-3.64 (m, 1H), 2.06-2.11 (m, 2H), 1.62-1.70 (d, J=7.2 Hz, 4H), 1.40-1.46 (m, 4H). Mass spectrum (LCMS, ESI pos.): Calcd for C₂₉H₂₅CIF₃IN₂O₄: 684.05; found 685.1 (M + H).

### EXAMPLE 48

Sodium; 5-{(*3S*)-3-(4-chlorophenyl)-4-[(*R*)-1-4-chlorophenyl)-ethyl]-7-ethynyl-2,5-dioxo-2,3,4,5-tetrahydro-benzo[e][1,4]diazepin-1-yl}-valerate 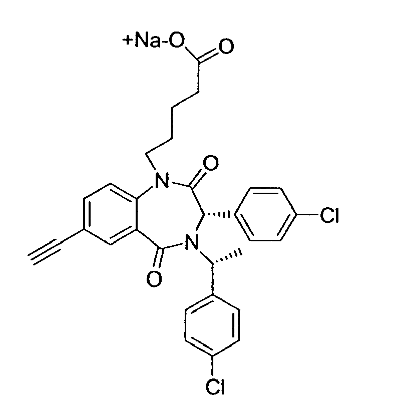 ¹H NMR (400 MHz, DMSO-d₆) δ 7.59-7.64 (m, 3H), 7.30-7.40 (m, 3H), 7.09 (d, *J*=8.5 Hz, 1H), 6.92 (d, *J*=8.5, 2H), 6.54 (d, *J*=8.8, 2H), 6.32-6.39 (m, 1H), 5.36 (s, 1H), 4.24-4.32 (m, 1H), 3.76-3.84 (m, 1H), 3.52 (s, 1H), 2.12-2.20 (m, 2H), 1.73(d, *J*=7.1 Hz, 3H), 1.52-1.64 (m, 4H). Mass spectrum (LCMS, ESI pos.): Calcd for C₃₀H₁₅Cl₂N₂NaO₄: 570.1; found 571.2 (M + H).

### EXAMPLE 49

5- {3-(4-Chlorophenyl)-4-[1-(4-chlorophenyl)-2-hydroxy-ethyl]-7-iodo-2,5-dioxo-2,3,4,5-tetrahydro-benzo[e][1,4]diazepin-1-yl}-valeric acid 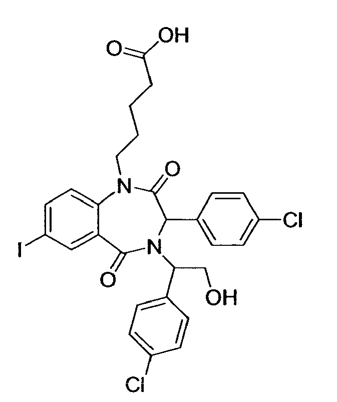 ¹H NMR (400 MHz, DMSO-d₆) δ 7.85 (d, *J*=2.1 Hz, 1H), 7.55-7.60 (m, 3H), 7.36-7.40 (m, 2H), 6.88-6.96 (m, 3H), 6.58 (d, *J*=7.7,2H), 6.20-6.28 (m, 1H), 5.40 (s, 1H), 4.12-4.33 (m, 3H), 3.70-3.80 (s, 1H), 2.12-2.20 (m, 2H), 1.50-1.76 (m, 6H). Mass spectrum (LCMS, ESI pos.): Calcd for C₂₈H₂₅Cl₂IN₂O₅: 666.02; found 667.2 (M + H).

### EXAMPLE 50

Sodium; 5-{*(3S)*-3-(4-chlorophenyl)-4-[*(R)*-1-(4-chlorophenyl)-ethyl)-7-iodo-2,5-dioxo-2,3,4,5-tetrahydro-benzo[e][1,4]diazepin-1-yl}-valerate 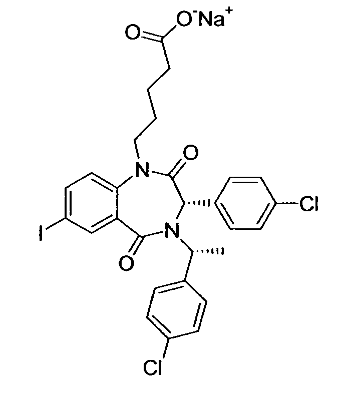 ¹H NMR (400 MHz, DMSO-d₆) δ 7.76 (d, *J*=2.1 Hz, 1H), 7.56-7.64 (m, 3H), 7.39-7.44 (m, 2H), 6.98-7.03 (m, 2H), 6.90-6.95 (m, 1H), 6.57 (d, J=7.7 Hz, 1H), 6.16-6.22 (m, 1H), 5.26 (s, 1H), 4.15-4.24 (m, 1H), 3.60-3.70 (m, 1H), 1.76-1.84 (m, 2H), 1.64 (d, *J*=7.1 Hz, 3H), 1.44-1.56 (m, 1H), 1.28-1.37 (m, 4H). Mass spectrum (LCMS, ESI pos.): Calcd for C₂₈H₂₄Cl₂IN₂NaO₄: 672.01; found 651.2 (M -Na)⁺.

### EXAMPLE 51

5-[(3*S*)-3-(4-Chlorophenyl)-4-[(*R*)-1-(4-chlorophenyl)ethyl]-7-(methylthio)-2,5-dioxo-1,4-benzodiazepin-1-yl]valeric acid 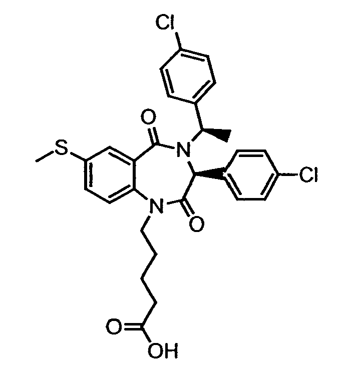
a) (3*S*)-3-(4-Chlorophenyl)-4-[(*R*)-1-(4-chlorophenyl)ethyl]-7-(methylthio)-1,3-dihydro-1,4-benzodiazepine-2,5-dione.
   (3*S*)-3-(4-Chlorophenyl)-4-[(*R*)-1-(4-chlorophenyl)ethyl]-7-iodo-1,3-dihydro-1,4-benzodiazepine-2,5-dione (221 mg, 0.400 mmol), sodium thiomethoxide (38 mg, 0.521 mmol) and *tetrakis*(triphenylphosphine)palladium (0) (46 mg, 0.04 mmol) were suspended in n-butanol (5mL) under nitrogen atmosphere. The reaction was warmed up to 110°C for 16 h. Then, the reaction was allowed to cool down to room temperature, partitioned between ethyl acetate and sodium hydroxide (1N), and washed with hydrochloric acid (1N). The organic layer was collected, dried with sodium sulfate, filtered and concentrated under vacuum. The residue was purified by column chromatography (AcOEt/Hexanes, 1:1) to give the title compound (91 mg, 48%) identified as the "lower diastereomer" (in a TLC performed with AcOEt/Hexanes, 1:1): ' 1 H NMR (400 MHz, CDCl₃) δ 9.16 (s, 1H), 7.50 (d, J = 2.3 Hz, 1H), 7.45 (d, J = 8.5 Hz, 2H), 7.24 (d, J = 8.5 Hz, 2H), 7.00 (dd, J = 2.3, J = 8.5 Hz, 1H), 6.83 (d, J = 8.5 Hz, 2H), 6.61 (d, J = 8.5 Hz, 1H), 6.56 (d, J = 8.5 Hz, 2H), 6.39 (m, 1H), 5.16 (s, 1H), 2.33 (s, 3H), 1.64 (d, J = 7.19 Hz, 3H); together with (3R)-3-(4-chlorophenyl)-4-[(R)-1-(4-chlorophenyl)ethyl]-7-(methylthio)-1,3-dihydro-1,4-benzodiazepine-2,5-dione (45 mg, 24%) identified as the "upper diastereomer" (in a TLC performed with AcOEt/Hexanes, 1:1): ¹H NMR (400 MHz, CDCl₃) δ 8.73 (s, 1H), 7.60 (d, *J* = 2.3 Hz, 1H), 7.24 (d, *J* = 8.5 Hz, 2H), 7.15 (d, *J* = 8.5 Hz, 2H), 7.04 (m, 3H), 6.93 (d, J = 8.5 Hz, 2H), 6.45 (d, J = 8.5 Hz, 1H), 6.38 (m, 1H), 5.01 (s, 1H), 2.36 (s, 3H), 1.59 (d, *J =* 7.2 Hz, 3H).
b) 5-[(3*S*)-3-(4-Chlorophenyl)-4-[(*R*)-1-(4-chlorophenyl)ethyl]-7-(methylthio)-2,5-dioxo-1,4-benzodiazepin-1-yl]valeric acid
   The title compound was prepared from (3*S*)-3-(4-Chlorophenyl)-4-[(*R*)-1-(4-chlorophenyl)ethyl]-7-(methylthio)-1,3-dihydro-1,4-benzodiazepine-2,5-dione following the procedure described for example 1, step b: ¹H NMR (400 MHz, Cl₃CD) δ 10.89 (s, 1H, Acid-OH), 7.38 (d, J = 8.5 Hz, 2H), 7.33 (d, J = 2.3 Hz, 1H), 7.20 (d, J = 8.5 Hz, 2H), 6.97 (dd, *J* = 2.3, *J* = 8.5 Hz, 1H), 6.75 (d, J = 8.5 Hz, 2H), 6.67 (d, *J =* 8.5 Hz, 1H), 6.36 (d, *J* = 8.5 Hz, 2H), 6.29 (m, 1H), 5.26 (s, 1H), 4.28 (m, 1H), 3.54 (m, 1H), 2.35 (s, 3H), 2.34 (m, 2H), 1.63 (d, *J* = 7.19 Hz, 3H), 1.46 (m, 4H). Mass spectrum (LCMS, ESI pos.): Calcd for C₂₉H₂₈Cl₂N₂O₄S: 570.1; found 571.0 (M+H).

### EXAMPLE 52

5-[(3R)-3-(4-Chlorophenyl)-4-[(R)-1-(4-chlorophenyl)ethyl]-7-(methylthio)-2,5-dioxo-1,4-benzodiazepin-1-yl]valeric acid 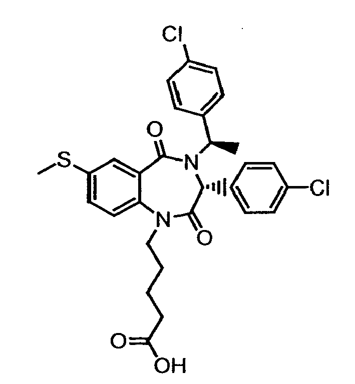 ¹H NMR (400 MHz, CDCl₃) δ 7.42 (d, J = 2.3 Hz, 1H), 7.27 (m, 4H), 6.98 (m, 3H), 6.75 (d, J = 8.5 Hz, 2H), 6.62 (d, J = 8.5 Hz, 1H), 6.37 (m, 1H), 5.22 (s, 1H), 4.05 (m, 1H), 3.37 (m, 1H), 2.35 (s, 3H), 2.19 (m, 2H), 1.58 (d, *J=* 7.2 Hz, 3H), 1.38 (m, 4H). Mass spectrum (LCMS, ESI pos.): Calcd for C₂₉H₂₈Cl₂N₂O₄S: 570.1; found 571.0 (M+H).

### EXAMPLE 53

5-[(3*S*)-3-(4-Chlorophenyl)-4-[(*R*)-1-(4-chlorophenyl)ethyl]-7-(trifluoromethylthio)-2,5-dioxo-1,4-benzodiazepin-1-yl]valeric acid 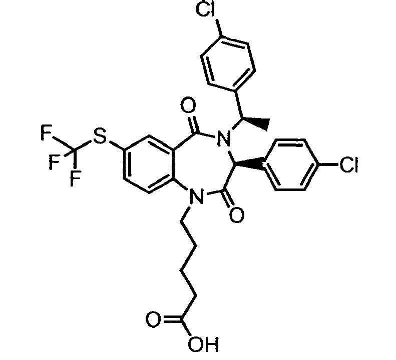
a) (3*S*)-3-(4-Chlorophenyl)-4-[(*R*)-1-(4-chlorophenyl)ethyl]-7-(trifluoromethylthio)-1,3-dihydro-1,4-benzodiazepine-2,5-dione (3*S*)-3-(4-Chlorophenyl)-4-[(*R*)-1-(4-chlorophenyl)ethyl]-7-iodo-1,3-dihydro-1,4-benzodiazepine-2,5-dione (248 mg, 0.449 mmol), copper trifluoromethanethiol (79.5 mg, 0.483 mmol), copper iodide (4 mg, 0.021 mmol), ethylene glycol (54.5 mg, 0.878 mmol) and potassium carbonate (121 mg, 0.878 mmol), were suspended in isopropanol (5 mL) under nitrogen atmosphere. The reaction was warmed up to 110°C for 24 h. Then, the reaction was allowed to cool to room temperature and was partitioned between ethyl acetate and sodium hydroxide (1N), washed with hydrochloric acid (1N). The organic layer was collected, dried (Na₂SO₄), filtered and concentrated under vacuum. Purification by chromatography on silica (AcOEt/Hexanes, 3:7) afforded the title compound (203 mg, 86%) identified as the "lower diastereomer" (in a TLC performed with AcOEt/Hexanes, 1:1): ¹H NMR (400 MHz, CDCl₃) δ 9.31 (s, 1H), 7.98 (d, *J* = 1.0 Hz, 1H), 7.44 (d, *J* = 8.5 Hz, 2H), 7.40 (dd, *J* = 1.0, J = 8.5 Hz, 1H), 7.25 (d, J = 8.5 Hz, 2H), 6.87 (d, J = 8.5 Hz, 2H), 6.55 (d, J = 8.5 Hz, 2H), 6.47 (d, J = 8.5 Hz, 1H), 6.38 (m, 1H), 5.22 (s, 1H), 1.64 (d, *J =* 7.2 Hz, 3H); together with (3R)-3-(4-chlorophenyl)-4-[(*R*)-1-(4-chlorophenyl)ethyl]-7-(trifluoromethylthio)-1,3-dihydro-1,4-benzodiazepine-2,5-dione (24 mg, 10%) identified as the "upper diastereomer" (in a TLC performed with AcOEt/Hexanes, 1:1): ¹H NMR (400 MHz, CDCl₃) δ 8.28 (s, 1H), 8.07 (d, J = 2.0 Hz, 1H), 7.44 (dd, J = 2.0, J = 8.5 Hz, 1H), 7.24 (d, *J =* 8.5 Hz, 2H), 7.19 (d, *J =* 8.5 Hz, 2H), 7.07 (d, J = 8.5 Hz, 2H), 6.92 (d, J = 8.5 Hz, 2H), 6.36 (m, 1H), 6.27 (d, J = 8.5 Hz, 1H), 5.01 (s, 1H), 1.58 (d, *J*= 7.2 Hz, 3H).
b) 5-[(3*S*)-3-(4-Chlorophenyl)-4-[(*R*)-1-(4-chlorophenyl)ethyl]-7-(trifluoromethylthio)-2,5-dioxo-1,4-benzodiazepin-1-yl]valeric acid.

The title compound was prepared from (3*S*)-3-(4-chlorophenyl)-4-[(*R*)-1-(4-chlorophenyl)ethyl]-7-(trifluoromethylthio)-1,3-dihydro-1,4-benzodiazepine-2,5-dione following the general procedure for the alkylation at the 1-position, followed by deprotection following the procedure for example 1, step b: ¹H NMR (400 MHz, Cl₃CD) δ 10.93 (s, 1H, Acid-OH), 7.83 (s, 1H), 7.38 (m, 3H), 7.20 (d, J= 7.9 Hz, 2H), 6.77 (d, *J*= 8.0 Hz, 2H), 6.51 (d, J = 8.5 Hz, 1H), 6.35 (d, J = 7.9 Hz, 2H), 6.28 (m, 1H), 5.28 (s, 1H), 4.25 (m, 1H), 3.55 (m, 1H), 2.27 (br s, 2H), 1.62 (d, J= 6.8 Hz, 3H), 1.49 (m, 4H).

### EXAMPLE 54

5-[(3S)-3-(2-Allyloxy-4-chlorophenyl)-4-[(*R*)-1-(4-chlorophenyl)ethyl]-7-iodo-2,5-dioxo-1,4-benzodiazepin-1-yl]valeric acid 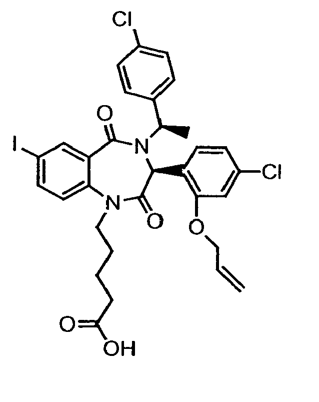
a) 5-Chloro-2-hydroxymethylphenol
   4-Chlorosalicilic acid (25 g, 145 mmol) was dissolved in tetrahydrofuran (100 mL) and cooled down to 0°C under argon atmosphere using an ice-water bath. Carefully, borane methyl sulfide complex (101 mL, 2M in THF) was added drop wise over a period of 30 minutes, and the resulting solution was refluxed for 16 h. The reaction was allowed to reach room temperature and poured into ice water, extracted with ethyl acetate, and washed with hydrochloric acid (1N). The organic layer was collected, dried with sodium sulfate, filtered and concentrated under vacuum. The residue was purified by chromatography on silica to give the title compound (83%): ¹H NMR (400 MHz, CDCl₃) δ 7.66 (br s, 1H), 6.96 (d, J = 8.0 Hz, 1H), 6.91 (d, J = 0.95 Hz, 1H), 6.85 (dd, *J* = 0.95, 8.0 Hz, 1H), 8.86 (s, 2H).
b) 4-Chloro-2-hydroxybenzaldehyde
   2,3-Dichloro-5,6-dicyano-1-4-benzoquinone (DDQ, 9.92 g, 43.7 mmol) was added to a solution of 5-chloro-2-hydroxymethylphenol in dichloromethane (65 mL) and tetrahydrofuran (15 mL),. The reaction mixture was stirred at room temperature for 4 h. Then, the solvent was evaporated under vacuum and the crude was purified by chromatography on silica to afford the title compound (72%): ¹H NMR (400 MHz, CDCl₃) δ 11.17 (s, 1H), 9.86 (s, 1H), 7.49 (d, *J=* 7.3 Hz, 1H), 7.00 (m, 2H).
c) 2-Allyloxy-4-chlorobenzaldehyde
   A mixture of 4-chloro-2-hydroxybenzaldehyde (254 mg, 1.62 mmol) and cesium carbonate (793 mg, 2.43 mmol) in THF (3 mL) was heated to reflux for 3 h. After the reaction mixture was cooled at room temperature, the solvent was evaporated and the residue partitioned between ethyl acetate and hydrochloric acid (1N). The organic layer was collected, dried with sodium sulfate, filtered and concentrated under vacuum. The residue was purified by column chromatography (AcOEt/Hexanes, 1:9) to give the title product (97%): ¹H NMR (400 MHz, Cl₃CD) δ 10.46 (s, 1H), 7.80 (d, *J=* 8.3 Hz, 1H), 7.05 (dd, *J =* 0.78, 8.3 Hz, 1H), 6.99 (d, *J =* 0.78 Hz, 1H), 6.09 (m, 1H), 5.45 (m, 2H), 4.67 (m, 2H).
d) (3*S*)-3-(2-Allyloxy-4-chlorophenyl)-4-[(*R*)-1-(4-chlorophenyl)ethyl]-7-iodo-1,3-dihydro-1,4-benzodiazepine-2,5-dione
   The title compound was prepared following the general procedure for the synthesis of diazepines and was identified as the "lower diastereomer" (in a TLC performed with AcOEt/Hexanes, 1:1): ¹H NMR (400 MHz, CDCl₃) δ¹H NMR 9.49 (s, 1H), 7.99 (d, *J =* 1.9 Hz, 1H), 7.38 (m, 3H), 7.15 (d, J = 8.4 Hz, 2H), 6.40 (m, 3H), 6.32 (m, 1H), 6.24 (d, J= 7.8 Hz, 1H), 5.87 (m, 1H), 5.29 (m, 3H), 4.31 (m, 1H), 1.62 (d, J= 7.2 Hz, 3H). In this reaction (3R)-3-(2-allyloxy-4-chlorophenyl)-4-[(*R*)-1-(4-chlorophenyl)ethyl]-7-iodo-1,3-dihydro-1,4-benzodiazepine-2,5-dione was obtained and identified as the "upper diastereomer" (in a TLC performed with AcOEt/Hexanes, 1:1): ¹H NMR (400 MHz, CDCl₃) δ 9.22 (s, 1H), 8.06 (d, J= 1.6 Hz, 1H), 7.45 (dd, *J*= 1.6, 8.3 Hz, 1H), 7.22 (d, *J*= 8.3 Hz, 2H), 7.08 (d, J= 8.4 Hz, 2H), 6.76 (d, J= 8.4 Hz, 1H), 6.65 (dd, J = 1.3, J = 8.4 Hz, 1H), 6.59 (d, *J*= 1.3 Hz, 1H), 6.28 (m, 2H), 5.83 (m, 1H), 5.24 (m, 1H), 5.05 (s, 1H), 4.35 (m, 2H), 1.50 (d, *J* = 7.1 Hz, 3H).
e) 5-[(3*S*)-3-(2-Allyloxy-4-chlorophenyl)-4-[(*R*)-1-(4-chlorophenyl)ethyl]-7-iodo-2,5-dioxo-1,4-benzodiazepin-1-yl]valeric acid
   The title compound was prepared from (3*S*)-3-(2-allyloxy-4-chlorophenyl)-4-[(*R*)-1-(4-chlorophenyl)ethyl]-7-iodo-1,3-dihydro-1,4-benzodiazepine-2,5-dione following the general procedure for the alkylation at the 1-position, followed by deprotection following the procedures described for example 1, step b: ¹H NMR (400 MHz, CDCl₃) δ 8.63 (br s, 1H), 7.94 (d, *J*= 2.1 Hz, 1H), 7.45 (m, 3H), 7.26 (d, *J* = 8.5 Hz, 2H), 6.56 (d, *J* = 8.6 Hz, 1H), 6.48 (d, *J* = 1.9 Hz, 1H), 6.43 (m, 1H), 6.35 (m, 1H), 6.16 (m, 1H), 5.96 (m, 1H), 5.35 (m, 2H), 5.23 (s, 1H), 4.42 (m, 2H), 4.20 (m, 1H), 3.54 (m, 1H), 2.34 (m, 2H), 1.75 (d, *J* = 7.1 Hz, 3H), 1.56 (m, 4H). Mass spectrum (LCMS, ESI pos.): Calcd for C₃₁H₂₉Cl₂IN₂O₅: 706.05; found 706.7 (M+H).

### EXAMPLE 55

5-[(3*S*)-3-(4-chloro-2-hydroxyphenyl)-4-[(*R*)-1-(4-chlorophenyl)ethyl]-7-iodo-2,5-dioxo-1,4-benzodiazepin-1-yl]valeric acid 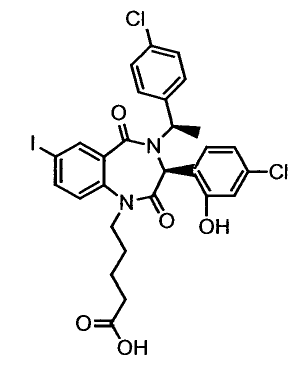
a) 5-[(3*S*)-3-(4-chloro-2-hydroxyphenyl)-4-[(*R*)-1-(4-chlorophenyl)ethyl]-7-iodo-2,5-dioxo-1,4-benzodiazepin-1-yl]valeric acid *tert*-butyl ester
   5-[(3*S*)-3-(2-Allyloxy-4-chlorophenyl)-4-[(*R*)-1-(4-chlorophenyl)ethyl]-7-iodo-2,5-dioxo-1,4-benzodiazepin-1-yl]valeric acid *tert*-butyl ester (224 mg, 0.29 mmol) and dichloro *bis*(triphenylphosphine)palladium (II) (10 mg, 0.015 mmol) were dissolved at room temperature in THF (5 mL). Sodium borohydride (11 mg, 0.29 mmol) was added and the reaction was stirred under argon for 30 minutes. Methanol (10 mL) was added and the reaction was stirred for 15 additional minutes. The solvent was evaporated under vacuum and the residue was purified by column chromatography (AcOEt/Hexanes, 1:2) to give 200 mg of the title compound contaminated with 5-[(3S)-3-(2-Allyloxy-4-chlorophenyl)-4-[(*R*)-1-(4-chlorophenyl)ethyl]-2,5-dioxo-1,4-benzodiazepin-1-yl]valeric acid tert-butyl ester. This mixture was carried out to the next step without further purification.
b) 5-[(3*S*)-3-(4-Chloro-2-hydroxyphenyl)-4-[(*R*)-1-(4-chlorophenyl)ethyl]-7-iodo-2,5-dioxo-1,4-benzodiazepin-1-yl]valeric acid.
   The title compound was synthesized from 5-[(3*S*)-3-(4-chloro-2-hydroxyphenyl)-4-[(*R*)-1-(4-chlorophenyl)ethyl]-7-iodo-2,5-dioxo-1,4-benzodiazepin-1-yl]valeric acid tert-butyl ester following the procedure for example 1, step b: ¹H NMR (400 MHz, Cl₃CD) δ 7.99 (d, J = 2.1 Hz, 1H), 7.42 (m, 3H), 7.22 (d, J = 8.5 Hz, 2H), 6.62 (d, J = 8.6 Hz, 1H), 6.50 (d, J = 1.9 Hz, 1H), 6.34 (m, 2H), 6.09 (m, 1H), 5.26 (s, 1H), 3.87 (m, 1H), 3.74 (m, 1H), 2.39 (m, 2H), 1.75 (m, 7H). Mass spectrum (LCMS, ESI pos.): Calcd for C₂₈H₂₅Cl₂IN₂O₅: 666.02; found 666.6 (M+H); together with 21 mg 5-[(3S)-3-(4-chloro-2-hydroxyphenyl)-4-[(*R*)-1-(4-chlorophenyl)ethyl]-2,5-dioxo-1,4-benzodiazepin-1-yl]valeric acid: ¹H NMR (400 MHz, Cl₃CD) δ 7.67 (m, 1H), 7.41 (d, J = 8.4 Hz, 2H), 7.21 (d, J = 8.4 Hz, 2H), 7.15 (m, 1H), 7.06 (m, 1H), 6.88 (d, *J=* 8.1 Hz, 1H), 6.54 (d, *J* = 1.9 Hz, 1H), 6.35 (m, 1H), 6.27 (dd, J = 1.9, *J =* 8.4 Hz, 1H), 6.10 *(d, J =* 8.4 Hz, 1H), 5.26 (s, 1H), 3.85 (m, 2H), 2.39 (m, 2H), 1.72 (m, 7H). Mass spectrum (LCMS, ESI pos.): Calcd for C₂₈H₂₆Cl₂N₂O₅: 540.1; found 540.8 (M+H).

### EXAMPLE 56

5-[(3S)-3-(4-Chlorophenyl)-4-[(*R*)-1-(4-chlorophenyl)ethyl]-2,5-dioxo-7-phenyl-1,4-diazepin-1-yl]valeric acid sodium salt 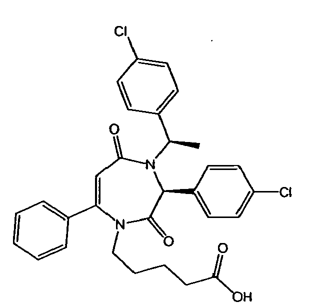
a) α-Bromo-α-(4-chlorophenyl)acetic acid methyl ester
   A solution of 4-chlorophenylacetic acid methyl ester (14.6 g, 79.1 mmol), N-bromosuccinimide (14.4 g, 80.7 mmol), and benzoyl peroxide (1.91 g, 7.89 mmol) in carbon tetrachloride (100 mL) was heated at reflux for 3 h. After the mixture was cooled at room temperature, hexanes (500 mL) was added. The reaction mixture was filtered and the solvent was evaporated in vacuo. The crude material was purified by column chromatography on silica (EtOAc/Hexanes, 15:85) to give the title compound as colorless oil (16.9 g, 63%): ¹H NMR (400 MHz, CDCl₃) δ 3.81 (s, 3H, CH₃), 5.34 (s, 1H, CH), 7.44 (dd, *J =* 60.4 Hz, *J =* 8.4 Hz, 4H, ArH).
b)α-(4-Chlorophenyl)-α-[(*R*)-*N*-[1-(4-chlorophenyl)ethyl]amino]acetic acid methyl ester hydrochloride
   A mixture of α-bromo-α-(4-chlorophenyl)acetic acid methyl ester (2.63 g, 10 mmol), (*R*)-1-(4-chlorophenyl)ethylamine (1.55 g, 10 mmol), potassium carbonate (2.76 g, 20 mmol), and tetrabutylammonium iodide (500 mg, 1.35 mmol) in dry acetonitrile (10 mL) was heated at 45 °C for 12 h. After the mixture was cooled at room temperature, the solvent was evaporated in vacuo. Then, the residue was partitioned between ice-cold water (50 mL) and ethyl acetate (70 mL). The aqueous layer was extracted twice with additional ethyl acetate (2 x 50 mL) and dried (Na₂SO₄). After evaporation of the solvent, the residue was chromatographed on silica (EtOAc/hexanes, 5:95 to 15:85), then converted to the HCl salt to give the title compound as a colorless powder (2.5 g, 74%, mixture of two diastereomers): ¹H NMR (400 MHz, CD₃OD) δ 1.74 (t, J = 6.8 Hz, 3H, CH₃), 3.69 (s, 1.5 H, 0.5 CH₃), 3.82 (s, 1.5 H, 0.5 CH₃), 4.41 (q, J = 6.8 Hz, 0.5 H, 0.5 CH), 4.49 (q, J = 6.8 Hz, 0.5 H, 0.5 CH), 4.99 (s, 0.5 H, 0.5 CH), 5.12 (s, 0.5 H, 0.5 CH), 7.42-7.56 (m, 8H, ArH). Mass spectrum (LCMS, ESI pos.): Calcd for C₁₇H₁₇Cl₂NO₂: 337.0; found: 338.0 (M + H)⁺.
c) (*R*,*S*)-α-(4-Chlorophenyl)-α-[(*R*)-*N*-[1-(4-chlorophenyl)ethyl]-*N-*(cinnamoyl)amino] acetic acid methyl ester
   To a stirred solution of (*R*,*S*)-α-(4-chlorophenyl)-α-[(*R*)-*N*-[1-(4-chlorophenyl)ethyl]amino]acetic acid methyl ester (1.0 g, 2.96 mmol) and 4-*(N,N*-dimethylamino)pyridine (100 mg, 0.82 mmol) in dichloromethane (30 mL) at 0°C was added cinnamoyl chloride (542 mg, 3.25 mmol). Then, *N,N-*diisopropylethylamine (2.0 mL, 11.5 mmol) was slowly added. After 20 min. of stirring at 0 °C, the reaction mixture was allowed warm up to 25 °C for 1 h., then diluted with ethyl acetate, washed with water, dried (Na₂SO₄), and concentrated to dryness under reduced pressure. Chromatography on silica (AcOEt/hexanes, 1:8) afforded the title compound (1.0 g, 74%) as a slightly yellow solid: Mass spectrum (LCMS, ESI pos.): Calcd for C₂₆H₂₃Cl₂NO₃: 467.0; found: 468.0 (M + H)⁺.
d)α-(4-Chlorophenyl)-α-[(*R*)-*N*-[1-(4-chlorophenyl)ethyl]-*N-*(cinnamoyl)amino] acetic acid
   A solution of (*R,S*)*-α-*(4-chlorophenyl)-α-[(*R*)-*N*-[1-(4-chlorophenyl)ethyl]-*N-*(cinnamoyl)amino]acetic acid methyl ester (453 mg, 1.0 mmol) and sodium hydroxide (600 mg, 15.0 mmol) in tetrahydrofuran (5 mL), methanol (8 mL), and water (2 mL) was stirred for 2 h at 25°C. The reaction mixture was concentrated under reduced pressure and the pH adjusted to 4 with 1N HCl. The resulting solution was extracted twice with AcOEt (20 mL), dried (Na₂SO₄) and evaporated to give the title compound as a colorless powder (420 mg, 92%, mixture of two diastereomers): ¹H NMR (400 MHz, CDCl₃) δ 1.62 (d, J = 7.0 Hz, 1.5 H, 0.5 CH₃), 1.84 (d, J = 7.0 Hz, 1.5 H, 0.5 CH₃), 4.84 (s, 1H, CH), 4.88 (s, 1H, CH), 5.45-5.53 (m, 1H, CH), 6.93-7.91 (m, 15H, 13 ArH + 2 CH). Mass spectrum (LCMS, ESI pos.): Calcd for C₂₅H₂ₗCl₂NO₃: 453.0; found: 454.0 (M + H)⁺.
e) (S)-α-(4-Chlorophenyl)-α-[(*R*)-N 1-(4-chlorophenyl)ethyl]-*N-*(cinnamoyl)amino] acetamide
   To a stirred solution of (*R*,*S*)-α-(4-Chlorophenyl)-α-[(*R*)-*N*-[1-(4-chlorophenyl)ethyl]-*N*-(cinnamoyl)amino]acetic acid (400 mg, 0.88 mmol), *O-*(7-azabenzotriazol-1-yl)-*N,N,N'*, *N'*-tetramethyluronium hexafluorophosphate (502 mg, 1.32 mmol), ammonium chloride (94 mg, 1.76 mmol), and 1-hydroxybenzotriazole (178 mg, 1.32 mmol) in dimethylformamide (3mL) was slowly added *N,N*-diisopropylethylamine (613 µL, 3.52 mmol). After 2 h, the reaction mixture was partitioned between ethyl acetate (100 mL) and water (60 mL), dried (Na₂SO₄), and evaporated. Purification by column chromatography (AcOEt/hexanes, 2:8) afforded the title compound (150 mg, 38%, "lower diastereomer in a TLC performed with AcOEt/Hexanes, 1:3") as a colorless solid: ¹H NMR (400 MHz, CDCl₃) δ 1.86 (d, J = 6.8 Hz, 3H, CH₃), 4.76 (s, 1H, CH), 5.40-5.49 (m, 1H, CH), 5.62 (br s, 1H, NH), 6.32 (br s, 1H, NH), 6.90-7.86 (m, 15H, 13 ArH + 2 CH). Mass spectrum (LCMS, ESI pos.): Calcd for C₂₅H₂₂Cl₂N₂O₂: 452.0; found: 453.0 (M + H)⁺.
f) (3*S*)-1,3,6,7-Tetrahydro-3-(4-chlorophenyl)-4-[(*R*)-1-(4-chlorophenyl)ethyl]-7-phenyl-6-(phenylseleno)-1,4-diazepine-2,5-dione
   Silver triflate (1.69 g, 6.6 mmol) was added to a stirred solution of (*S*)-α-(4-chlorophenyl)-α-[(*R*)-*N*-[1-(4-chlorophenyl)ethyl]-*N-*(cinnamoyl)amino]acetamide (1.5 g, 3.3 mmol), in acetonitrile (120 mL). To the resulting solution was successively added benzeneselenyl bromide (1.54 g, 6.53 mmol) and dimethylformamide (5.2 mL). After 6 h, the solvent was evaporated in vacuo. The residue was partitioned between ethyl acetate (100 mL) and 1N sodium hydrogenocarbonate (70 mL), dried (Na₂SO₄), and evaporated. Purification by column chromatography (AcOEt/hexanes, 1:3) afforded the title compound, which was recrystallized from Et₂O to give colorless prisms (855 mg, 43%): ¹H NMR (400 MHz, CDCl₃) δ 1.68 (d, J = 7.0 Hz, 3H, CH₃), 4.29 (d, J = 12.2 Hz, 1H, CH), 4.79 (d, J = 12.2 Hz, 1H, CH), 5.28 (s, 1H, CH), 6.21-6.25 (m, 3H, ArH), 6.31 (q, J = 7.0 Hz, 1H, CH), 6.71-6.76 (m, 4H, ArH), 6.93-7.11 (m, 6H, NH + 5ArH), 7.34-7.40 (m, 4H, ArH), 7.50 (d, J = 8.4 Hz, 2H, ArH). Mass spectrum (LCMS, ESI pos.): Calcd for C₃₁H₂₆Cl₂N₂O₂Se: 608.0; found: 609.0 (M + H)⁺.
g) (3*S*)-1,3-Dihydro-3-(4-chlorophenyl)-4-[(*R*)-1-(4-chlorophenyl)ethyl]-7-phenyl-1,4-diazepine-2,5-dione
   Hydrogen peroxide (500 µL, 30% in water) was slowly added to a solution of (3*S*)-1,3,6,7-tetrahydro-3-(4-chlorophenyl)-4-[(*R*)-1-(4-chlorophenyl)ethyl]-7-phenyl-6-(phenylseleno)-1,4-diazepine-2,5-dione (300 mg, 0.49 mmol) in tetrahydrofuran (6 mL). After 20 min., the solvent was evaporated under reduced pressure and the residue was partitioned between 1N sodium hydrogenocarbonate (20 mL) and ethyl acetate (50 mL), dried (Na₂SO₄) and evaporated. Purification by column chromatography (AcOEt/hexanes/CH₂Cl₂, 1:2:2) afforded the title compound (200 mg, 85%) as a colorless powder: ¹H NMR (400 MHz, CDCl₃) δ 1.70 (d, J = 7.2 Hz, 3H, CH₃), 5.28 (s, 1H, CH), 5.70 (d, *J =* 1.6 Hz, 1H, CH), 6.35 (q, *J =* 7.2 Hz, 1H, CH), 6.83 (d, J = 8.8 Hz, 2H, ArH), 7.09 (d, J = 8.8 Hz, 2H, ArH), 7.18-7.42 (m, 7H, ArH), 7.53 (d, J = 8.8 Hz, 2H, ArH). Mass spectrum (LCMS, ESI pos.): Calcd for C₂₅H₂₀Cl₂N₂O₂: 450.0; found: 451.0 (M + H)⁺.
h) 5-[(3*S*)-3-(4-Chlorophenyl)4-[(*R*)-1-(4-chlorophenyl)ethyl]-2,5-dioxo-7-phenyl-1,4-diazepin-1-yl]valeric acid sodium salt.
   The title compound was synthesized from (3*S*)-1,3-Dihydro-3-(4-chlorophenyl)-4-[(*R*)-1-(4-chlorophenyl)ethyl]-7-phenyl-1,4-diazepine-2,5-dione following the general procedure for the alkylation of the 1-position, followed by deprotection following the procedure for example 1, step b: ¹H NMR (400 MHz, CDCl₃) δ 1.45-1.53 (m, 4H, 2 CH₂), 1.68 (d, *J*= 7.2 Hz, 3H, CH₃), 2.29 (t, *J* = 6.8 Hz, 2H, CH₂), 2.87-2.93 (m, 1H, CH), 4.15-4.23 (m, 1H, CH), 5.40 (s, 1H, CH), 5.72 (s, 1H, CH), 6.32 (q, *J =* 7.2 Hz, 1H, CH), 6.81 (d, J = 7.6 Hz, 2H, ArH), 7.02-7.08 (m, 4H, ArH), 7.26-7.34 (m, 5H, ArH), 7.48 (d, J = 8.4 Hz, 2H, ArH). Mass spectrum (LCMS, ESI pos.): Calcd for C₃₀H₁₈Cl₂N₂O₄: 550.0; found: 551.0 (M + H)⁺.

### EXAMPLE 57

5-[(3*S*)-7-(2-Bromophenyl)-3-(4-chlorophenyl)-4-[(*R*)-1-(4-chlorophenyl)ethyl]-2,5-dioxo-1,4-diazepin-1-yl]valeric acid sodium salt. 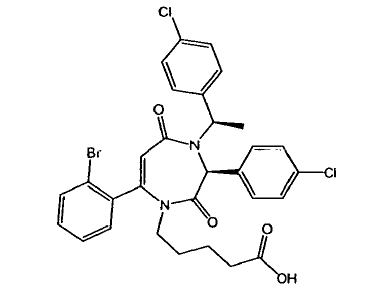 ¹H NMR (400 MHz, CDCl₃) δ 1.46-1.53 (m, 4H, 2 CH₂), 1.69 (d, *J=* 7.2 Hz, 3H, CH₃), 2.29 (t, *J =* 6.8 Hz, 2H, CH₂), 2.87-2.93 (m, 1H, CH), 4.15-4.23 (m, 1H, CH), 5.41 (s, 1H, CH), 5.66 (s, 1H, CH), 6.31 (q, *J =* 7.2 Hz, 1H, CH), 6.88 (d, J = 7.6 Hz, 2H, ArH), 7.10-7.60 (m, 10H, ArH). Mass spectrum (LCMS, ESI pos.): Calcd for C₃₀H₂₇BrCl₂N₂O₄: 628.0; found: 629.0 (M + H)⁺.

### EXAMPLE 58

5-[(3*S*)-3-(4-Chlorophenyl)-4-[(*R*)-1-(4-chlorophenyl)ethyl]-7-(2,5-dimethylphenyl)-2,5-dioxo-1,4-diazepin-1-yl]valeric acid sodium salt. 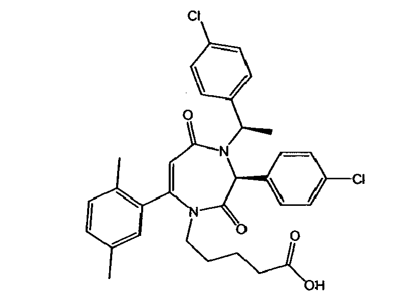 ¹H NMR (400 MHz, CDCl₃) δ 1.43-1.63 (m, 4H, 2 CH₂), 1.70 (d, *J =* 7.2 Hz, 3H, CH₃), 2.04 (s, 3H, CH₃), 2.12 (s, 3H, CH₃), 2.27-2.34 (m, 2H, CH₂), 2.87-2.93 (m, 1H, CH), 4.15-4.23 (m, 1H, CH), 5.51 (s, 1H, CH), 5.75 (s, 1H, CH), 6.26 (q, J = 7.2 Hz, 1H, CH), 6.77 (d, *J =* 7.6 Hz, 2H, ArH), 6.80-7.65 (m, 9H, ArH). Mass spectrum (LCMS, ESI pos.): Calcd for C₃₂H₃₂Cl₂N₂O₄: 578.0; found: 579.0 (M + H)⁺.

### EXAMPLE 59

5-[(3*S*)-3-(4-Chlorophenyl)-4-[(*R*)-1-(4-chlorophenyl)ethyl]-7-(2-methylphenyl)-2,5-dioxo-1,4-benzodiazepine-1-yl]valeric acid sodium salt 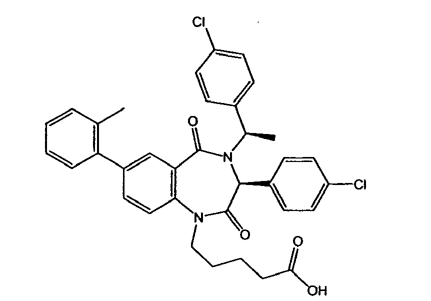
a) (3S)-3-(4-Chlorophenyl)-4-[(R)-1-(4-chlorophenyl)ethyl]-7-(2-methylphenyl)-1,4-benzodiazepine-2,5-dione
   A solution of (3*S*)-3-(4-chlorophenyl)-4-[(R)-1-(4-chlorophenyl)ethyl]-7-iodo-1,4-benzodiazepine-2,5-dione (120 mg, 0.22 mmol), *tetrakis*(triphenylphosphine)palladium(0) (25 mg, 0.022 mmol), 2-methylbenzene boronic acid (35 mg, 0.26 mmol) and sodium carbonate (220 µL, 2M in H₂O) in ethanol (300 µL) and toluene (5 mL) was heated under argon, at 90°C, for 10 h. After the mixture was cooled at room temperature, the solvent was evaporated in vacuo. The residue was partitioned between AcOEt (20 mL) and water (15 mL), dried (Na₂SO₄) and evaporated. Chromatography on silica (AcOEt/hexanes/CH₂Cl₂, 1:6:3) afforded the title compound (92 mg, 68%) as a colorless powder: ¹H NMR (400 MHz, CDCl₃) δ 1.78 (d, *J*= 7.2 Hz, 3H, CH₃), 1.99 (s, 3H, CH₃), 5.29 (s, 1H, CH), 6.51 (q, *J =* 7.2 Hz, 1H, CH), 6.64-6.67 (m, 2H, ArH), 6.71 (d, *J* = 8.4 Hz, 1H, ArH), 6.93 (d, *J* = 8.4 Hz, 2H, ArH), 7.05-7.11 (m, 1H, ArH), 7.15 (dd, *J =* 8.0 Hz, *J* = 2.0 Hz, 1H, ArH), 7.20-7.26 (m, 3H, ArH), 7.35 (d, *J* = 8.4 Hz, 2H, ArH), 7.56 (d, *J* = 8.4 Hz, 2H, ArH), 7.61 (d, *J* = 2.0 Hz, 1H, ArH), 7.91 (br s, 1H, NH). Mass spectrum (LCMS, ESI pos.): Calcd for C₃₀H₂₄Cl₂N₂O₂: 514.0; found: 515.0 (M + H)⁺.
b) 5-[(3*S*)-3-(4-Chlorophenyl)-4-[(*R*)-1-(4-chlorophenyl)ethyl]-7-(2-methylphenyl)-2,5-dioxo-1,4-benzodiazepin-1-yl]valeric acid sodium salt.
   The title compound was prepared from (3*S*)-3-(4-chlorophenyl)-4-[(*R*)-1-(4-chlorophenyl)ethyl]-7-(2-methylphenyl)-1 ,4-benzodiazepine-2,5-dione following successively the general procedure for the alkylation at the 1-position followed by the deprotection procedure described for example 1, step b: ¹H NMR (400 MHz, CDCl₃) δ 1.65 (m, 7H, CH₃ + 2 CH₂), 1.98 (s, 3H, CH₃), 2.41 (t, J = 7.6 Hz, 2H, CH₂), 3.70-3.77 (m, 1H, CH), 4.39-4.46 (m, 1H, CH), 5.37 (s, 1H, CH), 6.44 (q, J = 7.2 Hz, 1H, CH), 6.50-6.53 (m, 2H, ArH), 6.86 (d, J = 8.8 Hz, 2H, ArH), 6.91 (d, J = 8.4 Hz, 1H, ArH), 7.06-7.15 (m, 2H, ArH), 7.20-7.26 (m, 3H, ArH), 7.32 (d, J = 8.4 Hz, 2H, ArH), 7.50-7.52 (m, 3H, ArH). Mass spectrum (LCMS, ESI pos.): Calcd for C₃₅H₃₂Cl₂N_{Z}O₄: 614.0; found: 615.0 (M + H)⁺.

### EXAMPLE 60

5-[4-[1-(2-Amino-4-chlorophenyl)ethyl]-3-(4-chlorophenyl)-7-iodo-2,5-dioxo-1,4-benzodiazepin-1-yl]valeric acid sodium salt 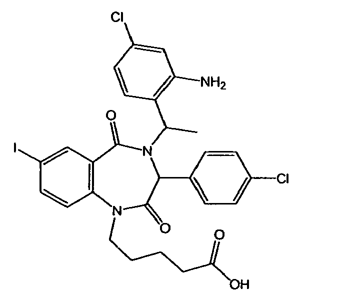
a) 1-(4-Chloro-2-nitrophenyl)ethanol
   A solution of methyl lithium (46 mL, 1.4 M in Et₂O), was slowly added under argon, at -78°C, to a stirred solution of 4-chloro-2-nitrobenzaldehyde (10g, 53.9 mmol) in dry tetrahydrofuran (150 mL). After 20 min. at -78°C, the reaction mixture was quenched with water (400 mL), extracted twice with ethyl acetate (500 mL), dried (Na₂SO₄), and concentrated to dryness under reduced pressure. Chromatography on silica (EtOAc/CH₂Cl₂/EtOH, 1:6:1) yielded the title compound (7.2 g, 66%) as yellow oil: ¹H NMR (400 MHz, CDCl₃) δ 1.56 (d, *J* = 6.4 Hz, 3H, ArH), 2.40 (br s, 1H, OH), 5.42 (q, *J* = 6.4 Hz, 1H, CH), 7.63 (dd, *J =* 8.4 Hz, *J =* 2.0 Hz, 1H, ArH), 7.82 (d, *J =* 8.4 Hz, 1H, ArH), 7.92 (d, *J* = 2.0 Hz, 1H, ArH).
b) [(*S*)-1-(4-Chloro-2-nitrophenyl)ethyl] (1*S*)-camphanoate and [(*R*)-1-(4-Chloro-2-nitrophenyl)ethyl] (1*S*)-camphanoate
   Diisopropylethylamine (9.3 mL, 53.3 mmol) was slowly added at 0 °C under argon to a solution of (*R*,*S*)-1-(4-chloro-2-nitrophenyl)ethanol (10.2 g, 50.8 mmol), 4-dimethylaminopyridine (244 mg, 2.0 mmol) and (1*S*)-camphanyl chloride (11.0 g, 50.8 mmol) in tetrahydrofuran (70 mL). Then, the reaction mixture was allowed warm up to room temperature. After 8 h, the solvent was evaporated in vacuo and the residue was partitioned between CH₂Cl₂ and 1N NaHCO₃, dried (Na₂SO₄) and filtered through silica gel. The solvent was evaporated under reduced pressure. The residue was dissolved in chloroform (100 mL), then hexanes (300 mL) was slowly added. After 10 min, the title compound started to crystallize. After 20 min, the prisms were filtered off and washed with small portions of dichloromethane/hexanes (1:5). This compound (4.8 g, 25%) was characterized as the title compound: ¹H NMR (400 MHz, CDCl₃) δ 1.03 (s, 3H, CH₃), 1.07 (s, 3H, CH₃), 1.15 (s, 3H, CH₃), 1.70 (d, *J=* 6.4 Hz, 4H, 2 CH₂), 1.88-2.01 (m, 2H, CH₂), 2.35-2.42 (m, 1H, CH), 6.52 (q, *J* = 6.4 Hz, 1H, CH), 7.64 (dd, *J =* 8.8 Hz, *J* = 2.4 Hz, 1H, ArH), 7.71 (d, *J =* 8.8 Hz, 1H, ArH), 7.99 (d, *J =* 2.0 Hz, 1H, ArH). The filtrate was evaporated and the residue recrystallized in ether to give the other diastereomer [(*R*)-1-(4-Chloro-2-nitrophenyl)ethyl] (1*S*)-camphanoate.
c) (*R,S*)-*N*-[1-(4-Chloro-2-nitrophenyl)ethyl]phthalimide
   Diisopropyl azodicarboxylate (1.46 mL, 7.44 mmol) was added under argon, at -78°C, to a solution of (*R,S*)*-*1-(4-chloro-2-nitrophenyl)ethanol (1.0 g, 4.96 mmol), triphenyl phosphine (730 mg, 4.96 mmol), and phthalimide (730 mg, 4.96 mmol) in tetrahydrofuran (20 mL). Then, the reaction mixture was allowed warm up to 25 °C. After 2 h, the solvent was evaporated under reduced pressure. Chromatography on silica (AcOEt/hexanes, 1:1) afforded the title compound (1.4 g, 85%) as a colorless solid: ¹H NMR (400 MHz, CDCl₃) δ 1.97 (d, *J* = 7.2 Hz, 3H, CH₃), 6.04 (q, *J =* 7.2 Hz, 1H, ArH), 7.58-7.61 (m, 1H, ArH), 7.72-7.75 (m, 2H, ArH), 7.81-7.84 (m, 3H, ArH), 7.81 (d, *J* = 8.8, 1H, ArH).
d) (*R*,*S*)-1-(4-Chloro-2-nitrophenyl)ethylamine
   A mixture of (*R*,*S*)-*N*-[1-(4-chloro-2-nitrophenyl)ethyl]phthalimide (1.2 g, 3.63 mmol) and hydrazine (1.2 mL, 38.2 mmol) in ethanol (10 mL) was heated at reflux for 1 h. After the mixture was cooled at room temperature, the precipitate was filtered off and the solvent was evaporated in vacuo. The crude material was purified by column chromatography on silica (EtOAc/EtOH/CH₂Cl₂, 4:1:5) to give the title compound (600 mg, 82%) as a colorless oil: ¹H NMR (400 MHz, CDCl₃) δ 1.44 (d, *J =* 6.5 Hz, 3H, CH₃), 1.58 (br s, 2H, NH₂), 4.60 (q, *J =* 6.5 Hz, 1H, CH), 7.58 (dd, *J =* 8.8 Hz, *J* = 2.0 Hz, 1H, ArH), 7.79-7.81 (m, 2H, ArH).
e) 5-[3-(4-Chlorophenyl)-4-[1-(4-chloro-2-nitrophenyl)ethyl]-7-iodo-2,5-dioxo-1,4-benzodiazepin-1-yl]valeric acid tert-butyl ester
   The title compound was prepared from (*R*,*S*)-1-(4-chloro-2-nitrophenyl)ethylamine following the general proceuder for the synthesis of diazepines: ¹H NMR (400 MHz, CDCl₃) δ 1.45 (s, 9H, 3 CH₃), 1.60-1.69 (m, 4H, 2 CH₂), 1.79 (d, J = 6.8 Hz, 3H, CH₃), 2.22 (t, J = 7.0 Hz, 2H, CH₂), 3.58-3.67 (m, 1H, CH), 4.17-4.25 (m, 1H, CH), 5.38 (s, 1H, CH), 6.52 (d, J = 8.8 Hz, 1H, ArH), 6.62-6.70 (m, 3H, 2 ArH + CH), 6.94 (d, J = 8.8 Hz, 2H, ArH), 7.45 (dd, *J =* 8.4 Hz, *J =* 2.0 Hz, 1H, ArH), 7.56 (dd, J = 8.8 Hz, *J =* 2.4 Hz, 1H, ArH), 7.67 (d, J = 8.4 Hz, 1H, ArH), 7.86 (d, J = 2.4 Hz, 1H, ArH), 7.97 (d, J= 2.0 Hz, 1H, ArH).
f) 5-[4-[1-(2-Amino-4-chlorophenyl)ethyl]-3-(4-chlorophenyl)-7-iodo-2,5-dioxo-1,4-benzodiazepin-1-yl]valeric acid *tert*-butyl ester
   A solution of ammonium chloride (220 mg, 4.3 mmol) in water (2.5 mL) was added to a solution of 5-[3-(4-chlorophenyl)-4-[1-(4-chloro-2-nitrophenyl)ethyl]-7-iodo-2,5-dioxo-1,4-benzodiazepin-1-yl]valeric acid *tert-*butyl ester (230 mg, 0.306 mmol) in ethanol (5 mL). The resulting solution was heated to 80 °C and iron (200 mg, 3.58 mmol) was added. After 2 h at 80°C, the reaction mixture was cooled at room temperature, filtered through celite and evaporated. The residue was partitioned between ethyl acetate and water, dried (Na₂SO₄), and evaporated. Chromatography on silica (EtOAc/hexanes, 1:1) afforded the title compound (210 mg, 95%) as a colorless solid: ¹H NMR (400 MHz, CDCl₃) δ 1.44 (s, 9H, 3 CH₃), 1.57-1.63 (m, 4H, 2 CH₂), 1.74 (d, *J =* 7.2 Hz, 3H, CH₃), 2.24 (t, *J =* 7.2 Hz, 2H, CH₂), 3.63-3.70 (m, 1H, CH), 4.28-4.35 (m, 1H, CH), 4.75 (br s, 2H, NH₂), 5.28 (s, 1H, CH), 6.27 (q, J = 7.2 Hz, 1H, CH), 6.60-6.69 (m, 5H, ArH), 6.90 (d, J = 9.2 Hz, 2H, ArH), 7.20 (d, *J =* 8.4 Hz, 1 H, ArH), 7.49 (dd, J = 8.8 Hz, *J =* 2.4 Hz, 1H, ArH), 7.93 (d, J = 2.0 Hz, 1H, ArH). Mass spectrum (LCMS, ESI pos.): Calcd for C₃₂H₃₄Cl₂IN₃O₄: 721.0; found: 722.0 (M + H)⁺.
g) 5-[4-[1-(2-Amino-4-chlorophenyl)ethyl]-3-(4-chlorophenyl)-7-iodo-2,5-dioxo-1,4-benzodiazepin-1-yl]valeric acid sodium salt
   The title compound was prepared (76%) from 5-[4-[1-(2-amino-4-chlorophenyl)ethyl]-3-(4-chlorophenyl)-7-iodo-2,5-dioxo-1,4-benzodiazepin-1-yl]valeric acid *tert-*butyl ester following the procedure described for example 1, step b: ¹H NMR (400 MHz, CDCl₃) δ 1.63-1.68 (m, 4H, 2 CH₂), 1.75 (d, J = 6.8 Hz, 3H, CH₃), 2.37-2.41 (m, 2H, CH₂), 3.64-3.71 (m, 1H, CH), 4.33-4.40 (m, 1H, CH), 5.29 (s, 1H, CH), 6.26 (q, J = 6.8 Hz, 1H, CH), 6.61-6.68 (m, 5H, ArH), 6.90 (d, J = 8.8 Hz, 2H, ArH), 7.21 (d, J = 8.4 Hz, 1H, ArH), 7.50 (dd, *J =* 8.8 Hz, J = 2.4 Hz, 1H, ArH), 7.93 (d, *J =* 2.0 Hz, 1H, ArH). Mass spectrum (LCMS, ESI pos.): Calcd for C₂₈H₂₆Cl₂IN₃O₄: 665.0; found: 666.0 (M + H)⁺.

### EXAMPLE 61

5-[4-[(2-Amino-4-chlorobenzyl]-7-bromo-3-(4-chlorophenyl)-2,5-dioxo-1,4-benzodiazepin-1-yl]valeric acid sodium salt 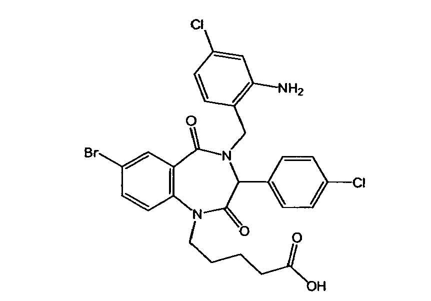
a) 5-[7-Bromo-3-(4-chlorophenyl)-4-[(4-chloro-2-nitrobenzyl]-2,5-dioxo-1,4-benzodiazepin-1-yl]valeric acid *tert*-butyl ester
   The title compound was prepared (63%) from 5-[7-bromo-3-(4-chlorophenyl)-3,4-dihydro-2,5-dioxo-1,4-benzodiazepin-1-yl]valeric acid *tert*-butyl ester and 4-chloro-2-nitrobenzyl chloride following the procedure described for the general procedure for the dialkylation of the benzodiazepine: Mass spectrum (LCMS, ESI pos.): Calcd for C₃₁H₃₀BrCl₂N₃O₆: 689.0; found: 690.0 (M + H)⁺.
b) 5-[4-(2-Amino-4-chlorobenzyl)-7-bromo-3-(4-chlorophenyl)-2,5-dioxo-1,4-benzodiazepin-1-yl]valeric acid sodium salt
   The title compound was prepared (46%) from 5-[7-bromo-3-(4-chlorophenyl)-4-[(4-chloro-2-nitrophenyl)methyl]-2,5-dioxo-1,4-benzodiazepin-1-yl]valeric acid *tert*-butyl ester following successively the procedure described for example 60, step e, f: ¹H NMR (400 MHz, CDCl₃) δ 1.62-1.78 (m, 4H, 2 CH₂), 2.36-2.40 (m, 2H, CH₂), 3.66-3.73 (m, 1H, CH), 4.24-4.31 (m, 1H, CH), 4.37 (d, J = 14.8 Hz, 1H, CH), 5.39 (s, 1H, CH), 5.42 (d, J = 14.8 Hz, 1H, CH), 6.60 (dd, *J=* 8.4 Hz, *J=* 2.4 Hz, 1H, ArH), 6.68-6.71 (m, 3H, ArH), 6.80 (d, J = 8.8 Hz, 1H, ArH), 6.97 (d, *J* = 8.4 Hz, 2H, ArH), 7.07 (d, *J* = 8.4 Hz, 1H, ArH), 7.34 (dd, *J =* 8.8 Hz, J = 2.4 Hz, 1H, ArH), 7.74 (d, *J =* 2.4 Hz, 1H, ArH). Mass spectrum (LCMS, ESI pos.): Calcd for C₂₇H₂₄BrCl₂N₃O₄: 603.0; found: 604.0 (M + H)⁺.

### EXAMPLE 62

4-(4-Chloro-benzyl)-3-(4-chloro-phenyl)-7-iodo-3,4-dihydro-1 H-benzo[e][1,4]diazepine-2,5-dione 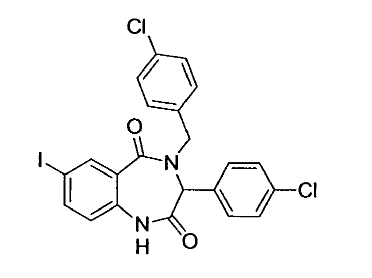 ¹H NMR (400 MHz, CDCl₃): δ 8.09 (s, 1H), 7.87 (s, 1H), 7.52 (d, J=8.3 Hz, 1H), 7.44-7.37 (br m, 2H), 7.36-7.30 (m, 2H), 7.10-7.04 (m, 2H), 6.87-6.77 (br m, 2H), 6.43 (d, J= 8.3 Hz, 1H), 5.33 (s, 1H), 5.03-4.91 (m, 2H). Mass Spectrum (LCMS, ESI, pos.): calcd. for C₂₂H₁₅Cl₂IN₂O₂: 535.9; found 537.03 (M+H).

### EXAMPLE 63

4-(R)-[1-(2-Amino-4-chloro-5-fluoro-phenyl)-ethyl]-3 (S)-(4-chloro-phenyl)-7-iodo-1-(2-morpholin-4-yl-ethyl)-3,4-dihydro-1H-benzo[e][1,4]diazepine-2,5-dione 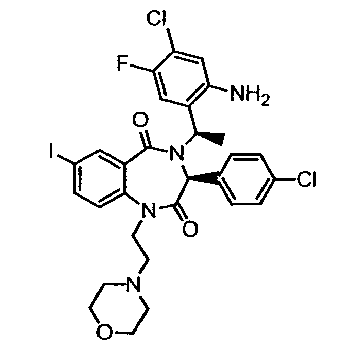
a) 1-(4-Chloro-3-fluoro-phenyl)-ethanol
   4-Chloro-3-fluoro-benzaldehyde (5.14 g, 32.4 mmol) was dissolved in THF (100 mL) and place at -78°C under nitrogen atmosphere. Methyl Lithium (1.6 M in diethyl ether, 22.3 mL) was added to the reaction during a period of 3 minutes maintaining the same temperature. After the addition, the reaction was stirred at room temperature for 1 h. Then, the reaction was poured over ice water and extracted with ethyl acetate and hydrochloric acid IN. The organic layer was collected, dried with sodium sulfate, filtered and concentrated under vacuum to produce a crude, which it was chromatograph over silica gel using 50% hexane- ethyl Acetate as solvent to yield 5.1 g of 1-(4-chloro-3-fluorophenyl)-ethanol. ¹H NMR (400 MHz, Cl₃CD) δ 7.37 (t, *J*= 8.14 Hz, 1H), 7.20 *(dd, J =* 1.86, 10.00 Hz, 1H), 7.09 *(dd, J =* 1.86, 8.14 Hz, 1H), 4.89 (m, 1H), 1.93 (br s, 1H), 1.49 (d, J = 6.51, 3 H).
b) 2-[1-(4-Chloro-3-fluoro-phenyl)-ethyl]-isoindole-1,3-dione
   1-(4-Chloro-3-fluoro-phenyl)-ethanol (5.1 g, 29.2 mmol), phthalimide (4.3 g, 29.2 mmol) and triphenylphosphine (11.49 g, 43.8 mmol) were dissolved in THF (100 ml) and place at -78°C under nitrogen atmosphere. Then, diisopropyl azodicarboxylate (8.86 g, 43.8 mmol) was added in one shot. The reaction was place at room temperature and stirred for 30 minutes. The solvent was evaporated and the crude was chromatograph over silica gel using 70% hexane- ethyl acetate as solvent to yield 4.08 g of 2-[1-(4-Chloro-3-fluorophenyl)-ethyl]-isoindole-1,3-dione. ¹H NMR (400 MHz, C13CD) δ 7.83 (m, 2H), 7.73 (m, 2H), 7.34 (m, 2H), 7.21 (dd, *J* = 1.93, 8.14 Hz, 1H), 5.53 (m, 1H), 1.91(d, *J* = 7.29, 3H).
c) 1-(4-Chloro-3-fluoro-phenyl)-ethylamine
   2-[1-(4-Chloro-3-fluoro-phenyl)-ethyl]-isoindole-1,3-dione (4.08 g, 13.4 mmol) was dissolved in THF (100 mL). Hydrazine (4 mL) was added. The reaction was stirred at 80°C for 1 h. Then, the solvent was evaporated and the crude was chromatograph over silica gel using ethyl acetate as solvent to yield 2.0 g of 1-(4-chloro-3-fluoro-phenyl)-ethylamine. ¹H NMR (400 MHz, Cl3CD) δ 7.34 (t, *J =* 7.97 Hz, 1H), 7.18 (dd, *J =* 1.90, 10.25 Hz, 1H), 7.08 (dd, *J* = 1.90, 7.97 Hz, 1H), 4.12 (m, 1H), 1.36(d, *J*=6.64,3H).
d) N-[ 1-(4-Chloro-3-fluoro-phenyl)-ethyl]-2,2,2-trifluoro-acetamide
   1-(4-Chloro-3-fluoro-phenyl)-ethylamine (2.0 g, 11.5 mmol) was dissolved in pyridine (20 ml) and place at 0°C under nitrogen atmosphere. Then, trifluoroacetic anhydride (3.63 g, 17.2 mmol) was added in one shot. The reaction was allowed to reach room temperature and stirred for 16h. The solvent was removed under vacuum and the crude was extracted with ethyl acetate- sodium hydroxide IN. The organic layer was collected and extracted again using hydrochloric acid (1N) and brine. The organic layer was collected, dried with sodium sulfate, filtered and concentrated under vacuum to produce a crude, which it was chromatograph over silica gel using 50% hexane- ethyl acetate as solvent to yield 1.374 g of N-[1-(4-chloro-3-fluoro-phenyl)-ethyl]-2,2,2-trifluoro-acetamide. ¹H NMR (400 MHz, Cl3CD) δ 7.49 (d, J= 7.29 Hz, 1H), 7.35 (t, J = 7.92 Hz, 1H), 7.11 (dd, J = 1.93, 9.86 Hz, 1H), 7.04 (dd, *J* = 1.93, 7.29 Hz, 1H), 5.06 (m, 1H), 1.53 (d, J = 7.07, 3H).
e) N-[1-(4-Chloro-3-fluoro-2-nitro-phenyl)-ethyl]-2,2,2-trifluoro-acetamide
   N-[1-(4-Chloro-3-fluoro-phenyl)-ethyl]-2,2,2-trifluoro-acetamide (1.374 g, 5.09 mmol) was dissolved at room temperature in concentrated sulfuric acid (25 mL). The solution was place at 0°C using an ice water bath, and potassium nitrate (0.567 g, 5.60 mmol) was added. The reaction was stirred at the same temperature for 15 minutes and then allowed to reach room temperature and stirred for 16 h. Then, the reaction was poured over ice water and extracted with ethyl Acetate-water. The organic layer was collected and extracted again using sodium hydroxide (1N) and brine. The organic layer was collected, dried with sodium sulfate, filtered and concentrated under vacuum to produce a crude, which it was chromatograph over silica gel using 80% hexane- ethyl acetate as solvent to yield 1.13 g of N-[1-(4-chloro-3-fluoro-2-nitro-phenyl)-ethyl]-2,2,2-trifluoro-acetamide.
   ¹H NMR (400 MHz, Cl3CD) δ 8.16 (d, J = 6.43 Hz, 1H), 7.30 (d, J = 9.00 Hz, 1H), 7.08 (br s, 1H), 5.52 (m, 1H), 1.65 (d, J = 7.07, 3H).
f) 1-(4-Chloro-3-fluoro-2-nitro-phenyl)-ethylamine
   N-[1-(4-Chloro-3-fluoro-2-nitro-phenyl)-ethyl]-2,2,2-trifluoro-acetamide (286 mg, 0.909 mmol) was dissolved at room temperature in THF (10 mL). Then, a solution of lithium hydroxide monohydrate (46 mg, 1.09 mmol) in water (2 mL) was added. The reaction was followed by TLC, using ethyl acetate as solvent. After 2 h., more lithium hydroxide monohydrate (46 mg, 1.09 mmol) in water (2 mL) was added. The reaction was stirred at room temperature for three days. Then, the reaction was extracted with ethyl acetate-sodium hydroxide (1N) and brine. The organic layer was collected, dried with sodium sulfate, filtered and concentrated under vacuum to produce a crude, which it was chromatograph over silica gel to yield 148 mg of 1-(4-chloro-3-fluoro-2-nitro-phenyl)-ethylamine. ¹H NMR (400 MHz, Cl3CD) δ 7.90 (d, *J=* 6.43 Hz, 1H), 7.64 (d, J = 10.07 Hz, 1H), 4.62 (m, 1H), 1.58 (br s, 2H), 1.33 (d, *J* = 6.43, 3H).
g) 4(R,S)-[1-(2-Amino-4-chloro-5-fluoro-phenyl)-ethyl]-3(S,R)-(4-chlorophenyl)-7-iodo-1-(2-morpholin-4-yl-ethyl)-3,4-dihydro-1H-benzo[e][1,4]diazepine-2,5-dione
   The title compound was synthesized following the general procedure for the synthesis of diazepines. ¹H NMR (400 MHz, Cl3CD) δ 7.79 (d, *J* = 1.93 Hz, 1H), 7.57 (dd, J = 1.93, 6.64 Hz, 1H), 7.32 (d, J = 10.23 Hz, 1H), 7.03 (d, J = 6.64 Hz, 1H), 6.85 (m, 3H), 6.57 (d, J = 8.36 Hz, 2H), 6.10 (m, 1H), 5.54 (s, 1H), 4.42 (m, 1H), 4.23 (m, 1H), 3.97 (br s, 2H), 3.74 (br s, 2H), 3.46 (m, 6H), 1.67 (d, J = 7.07 Hz, 3H). Mass spectrum (LCMS, ESI pos.): Calcd for C₂₉H₂₈Cl₂FIN₄O₃: 696.06; found 696.9 (M+H).

### EXAMPLE 64

4(R,S)-[1-(2-Amino-4-chloro-5-hydroxy-phenyl)-ethyl]-3(S,R)-(4-chlorophenyl)-7-iodo-1-(2-morpholin-4-yl-ethyl)-3,4-dihydro-1H-benzo[e] [1,4]diazepine-2,5-dione

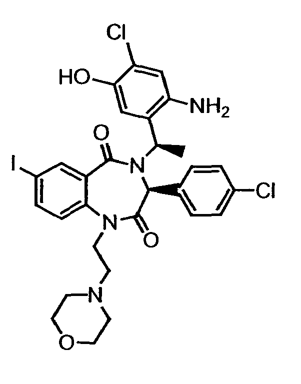

This compound was obtained as bi-product from Example 63 (step g). ¹H NMR (400 MHz, C13CD) δ 7.82 (d, J = 1.93 Hz, 1H), 7.59 (dd, J = 1.93, 8.79 Hz, 1H), 7.31 (s, 1H), 7.02 (s, 1H), 6.88 (m, 3H), 7.37 (d, J = 8.57 Hz, 2H), 6.05 (m, 1H), 5.76 (s, 1H), 4.41 (m, 1H), 4.24 (m, 1H), 3.97 (br s, 2H), 3.75 (br s, 2H), 3.47 (m, 6H), 1.75 (d, J = 6.86 Hz, 3H). Mass spectrum (LCMS, ESI pos.): Calcd for C₂₉H₂₉Cl₂IN₄O₄: 694.06; found 694.9 (M+H).

### EXAMPLE 65

3(S)-(4-Chloro-2-hydroxy-phenyl)-4(R)-[1-(4-chloro-phenyl)-ethyl]-7-iodo-3,4-dihydro-1H-benzo[e][1,4]diazepine-2,5-dione 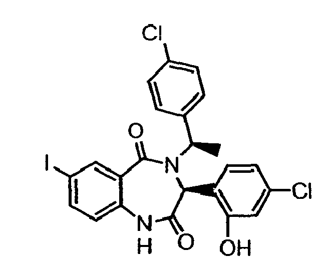 ¹H NMR (400 MHz, Cl3CD) δ 8.97 (s, 1H), 8.03 (d, *J* = 1.93 Hz, 1H), 7.66 (br s, 1H), 7.34 (d, *J* = 8.57 Hz, 2H), 7.21 (dd, *J* = 1.93, 8.36 Hz, 1H), 7.16 (d, *J* = 8.36 Hz, 2H), 6.42 (m, 2H), 6.31 (m, 2H), 6.22 (d, *J* = 8.57 Hz, 1H), 5.13 (s, 1H), 1.88 (br s, 1H), 1.62 (d, *J =* 7.07 Hz, 3H). Mass spectrum (LCMS, ESI pos.): Calcd for C₂₃H₁₇Cl₂IN₂O₃: 565.9; found 566.4 (M+H).

### EXAMPLE 66

4-[1-(2-Amino-4-chloro-phenyl)-ethyl]-3-(4-chloro-phenyl)-7-iodo-1-[3-(4-methyl-piperazin-1-yl)-propyl]-3,4-dihydro-1H-benzo [e] [1,4]diazepine-2,5-dione

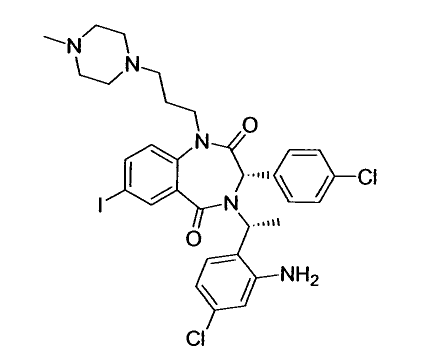

¹H NMR (400 MHz, CD₃OD) δ 7.91 (s, 1H), 7.72 (m, 1H), 7.67 (d, 1H, J= 6.4Hz), 7.51 (m, 1H), 7.34 (m, 1H), 6.98 (m, 3H), 6.45 (m, 2H), 6.23 (q, 1H, J = 6.4 Hz), 5.98 (m, 1H), 4.28 (m, 1H), 4.05-3.56 (bm, 10H), 3.40 (bs, 1H), 3.05 (s, 3H), 2.36-2.18 (bm, 2H), 1.89 (d, 3H, J = 6.8 Hz). Mass spectrum (LCMS, ESI pos.): Calcd for C₃₁H₃₄Cl₂IN₅O₂: 705.1; found 705.9 (M+H).

### EXAMPLE 67

4-(2-Amino-4-chloro-benzyl)-3-(4-chloro-phenyl)-1-(4-dimethylamino-butyl)-7-iodo-3,4-dihydro-1H-benzo[e][1,4]diazepine-2,5-dione 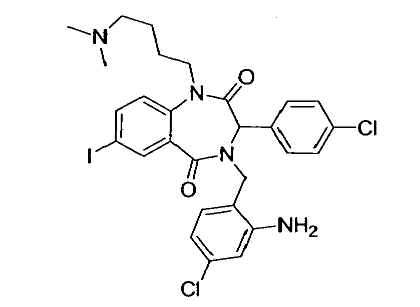 ¹H NMR (400 MHz, CD₃OD) δ 7.85 (d, 1H, *J* = 2.0 Hz), 7.48 (dd, 1H, *J* = 2.0, 8.8 Hz), 7.21 (d, 1H, *J* = 8.4 Hz), 7.04 (d, 2H, *J* = 8.4 Hz), 6.94 (d, 1H, *J =* 8.8 Hz), 6.81 (m, 3H), 6.62 (dd, 1H, *J =* 2.0, 8.8 Hz), 5.53 (s, 1H), 5.23 (d, 1H, *J* = 14.4 Hz), 4.63 (d, 1H, *J* = 14.4 Hz), 4.40 (m, 1H), 3.77 (m, 1H), 3.07 (m, 2H), 2.85 (d, 6H, *J* = 5.2 Hz), 1.71-1.52 (m, 4H). Mass spectrum (LCMS, ESI pos.): Calcd for C₂₈H₂₉Cl₂IN₄O₂: 650.07; found 651.0 (M+H).

### EXAMPLE 68

4-(2-Amino-4-chloro-benzyl)-3-(4-chloro-phenyl)-7-iodo-1-(4-morpholin-4-yl-butyl)-3,4-dihydro-1H-benzo[e][1,4]diazepine-2,5-dione 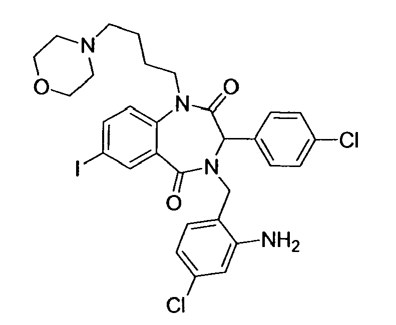 ¹H NMR (400 MHz, CD₃OD) δ 7.86 (d, 1H, *J =* 2.0 Hz), 7.65 (dd, 1H, *J =* 2.0, 8.8 Hz), 7.21 (d, 1H, *J*= 8.4 Hz), 7.04 (d, 2H, *J =* 8.8 Hz), 6.94 (d, H, *J =* 8.8 Hz), 6.80 (m, 3H), 6.60 (dd, 1H, *J =* 2.0, 8.8 Hz), 5.54 (s, 1H), 5.25 (d, 1H, *J =* 14.4 Hz), 4.62 (d, 1H, *J =* 14.4 Hz), 4.40 (m, 1H), 4.09 (m, 2H), 3.75 (m, 3H), 3.41 (m, 2H), 3.09 (m, 4H), 1.60 (m, 4H). Mass spectrum (LCMS, ESI pos.): Calcd for C₃₀H₃₁Cl₂IN₄O₃: 692.08; found 693.0 (M+H).

### EXAMPLE 69

4-(2-Amino-4-chloro-benzyl)-3-(4-chloro-phenyl)-7-iodo-1-[4-(4-methyl-piperazin-1-yl)-butyl]-3,4-dihydro-1H-benzo[e][1,4]diazepine-2,5-dione 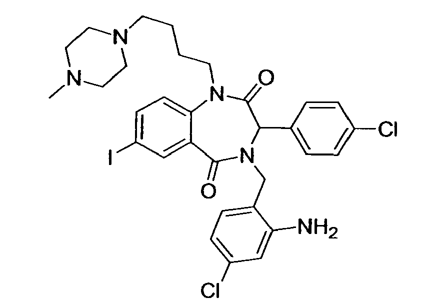 ¹H NMR (400 MHz, CD₃OD) δ 7.84 (d, 1H, J = 2.4 Hz), 7.64 (dd, 1H, *J =* 2.0, 8.4 Hz), 7.21 (d, 1H, *J =* 8.0 Hz), 7.04 (d, 2H, J = 8.4 Hz), 6.94 (d, H, *J =* 8.8 Hz), 6.80 (m, 3H), 6.63 (dd, 1H, *J =* 2.4, 8.4 Hz), 5.53 (s, 1H), 5.27 (d, 1H, J = 14.4 Hz), 4.61 (d, 1H, J = 14.4 Hz), 4.39 (m, 1H), 3.78 (m, 1H), 3.40 (m, 4H), 3.20 (m, 4H), 2.91 (s, 3H), 2.88 (m, 2H), 1.70-1.52 (m, 4H). Mass spectrum (LCMS, ESI pos.): Calcd for C₃₁H₃₄Cl₂IN₅O₂: 705.1; found 706.2 (M+H).

### EXAMPLE 70

4-(2-Amino-4-chloro-benzyl)-3-(4-chloro-phenyl)-1-(3-dimethylaminopropyl)-7-iodo-3,4-dihydro-1H-benzo[e][1,4]diazepine-2,5-dione 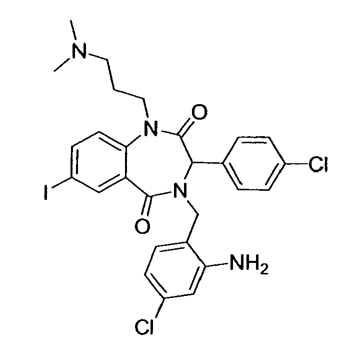 ¹H NMR (400 MHz, CD₃OD) δ 7.88 (d, 1H, *J =* 2.4 Hz), 7.67 (dd, 1H, *J =* 2.0, 8.4 Hz), 7.23 (d, 1H, *J =* 8.0 Hz), 7.07 (d, 2H, *J =* 8.4 Hz), 6.93 (d, 1H, *J =* 8.8 Hz), 6.85 (d, 2H, *J =* 8.8 Hz), 6.78 (d, 1H, *J =* 2.0 Hz), 6.62 (dd, 1H, *J =* 2.4, 8.0 Hz), 5.57 (s, 1H), 5.06 (d, 1H, *J =* 14.4 Hz), 4.85 (d, 1H, *J* = 14.4 Hz), 4.36 (m, 1H), 3.83 (m, 1H), 3.04 (m, 2H), 2.85 (d, 6H, *J* = 3.2 Hz), 2.04-1.90 (m, 2H). Mass spectrum (LCMS, ESI pos.): Calcd for C₂₇H₂₇Cl₂IN₄O₂: 636.06; found 636.9 (M+H).

### EXAMPLE 71

4-(2-Amino-4-chloro-benzyl)-3-(4-chloro-phenyl)-7-iodo-1-(3-morpholin-4-yl-propyl)-3,4-dihydro-1H-benzo[e][1,4]diazepine-2,5-dione 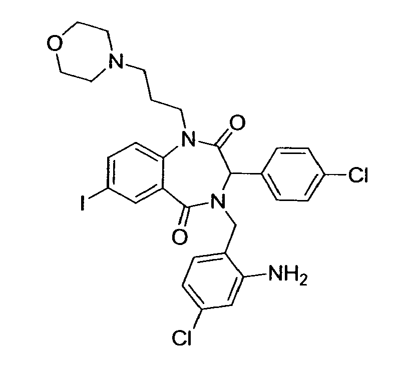 ¹H NMR (400 MHz, CD₃OD) δ 7.88 (d, 1H, *J* = 2.4 Hz), 7.66 (dd, 1H, *J* = 2.0, 8.4 Hz), 7.24 (d, 1H, *J* = 8.0 Hz), 7.08 (d, 2H, *J* = 8.4 Hz), 6.95 (d, 1H, *J =* 8.8 Hz), 6.86 (d, 2H, *J =* 8.4 Hz), 6.79 (d, 1H, J = 2.4 Hz), 6.63 (dd, 1H, J = 2.4, 8.4 Hz), 5.58 (s, 1H), 5.04 (d, 1H, J = 14.8 Hz), 4.84 (d, 1H, *J =* 14.8 Hz), 4.39 (m, 1H), 4.09 (m, 2H), 3.82 (m, 1H), 3.74 (m, 2H), 3.42 (m, 2H), 3.08 (m, 4H), 2.04-1.95 (m, 2H). Mass spectrum (LCMS, ESI pos.): Calcd for C₂₉H₂₉Cl₂IN₄O₃: 678.07; found 679.0 (M+H).

### EXAMPLE 72

4-(4-Chloro-2-hydroxybenzyl)-3-(4-chlorophenyl)-7-iodo-1-[2-(2-methoxyethoxy)ethyl]-3,4-dihydro-1*H*-benzo[e][1,4]diazepine-2,5-dione 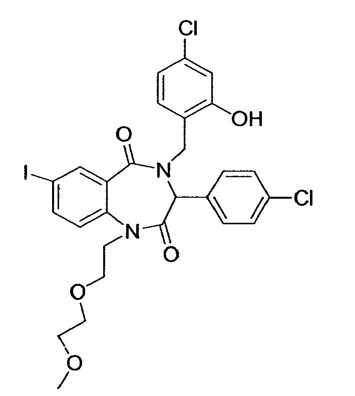 ¹H NMR (400 MHz, CDCl₃) δ 7.95 (d, J = 2.1 Hz, 1H), 7.56 (dd, J = 2.2 Hz, 8.6 Hz, 1H), 7.13 (t, J = 8.0 Hz, 2H), 7.07 (d, J = 2.1 Hz, 1H), 6.98 (d, J = 8.6 Hz, 2H), 6.82 (dd, *J =* 2.1 Hz, 7.9 Hz, 1H), 6.59 (dd, *J =* 1.0 Hz, 8.8 Hz, 2H), 5.51 (s, 1H), 5.41 (d, J = 15.0 Hz, 1H), 5.33 (s, 1H), 4.41 (d, 15.0 Hz, 1H), 4.05-4.11 (m, 2H), 3.92-3.99 (m, 1H), 3.76-3.69 (m, 1H), 3.64-3.56 (m, 2H), 3.54-3.48 (m, 2H), 3.37 (s, 3H). Mass spectrum (LCMS, ESI pos.): Calcd for C₂₇H₂₅C₁₂IN₂O₅: 654.02; found 654.8(M+H).

### EXAMPLE 73

4-[(*S*)-1-(2-Amino-4-chlorophenyl)ethyl]-(3*R*)-3-(4-chlorophenyl)-7-iodo-1-(2-morpholin-4-ylethyl)-3,4-dihydro-1*H*-benzo[e][1,4]diazepine-2,5-dione 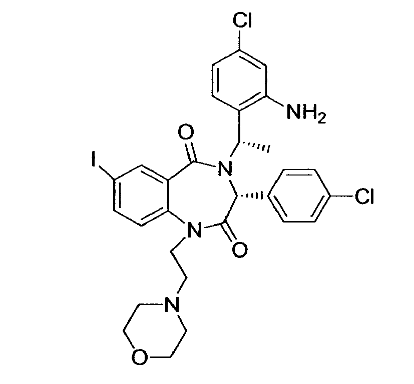 ¹H NMR (400 MHz, CDCl₃) δ 7.86 (d, *J* = 2.1 Hz, 1H), 7.58 (dd, *J* = 2.1 Hz, 8.5 Hz, 1H), 7.28 (d, *J=* 8.1 Hz, 1H), 6.92 (m, 3H), 6.76 (m, 3H), 6.57 (dd, *J* = 2.1 Hz, 8.4 Hz, 1H), 6.27 (q, *J* = 7.3 Hz, 1H), 5.31 (s, 1H), 4.63-4.55 (m, 1H), 3.82-3.73 (m, 1H), 3.60-3.54 (m, 2H), 3.53-3.45 (m, 2H), 2.69-2.61 (m, 1H), 2.46-2.34 (m, 3H), 2.27-2.19 (m, 2H), 1.75 (s, 3H). Mass spectrum (LCMS, ESI pos.): Calcd for C₂₉H₂₉Cl₂IN₄O₃: 678.07; found 678.9(M+H).

### EXAMPLE 74

4-(2-Amino-4-chlorobenzyl)-3-(4-chlorophenyl)-7-iodo-1-[3-(4-methyl-piperazin-1-yl)propyl]-3,4-dihydro-1*H*-benzo[e][1,4]diazepine-2,5-dione 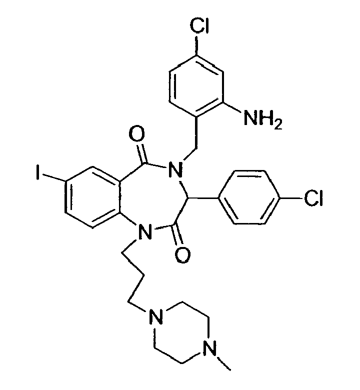 ¹H NMR (400 MHz, CD₃OD) δ 7.83 (d, *J =* 2.1 Hz, 1H), 7.63 (dd, *J =* 2.1 Hz, 8.6 Hz, 1H), 7.20 (d, *J =* 7.9 Hz, 1H), 7.07-7.02 (m, 2H), 6.95 (d, *J =* 8.8 Hz, 1H), 6.85-6.80 (m, 2H), 6.77 (d, *J* = 2.1 Hz, 1H), 6.57 (dd, *J* = 2.1 Hz, 7.9 Hz 1H), 5.50 (s, 1H), 5.13 (d, *J =* 14.4 Hz , 1H), 4.73 (d, *J* = 14.6 Hz 1H), 4.44-4.32 (m, 1H), 3.81-3.70 (m, 1H), 3.37 (s, 2H), 2.10-2.72 (m, 11H), 1.63-1.85 (m, 2H). Mass spectrum (LCMS, ESI pos.): Calcd for C₃₀H₃₂Cl₂IN₅O₂: 691.1; found 692.0 (M+H).

### EXAMPLE 75

5-(2-Allyloxy-4-chlorobenzyloxy)-3-(4-chlorophenyl)-7-iodo-1-[2-(2-methoxyethoxy)ethyl]-1,3-dihydrobenzo[e][1,4]diazepin-2-one 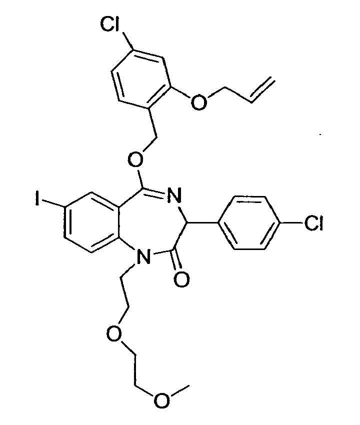 ¹H NMR (400 MHz, CDCl₃) δ 8.01 (d, *J* = 2.1 Hz, 1H), 7.86 (dd, *J* = 2.1 Hz, 8.5 Hz, 1H), 7.58-7.47 (m, 3H), 7.42-7.330 (m, 3H), 6.96 (dd, *J* = 1.9 Hz, 8.1 Hz, 1H), 6.92 (d, *J* = 1.9 Hz, 1H), 6.13-6.02 (m, 1H), 5.51-5.31 (m, 4H), 4.67 (s, 1H), 4.63 (m, 2H), 4.12-4.02 (m, 1H), 3.97-3.89 (m, 1H), 3.87-3.79 (m, 1H), 3.66-3.59 (m, 1H), 3.58-3.54 (m, 2H), 3.51-3.45 (m, 2H), 3.36 (s, 3H). Mass spectrum (LCMS, ESI pos.): Calcd for C₃₀H₂₉Cl₂IN₂O₅ : 694.05; found 694.9 (M+H).

### EXAMPLE 76

4-(2-Amino-4-chlorobenzyl)-3-(4-chlorophenyl)-7-iodo-3,4-dihydro-1*H-*benzo[e] [1,4]diazepine-2,5-dione 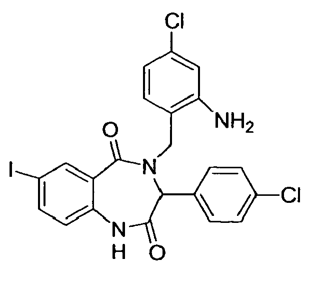 ¹H NMR (400 MHz, CDCl₃) δ 8.74 (s, 1H), 8.04 (s, 1H), 7.56 (dd, J = 1.9 Hz, 8.3 Hz, 1H), 7.12-7.69 (m, 3H), 6.91-6.81 (m, 2H), 6.71 (d, J = 1.9 Hz, 1H), (dd, *J =* 1.9 Hz, 7.9 Hz, 1H), 6.52 (d, *J =* 8.3 Hz, 1H), 5.5 (d, *J* = 14.5 Hz, 1H), 4.93 (s, 2H), 4.34 (d, *J* = 15.0 Hz, 1H).

### EXAMPLE 77

4-[1-(3-Amino-4-chlorophenyl)ethyl]-3-(4-chlorophenyl)-7-iodo-3,4-dihydro-1*H*-benzo[e][1,4]diazepine-2,5-dione 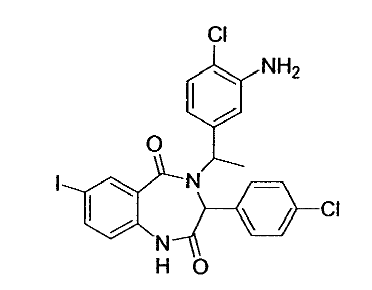 ¹H NMR (400 MHz, CD₃OD) δ 8.06 (d, J = 2.1 Hz, 1H), 7.85 (s, 1H), 7.49 (dd, *J =* 2.1 Hz, 8.3 Hz, 1H), 7.22 (d, *J =* 8.1 Hz, 1H), 7.02 (s, 1H), 6.97 (d, J = 8.5 Hz, 2H), 6.91 (dd, *J =* 1.9 Hz, 8.3 Hz, 1 H), 6.66 (m, 2H), 6.43 (d, *J =* 8.3 Hz, 1H), 6.40-6.36 (m, 1H), 5.31 (s, 1H), 1.7 (d, J = 7.3 Hz, 3H). Mass spectrum (LCMS, ESI pos.): Calcd for C₂₃H₁₈Cl₂IN₃O₂: 564.98; found 565.5 (M+H).

### EXAMPLE 78

4-Benzyl-7-bromo-3-(4-chlorophenyl)-1-methyl-1,4-benzodiazepine-2,5-dione 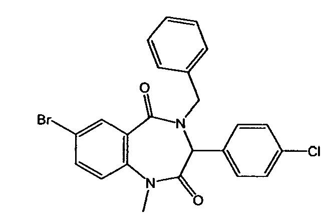 ¹H NMR (400 MHz, CDCl₃) δ 3.43 (s, 3H), 4.65 (d, *J=* 14.4 Hz, 1H), 5.38 (d, *J =* 14.4 Hz, 1H), 5.47 (s, 1H), 6.60 (dd, *J =* 8.4 Hz, 1.2 Hz, 2H), 6.75 (d, J = 8.8 Hz, 1H), 6.96 (d, J = 8.8 Hz, 2H), 7.28-7.41 (m, 4H), 7.48-7.51 (m, 2H), 7.80 (d, J = 2.4 Hz, 1H). Mass spectrum (LCMS, ESI pos.): Calcd for C₂₃H₁₈BrClN₂O₂: 468; found: 469 (M + H)⁺.

### EXAMPLE 79

7-Bromo-3-(4-chlorophenyl)-1-methyl-4-(1-phenethyl)-1,4-benzodiazepine-2,5-dione 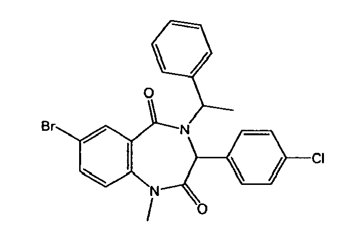 Mass spectrum (LCMS, ESI pos.): Calcd for C₂₄H₂₀BrClN₂O₂: 482; found: 483 (M + H)⁺.

### EXAMPLE 80

1,3-Dihydro-4-[1-(2-amino-4-chlorophenyl)ethyl]-3-(4-chlorophenyl)-7-iodo-1,4-benzodiazepine-2,5-dione 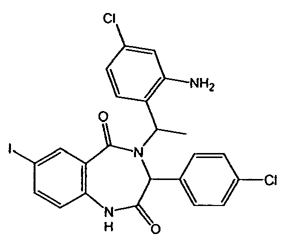 ¹H NMR (400 MHz, DMSO-d₆) δ 1.61 (d, J = 6.8 Hz, 3H), 5.00 (s, 1H), 5.78 (s, 2H), 6.09 (q, J = 6.8 Hz, 1H), 6.51-6.54 (m, 1H), 6.67 (d, J = 8.4 Hz, 1H), 6.77-6.81 (m, 3H), 7.08 (d, J = 8.8 Hz, 2 H), 7.26 (d, J = 8.0 Hz, 1H), 7.59 (dd, J = 8.8 Hz, J = 2.0 Hz, 1H), 7.79 (d, J = 1.6 Hz, 1H), 10.98 (br s, 1H). Mass spectrum (LCMS, ESI pos.): Calcd for C₂₃H₁₈Cl₂IN₃O₂: 565; found: 566 (M + H)⁺.

### EXAMPLE 81

3-(4-Chlorophenyl)-4-[1-(4-chloro-2-nitrophenyl)ethyl]-7-iodo-1-[2-(4-morpholino)ethyl]-1,4-benzodiazepine-2,5-dione 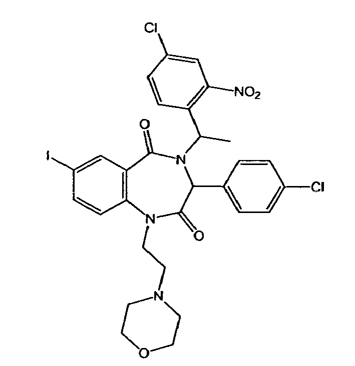 ¹H NMR (400 MHz, CDCl₃) δ 1.81 (d, *J =* 6.8 Hz, 3H), 2.23-2.28 (m, 2H), 2.37-2.46 (m, 3H), 2.59-2.66 (m, 1H), 3.54-3.63 (m, 5H), 4.44-4.51 (m, 1H), 5.42 (s, 1H), 6.57-6.70 (m, 4H), 6.94 (d, *J* = 8.4 Hz, 2H), 7.44 (dd, *J* = 8.8 Hz, 2.0 Hz, 1H), 7.55 (dd, *J =* 8.4 Hz, 2.0 Hz, 1H), 7.63 (d, *J =* 8.0 Hz, 1H), 7.86 (d, *J* = 2.0 Hz, 1H), 7.96 (d, *J* = 2.0 Hz, 1H). Mass spectrum (LCMS, ESI pos.): Calcd for C₂₉H₂₇Cl₂IN₄O₅: 708; found: 709 (M + H)⁺.

### EXAMPLE 82

5-[(3,*S*)-3-(4-Chlorophenyl)-4-[(*R*)-1-(4-chlorophenyl)ethyl]-2,5-dioxo-7-(propyn-1-yl)-1,4-benzodiazepin-1-yl] valeric acid sodium salt 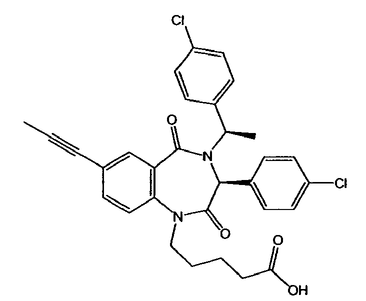
a) (3S)-3-(4-Chlorophenyl)-4-[(*R*)-1-(4-chlorophenyl)ethyl]-7-(propyn-1-yl)-1,4-benzodiazepine-2,5-dione
   Propyne (2 mL) was condensed in a sealed tube at -78 °C. Then, (3*S*)-3-(4-Chlorophenyl)-4-[(*R*)-1-(4-chlorophenyl)ethyl]-7iodo-1,4-benzodiazepine-2,5-dione (200 mg, 0.362 mmol), copper iodide (15 mg, 0.0788 mmol), dichlorobis(triphenylphosphine)palladium (II) (39mg, 0.0556 mmol), triethylamine (2 mL), and acetonitrile (4 mL) were added. The sealed tube was closed and the reaction mixture was allowed warm up to room temperature. After 12 h, the reaction mixture was cooled to -78 °C and the sealed tube was opened and allowed to warm up to room temperature under vigorous stirring. Then, solvents were evaporated in vacuo and the residue was purified by column chromatography on silica (EtOAc/Hexanes, 1:1) to give the title compound as colorless solid (150 mg, 89%): ¹H NMR (400 MHz, CDCl₃) δ 1.73 (d, *J* = 7.1 Hz, 3H), 2.06 (s, 3H), 5.24 (s, 1H), 6.49 (q, *J* = 7.1 Hz, 1H), 6.62-6.65 (m, 3H), 6.93 (d, *J* = 8.4 Hz, 2H), 7.20 (dd, *J =* 8.0 Hz, *J =* 2.0 Hz, 1H), 7.33 (d, *J* = 8.4 Hz, 2H), 7.53 (d, *J* = 8.8 Hz, 2H), 7.79 (d, *J* = 2.0 Hz, 1H), 8.57 (br s, 1H). Mass spectrum (LCMS, ESI pos.): Calcd for C₂₆H₂₀Cl₂N₂O₂: 463; found: 464 (M + H)⁺.
b) 5-[(3*S*)-3-(4-Chlorophenyl)-4-[(*R*)-1-(4-chlorophenyl)ethyl]-2,5-dioxo-7-(propyn-1-yl)-1,4-benzodiazepin-1-yl]valeric acid sodium salt
   The title compound was prepared (58%) from (3S)-3-(4-chlorophenyl)-4-[(R)-1-(4-chlorophenyl)ethyl]-7-(propyn-1-yl)-1,4-benzodiazepine-2,5-dione following successively the general procedure for the alkylation of diazepines at position 1, followed by example 1, step b. ¹H NMR (400 MHz, CDCl₃) δ 1.60-1.74 (m, 7H), 2.02 (s, 3H), 2.37 (t, J = 6.8 Hz, 2H), 3.64-3.71 (m, 1H), 4.27-4.34 (m, 1H), 5.31 (s, 1H), 6.42 (q, *J =* 6.8 Hz, 1H), 6.46-6.49 (m, 2H), 6.76 (d, J = 8.4 Hz, 1H), 6.87 (d, J = 8.4 Hz, 2H), 7.16 (dd, J = 8.4 Hz, 2.0 Hz, 1H), 7.29 (d, J = 8.4 Hz, 2H), 7.48 (d, J = 8.8 Hz, 2H), 7.67 (d, J = 2.0 Hz, 1H), 7.90 (br s, 1H). Mass spectrum (LCMS, ESI pos.): Calcd for C₃₁H₂₈Cl₂N₂O₄: 562; found: 563 (M + H)⁺.

### EXAMPLE 83

4-[1-(2-Amino-4-chlorophenyl)ethyl]-3-(4-chlorophenyl)-7-iodo-1-[2-(2-methoxyethoxy)ethyl]-1,4-benzodiazepine-2,5-dione 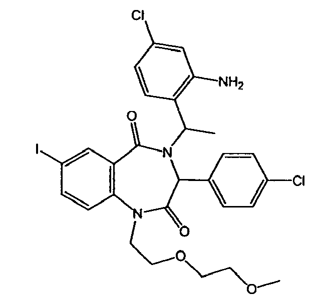 ¹H NMR (400 MHz, CDCl₃) δ 1.75 (d, J = 6.8 Hz, 3H), 3.36 (s, 3H), 3.54-3.64 (m, 4H), 3.71-3.76 (m, 1H), 3.85-3.90 (m, 1H), 3.97-4.03 (m, 1H), 4.17-4.24 (m, 1H), 4.77 (br s, 2H), 5.27 (s, 1H), 6.27 (q, J = 6.8 Hz, 1H), 6.62 (dd, *J =* 8.4 Hz, 2.4 Hz, 1H), 6.66-6.70 (m, 3H), 6.90 (d, J = 8.4 Hz, 2H), 6.97 (d, *J* = 8.8 Hz, 1H), 7.21 (d, J = 8.4 Hz, 1H), 7.48 (dd, J = 8.4 Hz, 2.0 Hz, 1H), 7.90 (d, J = 2.0 Hz, 1H). Mass spectrum (LCMS, ESI pos.): Calcd for C₂₈H₂₈Cl₂IN₃O₄: 667; found: 668 (M + H)⁺_{.}

### EXAMPLE 84

4-[(R)-1-(2-amino-4-chlorophenyl)ethyl]-(3S)-3-(4-chlorophenyl)-7-iodo-1-[2-(2-methoxyethoxy)ethyl]-1,4-benzodiazepine-2,5-dione 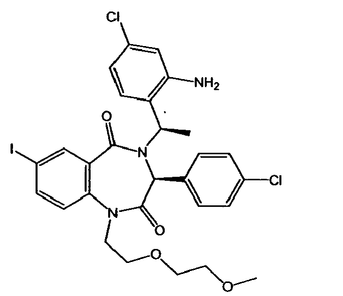 Mass spectrum (LCMS, ESI pos.): Calcd for C₂₈H₂₈Cl₂IN₃O₄: 667; found: 668 (M + H)⁺.

### EXAMPLE 85

(3R)-4-[(S)-1-(2-amino-4-chlorophenyl)ethyl]-3-(4-chlorophenyl)-7-iodo-1-[2-(2-methoxyethoxy)ethyl]-1,4-benzodiazepine-2,5-dione 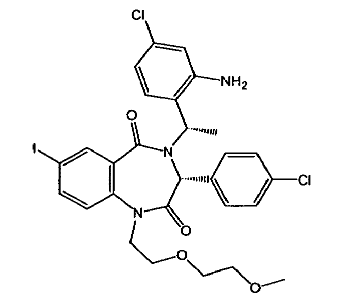 Mass spectrum (LCMS, ESI pos.): Calcd for C₂₈H₂₈Cl₂IN₃O₄: 667; found: 668 (M + H)⁺.

### EXAMPLE 86

4-[1-(2-Amino-4-chlorophenyl)ethyl]-3-(4-chlorophenyl)-7-iodo-1-[2-(4-morpholino)ethyl]-1,4-benzodiazepine-2,5-dione 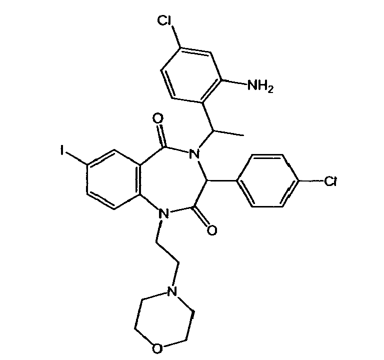 ¹H NMR (400 MHz, CDCl₃) δ 1.76 (d, J = 7.1 Hz, 3H), 2.26-2.31 (m, 2H), 2.42-2.50 (m, 3H), 2.60-2.66 (m, 1H), 3.53-3.70 (m, 5H), 4.48-4.55 (m, 1H), 4.74 (br s, 2H), 5.28 (s, 1H), 6.28 (q, *J* = 7.1 Hz, 1H), 6.60 (dd, J = 8.8 Hz, 2.4 Hz, 1H), 6.66-6.69 (m, 3H), 6.89 (d, *J =* 8.4 Hz, 2H), 7.21-7.19 (d, J = 8.4 Hz, 1H), 7.48 (dd, J = 8.4 Hz, 2.0 Hz), 7.93 (d, *J =* 2.0 Hz, 1H). Mass spectrum (LCMS, ESI pos.): Calcd for C₂₉H₂₉Cl₂IN₄O₃: 678; found: 679 (M + H)⁺.

### EXAMPLE 87

(3S)-4-[(R)-1-(2-Amino-4-chlorophenyl)ethyl]-3-(4-chlorophenyl)-7-iodo-1-[2-(4-morpholino)ethyl]-1,4-benzodiazepine-2,5-dione 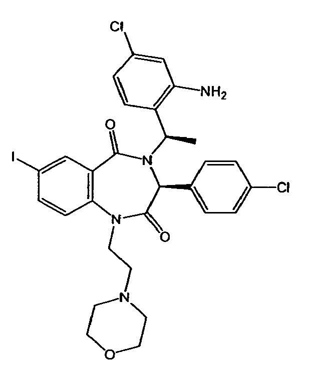 Mass spectrum (LCMS, ESI pos.): Calcd for C₂₉H₂₉Cl₂fN₄O₃: 678; found: 679 (M + H)⁺.

### EXAMPLE 88

(3R)-4-[(S)-1-(2-amino-4-chlorophenyl)ethyl]-3-(4-chlorophenyl)-7-iodo-1-[2-(4-morpholino)ethyl]-1,4-benzodiazepine-2,5-dione 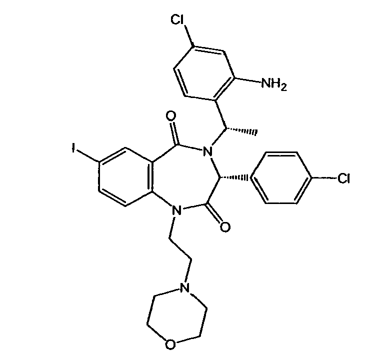 Mass spectrum (LCMS, ESI pos.): Calcd for C₂₉H₂₉Cl₂IN₄O₃: 678; found: 679 (M + H)⁺.

### EXAMPLE 89

3-(4-Chlorophenyl)-4-[1-(2,6-dichloro-3-pyridyl)ethyl]-7-iodo-1,4-benzodiazepine-2,5-dione 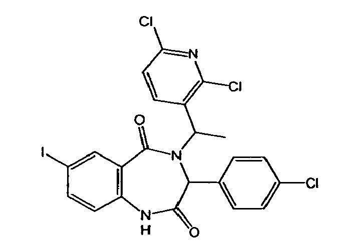 ¹H NMR (400 MHz, DMSO-*d*₆) δ 1.69 (d, J = 6.8 Hz, 3H), 5.28 (s, 1H), 6.09 (q, *J* = 6.8 Hz, 1H), 6.59 (d, J = 8.4 Hz, 1H), 6.87 (d, J = 8.4 Hz, 2H), 7.20 (d, *J =* 8.8 Hz, 2H), 7.55-7.59 (m, 2H), 7.78 *(d, J =* 2.0 Hz, 1H), 8.21 (d, J = 8.0 Hz, 1H), 10.9 (s, 1H). Mass spectrum (LCMS, ESI pos.): Calcd for C₂₂H₁₅Cl₃IN₃O₂: 585; found: 586 (M + H)⁺.

### EXAMPLE 90

1,3-Dihydro-4-[1-(2-amino-4-chlorophenyl)ethyl]-3-(4-chlorophenyl)-7-iodo-1-methyl-1,4-benzodiazepine-2,5-dione 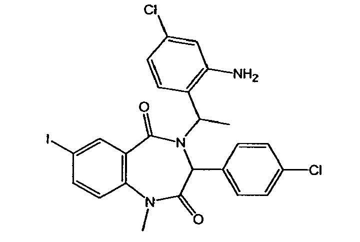 ¹H NMR (400 MHz, CDCl₃) δ 1.75 (d, J = 6.8 Hz, 3H), 3.46 (s, 3H), 5.34 (s, 1H), 5.95 (q, J = 6.8 Hz, 1H), 6.55 (d, J = 8.4 Hz, 2H), 6.61 (d, J = 8.8 Hz, 1H), 6.88-6.91 (m, 3H), 7.30-7.32 (m, 2H), 7.53 (dd, J = 8.8 Hz, 2.0 Hz, 1H), 7.97 (d, J = 2.0 Hz, 1H). Mass spectrum (LCMS, ESI pos.): Calcd for C₂₄H₂₀Cl₂IN₃O₂ 579; found: 580 (M + H)⁺.

### EXAMPLE 91

1,3-Dihydro-4-[1-(2-acetylamino-4-chlorophenyl)ethyl]-3-(4-chlorophenyl)-7-iodo-1,4-benzodiazepine-2,5-dione

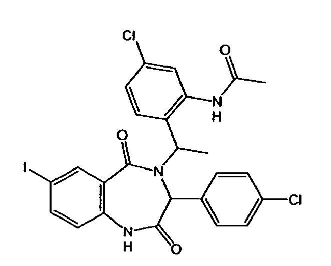

Acetyl chloride (15 µL, 0.212 mmol) was added at 0 °C under argon to a solution of 1,3-dihydro-4-[1-(2-amino-4-chlorophenyl)ethyl]-3-(4-chlorophenyl)-7-iodo-1,4-benzodiazepine-2,5-dione (100 mg, 0.177 mmol), in tetrahydrofuran (5 mL) and chloroform (5 mL). Then, triethylamine (30 µL, 0.21 mmol) was added drop wise and the reaction mixture was allowed warm up to room temperature. After 30 minutes, the solution was concentrated under reduced pressure and the residue was petitioned between ethylacetate and 1N sodium bicarbonate. Organic layer was dried (Na₂SO₄), evaporated, and the residue was purified by column chromatography to give the title compound (49 mg, 45%) as a colorless powder: ¹H NMR (400 MHz, CDCl₃) δ 1.81 (d, *J* = 6.8 Hz, 3H), 2.44 (s, 3H), 5.33 (s, 1H), 6.37 (q, *J =* 6.8 Hz, 1H), 6.47 (d, *J =* 8.4 Hz, 2H), 6.55 (d, J = 8.4 Hz, 1 H), 6.98 (d, J = 8.4 Hz, 2H), 7.03 (dd, J = 8.4 Hz, 2.0 Hz, 1H), 7.36 (d, J = 8.4 Hz, 1H), 7.59 (dd, J = 8.4 Hz, 2.0 Hz, 1H), 7.98 (d, J = 2.0 Hz, 1H), 8.28 (br s, 1H), 8.54 (d, J = 2.0 Hz, 1H), 9.94 (s, 1H). Mass spectrum (LCMS, ESI pos.): Calcd for C₂₅H₂₀Cl₂IN₃O₃: 607; found: 608 (M + H)⁺.

### EXAMPLE 92

1,3-Dihydro-4-[1-(2-azido-3-pyridyl)ethyl]-3-(4-chlorophenyl)-7-iodo-1,4-benzodiazepine-2,5-dione 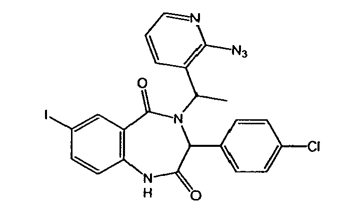 ¹H NMR (400 MHz, CD₃OD) δ 1.94 (d, *J=* 6.8 Hz, 3H), 5.06 (s, 1H), 6.50 (d, *J =* 8.4 Hz, 1H), 6.85 (q, *J =* 6.8 Hz, 1H), 7.01-7.04 (m, 2H), 7.19 (d, *J =* 8.8 Hz, 2H), 7.45 (t, *J =* 7.0 Hz, 1H), 7.56 (dd, *J =* 8.8 Hz, 2.0 Hz, 1H), 7.94 (d, *J* = 7.2 Hz, 1H), 8.06 (d, *J* = 2.4 Hz, 1H), 9.12 (d, *J* = 6.8 Hz, 1H). Mass spectrum (LCMS, ESI pos.): Calcd for C₂₂H₁₆ClIN₆O₂: 559; found: 560 (M + H)⁺.

### EXAMPLE 93

4-[1-(2-Amino-4-chlorophenyl)ethyl]-3-(4-chlorophenyl)-1-[2-(2-methoxyethoxy)ethyl]- 7-(propyn-1-yl)-1,4-benzodiazepine-2,5-dione hydrochloride 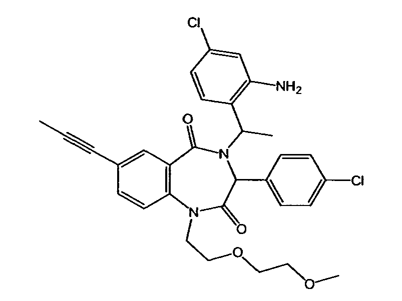 ¹H NMR (400 MHz, DMSO-*d*_{*6*}) δ 1.61 (d, J= 6.8 Hz, 3H), 2.00 (s, 3H), 3.17 (s, 3H), 3.30-3.65 (m, 6H), 3.85-3.92 (m, 1H), 4.33-4.39 (m, 1H), 5.13 (s, 1H), 6.08 (q, J = 6.8 Hz, 1H), 6.52 (dd, J = 8.4 Hz, 2.0 Hz, 1H), 6.69 (d, J = 7.6 Hz, 2H), 6.78 (d, J = 2.0 Hz, 1H), 6.98 (d, J = 8.4 Hz, 2H), 7.20-7.28 (m, 3H), 7.40 (d, J = 2.0 Hz, 1H). Mass spectrum (LCMS, ESI pos.): Calcd for C₃₁H₃₁Cl₂N₃O₄₁: 579; found: 580 (M + H)⁺.

### EXAMPLE 94

(3S)-4-[(R)-1-(2-Amino-4-chlorophenyl)ethyl]-3-(4-chlorophenyl)-1-[2-(2-methoxyethoxy)ethyl]- 7-(propyn-1-yl)-1,4-benzodiazepine-2,5-dione hydrochloride 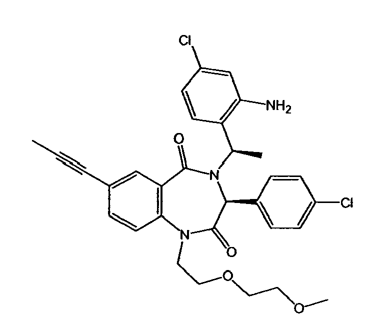 Mass spectrum (LCMS, ESI pos.): Calcd for C₃₁H₃₁Cl₂N₃O₄: 579; found: 580 (M + H)⁺.

### EXAMPLE 95

4-[1-(2-Amino-4-chlorophenyl)ethyl]-3-(4-chlorophenyl)-1-[2-(4-morpholino)ethyl]-7-(propyn-1-yl)-1,4-benzodiazepine-2,5-dione hydrochloride 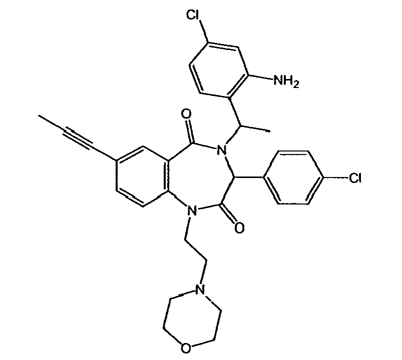 ¹H NMR (400 MHz, CDCl₃) δ 1.76 (d, J = 6.8 Hz, 3H), 2.02 (s, 3H), 2.22-2.49 (m, 5H), 2.61-2.67 (m, 1H), 3.53-3.71 (m, 5H), 4.49-4.56 (m, 1H), 4.76 (s, 2H), 5.27 (s, 1H), 6.30 (q, J = 6.8 Hz, 1H), 6.60 (dd, J = 8.4 Hz, 2.0 Hz, 1H), 6.66-6.68 (m, 3H), 6.83 (d, J = 8.8 Hz, 1H), 6.87 (d, J = 8.4 Hz, 2H), 7.16-7.21 (m, 2H), 7.66 (d, *J* = 2.0 Hz, 1H). Mass spectrum (LCMS, ESI pos.): Calcd for C₃₂H₃₂Cl₂N₄O₃: 590; found: 591 (M + H)⁺.

### EXAMPLE 96

(3S)-4-[(R)-1-(2-Amino-4-chlorophenyl)ethyl]-3-(4-chlorophenyl)-1-[2-(4-morpholino)ethyl]-7-(propyn-1-yl)-1,4-benzodiazepine-2,5-dione hydrochloride 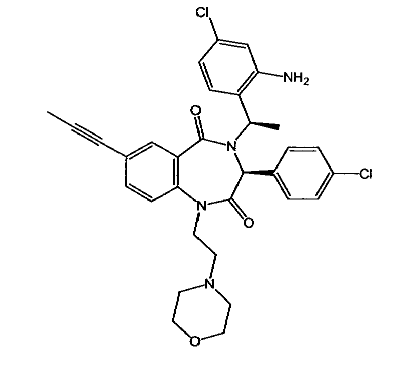 Mass spectrum (LCMS, ESI pos.): Calcd for C₃₂H₃₂Cl₂N₄O₃: 590.19; found: 591.0 (M + H)⁺.

### EXAMPLE 97

4-[1-(2-Amino-4-chlorophenyl)ethyl]-3-(4-chlorophenyl)-7-iodo-2,3-dihydro-1H-1,4-benzodiazepin-5-one 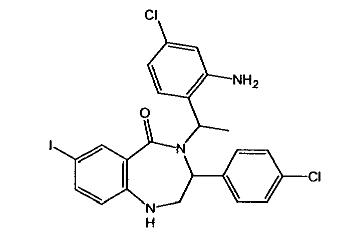
a) 4-[1-(4-Chloro-2-nitrophenyl)ethyl]-3-(4-chlorophenyl)-7-iodo-2,3-dihydro-1*H*-1,4-benzodiazepin-5-one
   A solution of borane methyl sulfide complex (1.68 mL, 2M in THF) was slowly added to a solution of 4-[1-(4-chloro-2-nitrophenyl)ethyl]-3-(4-chlorophenyl)-7-iodo-1,3-dihydro-1,4-benzodiazepine-2,5-one (500 mg, 0.839 mmol) in anhydrous tetrahydrofuran (25 mL). The resulting solution was heated at 65 °C for 2 h. Then, the reaction mixture was cooled at 0 °C and NaOH (10 mL, 3N) was slowly added. The solvent was evaporated and the residue partitioned between ice-cold water and ethyl acetate, dried (Na₂SO₄), and evaporated. Purification by chromatography on silica (AcOEt/Hexanes/CH₂Cl₂, 1:2:1) afforded a powder, which was triturated in ether to give the title compound (370 mg, 76 %) a yellow solid: ¹H NMR (400 MHz, CDCl₃) δ 1.70 (d, J = 6.8 Hz, 3H, CH₃), 3.50 (d, J = 14.0 Hz, 1H), 3.73 (dd, *J =* 14.0, 6.4 Hz, 1H), 4.42 (br s, 1H, NH), 4.67 (d, *J =* 6.4 Hz, 1H), 6.17 (q, J = 6.8 Hz, 1H), 6.72 (d, J = 8.4 Hz, 2H), 7.07 (d, J = 8.4 Hz, 2H), 7.37 (dd, *J =* 8.4, 2.0 Hz, 1H), 7.50 (dd, *J =* 8.4, 2.0 Hz, 1H), 7.61-7.64 (m, 2H), 8.58 (d, J = 2.0 Hz, 1H). Mass spectrum (LCMS, ESI pos.): Calcd for C₂₃H₁₈Cl₂IN₃O₃: 580.9; found: 582.0 (M + H)⁺.
b) 4-[1-(2-Amino-4-chlorophenyl)ethyl]-3-(4-chlorophenyl)-7-iodo-2,3-dihydro-1*H*-1,4-benzodiazepin-5-one
   ¹H NMR (400 MHz, CDCl₃) δ 1.63 (d, J = 6.8 Hz, 3H), 3.60 (d, *J*= 14.0 Hz, 1H), 4.05 (dd, J = 14.0, 6.0 Hz, 1H), 4.72 (d, J = 5.2 Hz, 1H), 6.12 (d, J = 8.8 Hz, 1H), 6.33 (q, J = 6.8 Hz, 1H), 6.49 (br s, 1H), 6.54 (dd, J = 8.0, 1.6 Hz, 1H), 6.77 (d, J = 8.4 Hz, 2H), 6.95-7.01 (m, 3H), 7.28 (dd, J = 8.4, 2.0 Hz, 1H), 8.46 (d, J = 2.0 Hz, 1H). Mass spectrum (LCMS, ESI pos.): Calcd for C₂₃H₂₀Cl₂IN₃O: 551.0; found: 552.0 (M + H)⁺.

### EXAMPLE 98

4-[1-(2-amino-6-chloro-3-pyridyl)methyl]-3-(4-chlorophenyl)-7-iodo-1-[2-(2-methoxyethoxy)ethyl]-1,4-benzodiazepine-2,5-dione hydrochloride 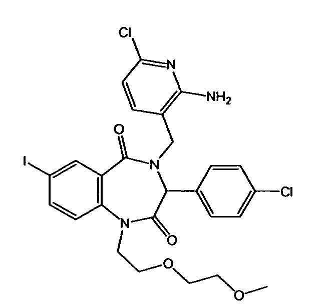 ¹H NMR (400 MHz, CDCl₃) δ 3.31 (s, 3H), 3.42-3.53 (m, 4H), 3.64-3.67 (m, 2H), 3.91-3.97 (m, 1H), 4.31-4.38 (m, 1H), 5.00 (dd, J= 33.6, 15.2 Hz, 2H), 5.56 (s, 1H), 6.85 (d, J = 7.6 Hz, 1H), 6.86-6.89 (m, 2H), 7.10-7.13 (m, 3H), 7.64 (dd, J = 8.4, 2.0 Hz, 1H), 7.86-7.88 (m, 2H). Mass spectrum (LCMS, ESI pos.): Calcd for C₂₆H₂₅Cl₂IN₄O₄: 654.0; found: 655.0 (M + H)⁺.

### EXAMPLE 99

4-[1-(3-Amino-4-chlorophenyl)cyclopropyl]-3-(4-chlorophenyl)-1-[2-(4-morpholino)ethyl]-7-iodo-1,4-benzodiazepine-2,5-dione methanesulfonate 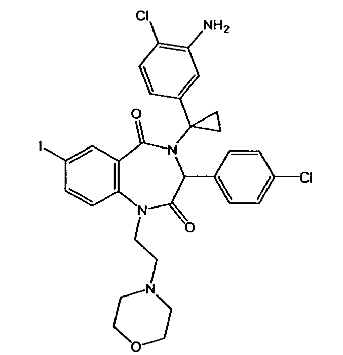 ¹H NMR (400 MHz, CDCl₃) δ 1.29-1.38 (m, 2H), 1.53-1.60 (m, 1H), 1.81-1.86 (m, 1H), 3.17-3.29 (m, 4H), 3.65-4.11 (m, 7H), 4.47-4.54 (m, 1H), 5.98 (s, 1H), 6.75 (d, *J* = 8.0 Hz, 2H), 6.86 (d, *J* = 8.8 Hz, 1H), 7.11 (d, *J* = 8.0 Hz, 2H), 7.21 (d, J = 8.0 Hz, 1H), 7.33 (d, J = 8.0 Hz, 1H), 7.42 (s, 1H), 7.64-7.67 (m, 1H), 7.83 (d, J = 1.6 Hz, 1H). Mass spectrum (LCMS, ESI pos.): Calcd for C₃₀H₂₉Cl₂lN₄O₃: 690.0; found: 691.0 (M + H)⁺.

### EXAMPLE 100

(3S)-4-[1-(2-Amino-4-chlorophenyl)ethyl]-3-(4-chlorophenyl)-7-iodo-1-[(R)-2-(1-piperazinyl)-2-oxoethyl]-1,4-benzodiazepine-2,5-dione hydrochloride 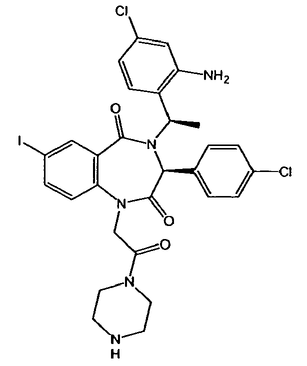 ¹H NMR (400 MHz, CD₃OD) δ 1.72 (d, J = 7.0 Hz, 3H), 3.31-3.40 (m, 2H), 3.88-3.95 (m, 6H), 4.64 (d, J = 16.8 Hz, 1H), 5.05 (d, J = 16.8 Hz, 1H), 5.31 (s, 1H), 6.26 (q, J = 7.0 Hz, 1H), 6.59 (dd, J = 8.4, 2.4 Hz, 1H), 6.79-6.83 (m, 2H), 6.93-7.00 (m, 4H), 7.28-7.30 (d, J = 8.0 Hz, 1H), 7.60 (dd, J = 8.8, 2.4 Hz, 1H), 7.80 (d, J = 2.0 Hz, 1H). Mass spectrum (LCMS, ESI pos.): Calcd for C₂₉H₂₈Cl₂IN₅O₃: 691.0; found: 692.0 (M + H)⁺.

### EXAMPLE 101

4-(4-Chloro-2-methyl-benzyl)-3-(4-chloro-phenyl)-7-iodo-1-(2-morpholin-4-yl-ethyl)-3,4-dihydro- 1H- benzo[e] [1,4]diazepine-2,5-dione 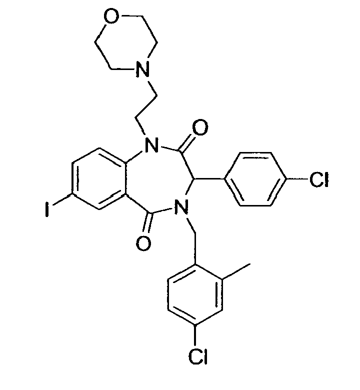 ¹H NMR (400 MHz, DMSO-d₆) δ 7.95-7.96 (d, *J*=2.1 Hz, 1H), 7.46-7.52 (m, 1H), 7.33-7.36(m, 1H), 7.28-7.29 (m, 4H), 7.16-7.24 (m, 2H), 6.99-7.02 (m, 2H), 6.68-6.73 (m, 3H), 5.34-5.40 (d, J=14.6 Hz, 1H), 5.30 (s, 1H), 4.68-4.72 (d, J=14.6 Hz, 1H), 4.46-4.56 (m, 1H), 3.58-3.63 (m, 5H), 2.40-2.54 (m, 5H). Mass spectrum (LCMS, ESI pos.): Calcd for C₂₉H₂₈Cl₂IN₃O₃: 663.0; found 664.1 (M + H).

### EXAMPLE 102

3-(S)-(4-Chloro-phenyl)-4-[1-(R)-(4-chloro-phenyl)-ethyl]-7-iodo-1-(2-morpholin-4-yl-2-oxo-ethyl)-3,4-dihydro-1H-benzo[e][1,4]diazepine-2,5-dione 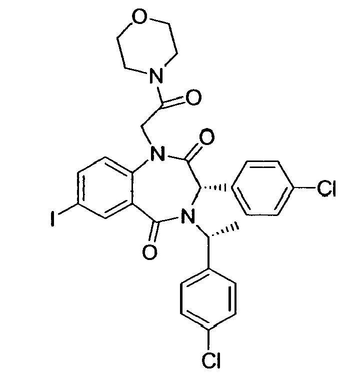 ¹H NMR (400 MHz, DMSO-d₆) δ 7.91-7.92 (d, *J*=2.1 Hz, 1H), 7.51-7.35 (m, 2H), 7.43-7.46(m, 1H), 7.35-7.40 (m, 2H), 6.90-6.96 (m, 2H), 6.72-6.76 (m, 2H), 6.60-6.63(d, J=8.6 Hz, 1H), 6.40-6.48 (m, 1H), 5.35-5.36 (s, 1H), 5.00-5.042 (d, J=16.0 Hz, 1 H), 4.02-4.07 (d, *J*=16.1 Hz, 1H), 3.44-3.88 (m, 9H), 1.71-1.74 (d, *J*=7.1 Hz, 2H). Mass spectrum (LCMS, ESI pos.): Calcd forC₂₉H₂₆Cl₂IN₃O₄: 677.0; found 677.8 (M + H).

### EXAMPLE 103

3-(S)-(4-Chloro-phenyl)-4-[1-(R)-(4-chloro-phenyl)-ethyl]-7-iodo-1-(2-morpholin-4-yl-ethyl)-3,4-dihydro-1H-benzo[e][1,4]diazepine-2,5-dione 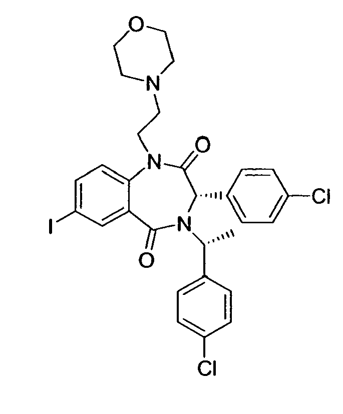 ¹H NMR (400 MHz, DMSO-d₆) δ 7.94-7.95 (d, *J*=2.1 Hz, 1H), 7.40-7.48 (m, 3H), 7.26-7.30 (m, 2H), 6.84-6.88 (m, 2H), 6.60-6.68 (d, *J*=8.8 Hz, 2H), 6.46-6.50 (d, *J*=7.5 Hz, 2H), 6.36.44(m, 1H), 5.30 (s, 1H), 4.44-4.54 (m, 1H), 4.04-4.10 (m, 1H), 3.50-3.68 (m, 5H), 2.58-2.60 (m, 1H), 2.36-2.46 (m, 3H), 2.24-2.28 (m, 1H), 1.73-1.75 (d, *J=*7.1 Hz, 2H). Mass spectrum (LCMS, ESI pos.): Calcd for C₂₉H₂₈Cl₂IN₃O₃: 663.0; found 664.1 (M + H).

### EXAMPLE 104

3-(R)-(4-Chloro-phenyl)-4-[1-(R)-(4-chloro-phenyl)-ethyl]-7-iodo-1-(2-morpholin-4-yl-ethyl)-3,4-dihydro-1 H-benzo[e] [1,4]diazepine-2,5-dione 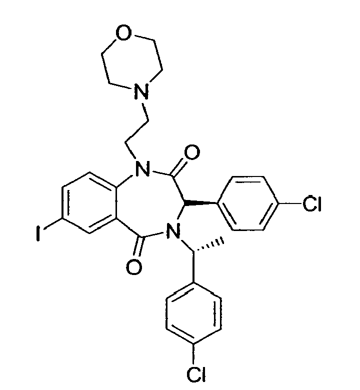 ¹H NMR (400 MHz, DMSO-d₆) δ 7.99-8.01 (d, *J*=2.1 Hz, 1H), 7.28-7.48 (m, 5H), 7.08-7.10 (d,*J*=8.6 Hz, 2H), 6.83-6.85 (d, J=8.6 Hz, 2H), 6.59-6.62 (d, J=8.6 Hz, 2H), 6.34-6.40 (m, 1H), 5.32-5.34 (s, 1H), 4.04-4.22 (m, 2H), 3.42-3.58 (m, 5H), 2.40-2.46 (m, 1H), 2.20-2.32 (m, 3H), 1.58-1.63 (d, *J*=7.1 Hz, 3H). Mass spectrum (LCMS, ESI pos.): Calcd forC₂₉H₂₈Cl₂IN₃O₃: 663.0; found 664.2 (M + H).

### EXAMPLE 105

3-(S)-(4-Chloro-phenyl)-4-[1-(R)-(4-chloro-phenyl)-ethyl]-7-iodo-1-[2-(2-methoxy-ethoxy)-ethyl]-3,4-dihydro-1 H-benzo[e][1,4]diazepine-2,5-dione 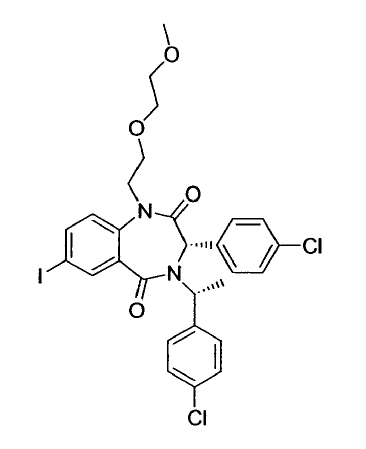 ¹H NMR (400 MHz, DMSO-d₆) δ 7.91-7.92 (d, *J*=2.1 Hz, 1H), 7.40-7.50 (m, 3H), 7.28-7.30 (m, 2H), 6.82-6.86 (m, 3H), 6.46-6.49 (m, 2H), 6.36-6.42 (m, 2H), 5.28 (s, 1H), 4.10-4.20 (m, 2H), 3.92-4.00 (m, 2H), 3.80-3.88 (m, 1H), 3.68-3.72 (m, 2H), 3.44-3.60 (m, 4H), 1.70-1.73 (d,*J*=7.3 Hz, 3H). Mass spectrum (LCMS, ESI pos.): Calcd forC₂₈H₂₇Cl₂IN₂O₄: 652.0; found 653.3 (M + H).

### EXAMPLE 106

3-(S)-(4-Chloro-phenyl)-4-[1-(R)-(4-chloro-phenyl)-ethyl]-7-(1-hydroxyimino-ethyl)-3,4-dihydro-1 H-benzo[e][1,4]diazepine-2,5-dione

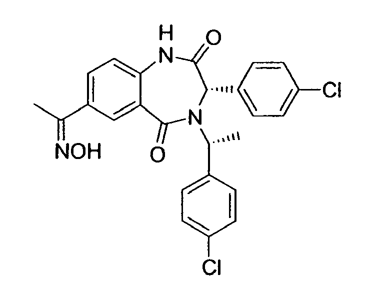

A solution of 7-Acetyl-3-(S)-(4-chloro-phenyl)-4-[1-(R)-(4-chloro-phenyl)-ethyl]-3,4-dihydro-1H-benzo[e][1,4]diazepine-2,5-dione (50 mg, 0.11 mmol), hydroxylamine hydrochloride salt (22 mg, 0.33 mmol), triethylamine (0.074 mL, 0.55 mmol) in EtOH (0.5 mL) was heated to 80°C. After 2 h reaction solution was applied to a prep tlc plate (Analtech Silica Gel GF, 20 X 20 cm, 2000 microns) and developed using methylene chloride/MeOH 10:1. Desired bands were scraped off, extracted with MeOH, filtered and concentrated to give the title compound (2.4 mg). ¹H NMR (400 MHz, DMSO-d₆) δ 7.82-7.83 (m, 1H), 7.47-7.51 (m, 3H), 7.32 (s, 3H), 7.26-7.30 (m, 2H), 6.80-6.84 (m, 2H), 6.66-6.70 (d, J=8.4 Hz, 2H), 6.52-6.55 (d, J=8.6 Hz, 2H), 6.36-6.42 (m, 2H), 5.17 (s, 1H), 1.67-1.71 (d, J=7.3 Hz, 3H). Mass spectrum (LCMS, ESI pos.): Calcd forC₂₅H₂₁Cl₂N₃O₃: 481.1; found 482.1 (M + H).

### EXAMPLE 107

7-Iodo-4-naphthalen-1-ylmethyl-3-(4-trifluoromethoxy-phenyl)-3,4-dihydro-1H-benzo[e][1,4]diazepine-2,5-dione 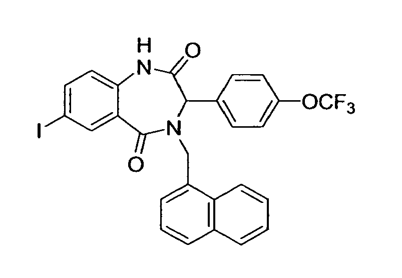 ¹H NMR (400 MHz, DMSO-d₆) δ 10.76-10.80 (s, 1H), 8.16-8.20 (d, J=8.0 Hz, 1H), 7.85-8.00 (m, 2H), 7.80-7.82 (m, 1 H), 7.54-7.61 (m, 5H), 7.00-7.20 (m, 4H), 6.60-6.64 (d, J=8.6 Hz, 1H), 5.86-6.00 (d, J=14.9 Hz, 1H), 5.50 (s, 1H), 5.10-5.16 (d, *J*=15.1 Hz, 1H). Mass spectrum (LCMS, ESI pos.): Calcd forC₂₇H₁₈F₃IN₂O₃: 602.0; found 603.3 (M + H).

### EXAMPLE 108

4-Benzo[1,3]dioxol-5-ylmethyl-3-(4-chloro-phenyl)-7-iodo-3,4-dihydro-1H-benzo[e][1,4] diazepine-2,5-dione 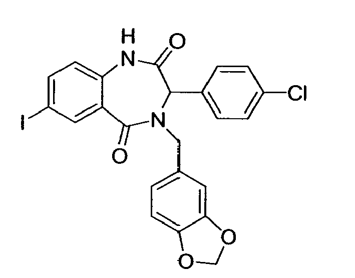 ¹H NMR (400 MHz, DMSO-d₆) δ 7.91-7.92 (m, 1H), 7.55-7.60 (m,1H), 7.38-7.41 (m, 1H), 7.25-7.30 (m, 3H), 7.10-7.13 (d, *J=* 8.6 Hz, 2H), 6.87-7.00 (m, 2H), 6.77-6.81 (d, *J*=7.7 Hz, 1H), 6.57-6.63 (m, 1H), 6.33-6.37 (m, 1H), 5.93-5.96 (m, 1H), 5.85-5.88 (m, 1H), 5.43-5.45 (s, 1H). Mass spectrum (LCMS, ESI pos.): Calcd for C₂₃H₁₆ClIN₂O₄: 545.9; found 547.8 (M + H).

### EXAMPLE 109

4-Benzo[1,3]dioxol-5-ylmethyl-7-iodo-3-(4-trifluoromethoxy-phenyl)-3,4-dihydro-1 H-benzo[e][1,4]diazepine-2,5-dione 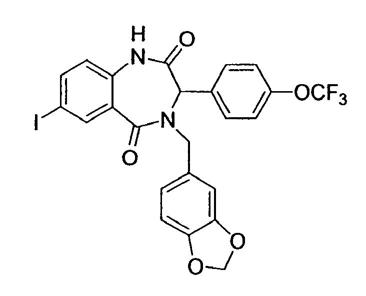 ¹H NMR (400 MHz, DMSO-d₆) δ 7.88-7.90 (m, 1H), 7.54-7.70 (m,1H), 7.30-7.40 (m, 1H), 7.15-7.17 (m, 1H), 6.95-7.04 (m, 3H), 6.78-6.81 (d, J=7.9 Hz, 2H), 6.59-6.6.63 (d, J=8.6 Hz, 2H), 6.32-6.36 (m, 1H), 5.93-5.96 (m, 1H), 5.83-5.87 (m, 2H), 5.48-5.49 (s, 1H). Mass spectrum (LCMS, ESI pos.): Calcd forC₂₄H₁₆F₃IN₂O₅: 596.01; found 597.2 (M + H).

### EXAMPLE 110

7-Iodo-4-(2-pyridin-2-yl-ethyl)-3-(4-trifluoromethoxy-phenyl)-3,4-dihydro-1H-benzo[e][1,4]diazepine-2,5-dione 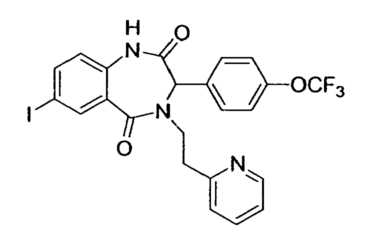 ¹H NMR (400 MHz, DMSO-d₆) δ 8.45-8.47(m, 1H), 7.82-7.84 (m,2H), 7.71-7.76 (m, 1H), 7.51-7.53 (m, 1H), 7.3 8-7.41 (m, 1H), 7.23-7.27 (m, 1H), 7.08-7.14 (m, 4H), 6.58-6.61 (d, J=8.4 Hz, 1H), 5.60 (s, 1H), 4.54-4.62 (m, 1H), 3.92-4.01 (m, 1H), 3.18-3.28 (m, 2H). Mass spectrum (LCMS, ESI pos.): Calcd forC₂₃H₁₇F₃IN₃O₃: 567.0; found 568.1 (M + H).

### EXAMPLE 111

4-Benzyl-3-(4-chloro-phenyl)-7-iodo-3,4-dihydro-1H-benzo[e] [1,4] diazepine-2,5-dione 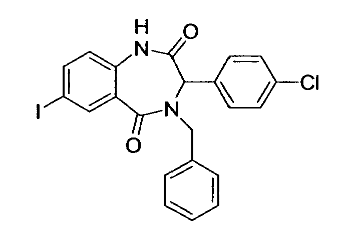 ¹H NMR (400 MHz, DMSO-d₆) δ 7.80-7.82 (m, 1H), 7.59-7.62 (m,2H), 7.20-7.50 (m, 7H), 6.90-7.00 (m, 2H), 6.65-6.70 (d, *J*=8.6 Hz, 1H), 5.55-5.58 (m, 1H), 5.20-5.30 (d, *J*=14 Hz, 1H), 4.77-4.84 (d, *J*=14.6 Hz, 1H). Mass spectrum (LCMS, ESI pos.): Calcd forC₂₂Hₗ₆ClIN₂O₂: 501.9; found 503.1 (M + H).

### EXAMPLE 112

3-(4-Chloro-phenyl)-7-iodo-4-phenethyl-3,4-dihydro-1H-benzo[e][1,4]diazepine-2,5-dione 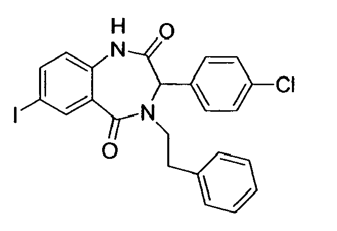 ¹H NMR (400 MHz, DMSO-d₆) δ 10.8-10.9 (s, 1H), 7.78-7.80 (m, 1H), 7.56-7.60 (m, 2H), 7.24-7.36 (m, 5H), 7.16-7.20 (m, 2H), 6.95-7.04 (m, 1H), 6.64-6.68 (d, *J*=8.4 Hz, 1H), 5.70-5.76 (s, 1H), 4.18-4.28 (m, 1H), 3.76-3.84 (m, 1 H), 2.84-3.10 (m, 2H). Mass spectrum (LCMS, ESI pos.): Calcd forC₂₃H₁₈ClIN₂O₂: 516.0; found 517.1 (M + H).

### EXAMPLE 113

4-Benzyl-7-iodo-3-(4-trifluoromethoxy-phenyl)-3,4-dihydro-1H-benzo[e][1,4]diazepine-2,5-dione 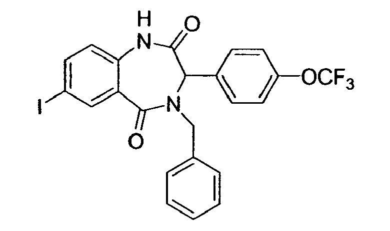 ¹H NMR (400 MHz, DMSO-d₆) δ 7.72-7.80 (s, 1H), 7.56-7.60 (m, 2H), 7.28-7.50 (m, 7H), 7.10-7.18 (m, 2H), 6.60-6.70 (m, 1H), 5.60 (s, 1H), 5.28-5.34 (d, *J*=14.6 Hz, 1H), 4.76-4.82(d, *J=*14.8 Hz, 1H). Mass spectrum (LCMS, ESI pos.): Calcd forC₂₃H₂₆F₃IN₂O₃: 552.0; found 553.1 (M + H).

### EXAMPLE 114

7-Iodo-4-phenethyl-3-(4-trifluoromethoxy-phenyl)-3,4-dihydro-1H-benzo[e][1,4]diazepine-2,5-dione 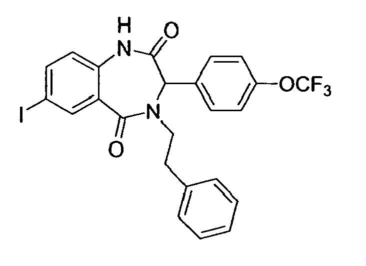 ¹H NMR (400 MHz, DMSO-d₆) δ 10.8-11.0 (s, 1H), 7.50-7.64 (m, 3H), 7.02-7.40 (m, 8H), 6.57-7.0 (m, 1H), 5.60 (s, 1H), 4.40-4.40 (m, 1H), 3.80-3.90 (m, 1H), 2.90-3.10(m, 2H). Mass spectrum (LCMS, ESI pos.): Calcd for C₂₄H₁₈F₃IN₂O₃: 566.0; found 567.0 (M + H).

### EXAMPLE 115

### Fluorescent peptide assay

The inhibition of MDM2 binding to p53 was measured using a p53 peptide analogue binding to MDM2 residues 17-125. The published crystal structure of this complex (Kussie *et al., Science* 274:948-953 (1996)) validates this fragment as containing the p53 binding site, and we have solved the x-ray structure of the p53 peptide analogue MPRFMDYWEGLN, described to be a peptide inhibitor of the MDM2 p53 interaction (Bottger *et al., J. Mol. Biol.* 269:744-756 (1997)). The assay uses N terminal fluorescein RFMDYWEGL peptide (Fl 9mer).

The mdm2 17-125 was produced as a glutathione S transferase fusion as follows: cDNA encoding residues 17-125 were cloned into pGEX4t-3 (Pharmacia) as follows. PCR was performed using ATCC item number 384988 containing partial human mdm2 sequence as template and the following primers: Forward: 5'-CTC TCT CGG ATC CCA GAT TCC AGC TTC GGA ACA AGA G; Reverse: 5'-TAT ATA TCT CGA GTC AGT TCT CAC TCA CAG ATG TAC CTG AG. The PCR product was then digested with BamHI and XhoI (sequence recognition sites underlined in primers), gel purified, and ligated into pGEX4t-3 which had also been digested with BamHI and Xhol. Plasmids were transfected into *E. coli* X90 strain, grown to an OD of 1.0 in TB 0.2% glucose 100 µg/mL ampicillin and induced with I mM IPTG. Cells were harvested 5 hours post induction, centrifuged, and resuspended in PBS 10 mL/g cell paste. Cells were lysed in an Avestin microfluidizer, centrifuged, and the supernatant bound to a glutathione sepharose 4B resin (Pharmacia). The resin was washed with PBS and the MDM2 17-125 cleaved from the GST by the addition of 2 µg/mL thrombin (Enzyme Research Labs). The cleaved MDM2 was further purified on Sepharose SP Fast Flow resin (Pharmacia), eluting with 20 mM HEPES pH 7.5 150 mM NaCl. Glutathione was added to 5 mM, and the protein stored at -70°C.

Test compound was incubated for 15 minutes with 30 nM fluorescein peptide Fl 9mer and 120 nM MDM2 17-125 in 50 mM HEPES pH 7.5, 150 mM NaCl, 3 mM octyl glucoside. The polarization of the fluorescein label was thereafter measured by excitation at 485 nm and emission at 530 nm. Polarization was expressed as a percent of a no compound control, using buffer with Fl 9mer but without MDM2 as background. Compounds of the present invention inhibited the binding of p53 to MDM2. The potency of the compounds was measured as IC₅₀, which is a measure of the concentration of the test compound required to inhibit 50% binding between MDM2 and p53. The IC₅₀ values for compounds of the present invention ranged from 0.05 µM to >100 µM. See examples below.

| Compound | range (µM) |
|---|---|
| 4-[(R)-1(2-amino-4-chlorophenyl)ethyl]-(3 S)-3-(4-c hlorophenyl)-7-iodo-1-[2-(2-methoxyethoxy)ethyl]-1, 4-benzodiazepine-2,5-dione | 0.1-1.0 |
| 4-[(R)-1(2-Amino-4-chlorophenyl)-ethyl]-(3S)-3-(4-chl oro-phenyl)-7-iodo-1-[3-(4-methyl-piperazin-1-yl)-prop, yl]-3,4-dihydro-1H-benzo[e][1,4]diazepine-2,5-dione | 0.1-1.0 |
| 5-{(3S)-3-(4-Chlorophenyl)-4-[(R)-1-(4-chlorop henyl)-ethyl]-7-iodo-2,5-dioxo-2,3,4,5-tetrahydr o-benzo[e][1,4]diazepin-1-yl}-pentanoic acid | 0.1-1.0 |
| (3S)-4-[1-(2-Amino-4-chlorophenyl)ethyl]-3-(4-ch lorophenyl)-7-iodo-1-[(R)-2-(1-piperazinyl)-2-oxo ethyl]-1,4-benzodiazepine-2,5-dione hydrochloride | 1.0-2.0 |
| 4-(R)-[1-(2-Amino-4-chloro-5-fluoro-phenyl)-ethyl]-3(S)-( 4-chloro-phenyl)-7-iodo-1-(2-morpholin-4-yl-ethyl)-3,4-di hydro-1H-benzo[e][1,4]diazepine-2,5-dione | 1.0-3.0 |

### EXAMPLE 116

### Tablet Preparation

Tablets containing 25.0, 50.0, and 100.0 mg, respectively, of the compound of Example 1 ("active compound") are prepared as illustrated below:

**TABLET FOR DOSES CONTAINING FROM 25-100 MG OF THE ACTIVE COMPOUND**

| Amount-mg | | | |
|---|---|---|---|
| Active compound | 25.0 | 50.0 | 100.00 |
| Microcrystalline cellulose | 37.25 | 100.0 | 200.0 |
| Modified food corn starch | 37.25 | 4.25 | |
| Magnesium stearate | 0.50 | 0.75 | 1.5 |

All of the active compound, cellulose, and a portion of the corn starch are mixed and granulated to 10% corn starch paste. The resulting granulation is sieved, dried and blended with the remainder of the corn starch and the magnesium stearate. The resulting granulation is then compressed into tablets containing 25.0, 50.0, and 100.0 mg, respectively, of active ingredient per tablet.

### EXAMPLE 117

### Intravenous Solution Preparation

An intravenous dosage form of the compound of Example 1 ("active compound") is prepared as follows:

| | |
|---|---|
| Active compound | 0.5-10.0 mg |
| Sodium citrate | 5-50 mg |
| Citric acid | 1-15 mg |
| Sodium chloride | 1-8 mg |
| Water for injection (USP) | q.s. to 1 ml |

Utilizing the above quantities, the active compound is dissolved at room temperature in a previously prepared solution of sodium chloride, citric acid, and sodium citrate in Water for Injection (USP, see page 1636 of United States Pharmacopeia/National Formulary for 1995, published by United States Pharmacopeial Convention, Inc., Rockville, Maryland (1994).

### EXAMPLE 118

### Benzodiazapinediones Suppress Tumor Cell Proliferation

The antiproliferative activities of benzodiazapinedione compounds were measured across a panel of human tumor cell lines. Table 1 lists exemplary compounds that were tested: TDP222669 (4-Chlorophemyl)-[3-(4-chlorophenyl)-7-iodo-2,5-dioxo-1,2,3,4-tetrahydrobenzo[e] [1,4]diazepine-4-yl]-acetic acid, Example 62 in WO03/041715), TDP665759 (4-[1-(2-Amino-4-chlorophenyl)-ethyl]-3-(4-chlorophenyl)-7-iodo-1-[3-(4-methylpiperazin-1-yl)-propyl]-3,4-dihydro-1H-benzo[e][1,4]diazepine-2,5-dione, Example 66 in WO2004096134), and TDP521252 (5-{(3S)-3-(4-Chlorophenyl)-4-[(R)-1-(4-chlorophenyl)-ethyl]-7-ethynyl-2,5-dioxo-2,3,4,5-tetrahydrobenzo[e][1,4]diazepine-1-yl}-pentanoic acid, Example 2 in WO2004096134). Other compounds can be tested using similar procedures as described herein.

**Cell Lines**: JAR choriocarcinoma cells (HTB-144), HepG2 hepatocellular carcinoma cells (HTB-8065) and PC3 prostate cancer cells (CRL-1435), all from ATCC (Manassas, VA) were maintained as recommended in RPMI, EMEM and F12K supplemented with 10% FBS, respectively. Other cell lines as detailed in Figure 1A were also purchased from ATCC and grown as recommended. The mdm2/p53 double null and mdmX/p53 double null cell lines were the generous gift of Dr. Guillerrnina Lozano (Univ. of Texas M.D. Anderson Cancer Center, Houston, Texas) and were maintained as described (Parant et al., 2001*, Nat Genet* 29, 92-95.).

Proliferation Assay: Cells were seeded at 1 x 10³ cells per well in 96 well plates. After allowing for adherence overnight, cells were treated with various concentrations of compounds for 3 days. Cells were labeled with BrdU for 6 hours, fixed and assayed by ELISA according to manufacturer's instructions (BrdU Cell Proliferation Assay, Exalpha, Watertown, MA). The values presented in Table 2 are the mean of three experiments, unless otherwise specified.

TDP222669 is a relatively potent Hdm2 inhibitor *in vitro* with an IC₅₀ of 0.57 µM from the fluorescence polarization (FP) assay (Table 1). The cellular potency of TDP222669 was poor, however, most likely due to low cell permeability. Replacement of the acid moiety of TDP222669 with a methyl group and introduction of a valeryl (TDP521252) or a 3-(4-methylpiperazin-1-yl) propyl (TDP665759) solubilizing moiety at position 1 of the 1,4-benzodiazepine-2,5-dione afforded compounds with roughly equivalent *in vitro* potencies relative to TDP222669 but approximately 3-66 fold improved cellular activity (Table 2, and Figure 1).

Consistent with their roles as inhibitors of HMD2-p53 interactions, these compounds were less effective at controlling the growth of tumor cells expressing mutant or null p53. Compared with mutant/null p53 cell lines, cells expressing wt p53 were about 4-fold more sensitive to the cell growth inhibitory activity of TDP222669 or TDP521252, or about 10-fold more sensitive to the cell growth inhibitory activity of TDP665759 (Table 2 & Fig.1). Similarly, tumor cells with reduced amount of Hdm2 mRNA were also less sensitive to the cell growth inhibitory activity of the compounds (data not shown).

### EXAMPLE 119

### Benzodiazapinediones cause the dissociation of cellular Hdm2 from p53

The direct effect of benzodiazepediones on the Hdm2: p53 complex in cells was studied in JAR choriocarcinoma cells, which have higher and thus more readily detectable basal levels of Hdm2 and p53.

JAR choriocarcinoma cells were first treated for 90 minutes with increasing doses of compound TDP521252 or TDP258625 (Table 1) as well as their inactive enantiomers TDP536356 and TDP258626, respectively. Subsequently, the cells were lysed in TritonX-100 buffer (lysis buffer) [1% Triton X-100, 10mM Tris pH 7.2, 5mM EDTA, 50mM NaCl, Complete Mini Protease Inhibitor Cocktail Tablets (Roche #1-836-153, 1 tablet/10 ml lysis buffer), Phosphatase Inhibitor Cocktail 1 (Sigma #P2850, 1:100 dilution), Phosphatase Inhibitor Cocktail 2 (Sigma #P5726, 1:100 dilution)]. Cell lysates were normalized to protein and immunoprecipitated with an anti-p53 antibody (FL-393, Santa Cruz Biotechnology, Santa Cruz, CA). The immunoprecipitates were washed twice in lysis buffer, resuspended in electrophoresis sample buffer (Lammeli buffer as known to a person skilled in the art), and subjected to protein analyses on a 4-12% Bis-Tris polyacrylamide gel with 1X MOPS running buffer (Invitrogen, Carlsbad, CA). Proteins on the gel were transferred to nitrocellulose membranes. The membranes were blocked with 5% milk buffer (5% nonfat dry milk in TBS/0.1% Tween-20) and probed in the same buffer with either anti-p53 or anti-Hdm2 antibodies (DO-1, Oncogene Research Products, Boston, MA and SMP-14, Santa Cruz Biotechnology, Santa Cruz, CA, respectively). After scanning images generated using Typhoon Scanner (Molecular Dynamics, Sunnyvale, CA), ratios of Hdm2: p53 were determined by ImageQuant analysis, and was normalized to the vehicle control.

It was observed that compounds of the invention cause the dissociation of cellular Hdm2 from p53 in a dose-dependent fashion. At a concentration of 40 µM, two-fold greater then the IC₅₀ for inhibition of cell proliferation, TDP521252 caused greater then 90% dissociation of Hdm2 from p53. Similar results have been observed as early as 15 minutes after treating cells with compounds (data not shown). In contrast, the inactive diastereomer, TDP556356, failed to cause significant dissociation of Hdm2 from p53. Similar results were observed with the purified enantiomers of TDP222669, TDP258625 and TDP258626.

### EXAMPLE 120

### Benzodiazapinediones Induces Apoptosis in HepG2 cells

Activation of p53 in tumor cell lines can lead to either a cytostatic or apoptotic event depending upon the cellular environment (Slee et al., 2004, *Oncogene* 23, 2809-2818). Experiments were performed to determine whether Benzodiazapinediones would act as cytostatic agents and prevent further tumor growth or induce programmed cell death and potentially lead to tumor regression. Particularly, two parameters of programmed cell death, caspase activity and chromatin condensation, were examined in HepG2 cells.

Caspase Assay: HepG2 hepatocellular carcinoma cells were treated with 40 µM TDP521252, TDP536356 or DMSO for 0, 8, 16, 24 or 48 hours. Cells were lysed and exposed to substrates for caspases 3 and 7 (ApoOne, Promega, Madison WI) and specific processing was measured as described by manufacturer over 2 hours. Rates of caspase activity were then calculated using the differential results at 20 and 40 minutes after substrate addition.

Hoechst Staining: For assessing apoptotic nuclei, HepG2 cells were plated onto four-chamber SonicSeal slides (Nunc, Naperville, IL). After exposure to compound, cells were fixed with 3.7% formaldehyde for 15 minutes, rinsed in PBS and subsequently stained with 2.5 µg/ml Hoeschst dye for 15 minutes. After staining, cells were rinsed again and mounted using Prolong Antifade (Molecular Probes, Eugene, OR) and then were examined and photographed with an Olympus IX81 microscope (Melville, NY).

The rate of caspase-3 and caspase-7 activation in cells treated with the active enantiomer TDP521252 is substantially increased compared to that observed for cells treated with either DMSO or the inactive enantiomer TDP536356 (Fig. 2). The induction of caspases was observed as early as 16 hours after addition of TDP521252 and increased approximately 4.5-fold over the following 32 hours. In additional studies, asynchronous HepG2 cells were exposed to both the active and the inactive enantiomers of TDP521252 over a three-day period after which time cells were harvested, fixed and stained with Hoechst dye to detect chromatin. Exposure to the active enantiomer caused an accumulation of condensed chromatin as seen by punctate staining of the nuclei (data not shown). These data indicate that compounds of the invention activate an apoptotic response in HepG2 cells.

### EXAMPLE 121

### Benzodiazapinediones Increase Gene Expression of p53-Regulated Genes

Stabilization of p53 results in the up-regulation of a multitude of genes involved in diverse activities including regulation of cell cycle and apoptosis (see Levine, 1997, *Cell* 88, 323-331; and Slee, supra). Experiments were performed to examine the effect of Benzodiazapinediones on gene expression of the p53-regulated genes, such as genes of PIG-3, PUMA, Hdm2 and p21 ^{waf1/cip1}.

HepG2 hepatocellular carcinoma cells were seeded at 6 X 10⁵ cells/well in a 6-well plate overnight. The following morning, cells were treated with 20 µM TDP521252 or its inactive enantiomer TDP536356 for various times over an 8-hour period. Total RNA was isolated using the RNeasy kit (Qiagen, Valencia, CA) according to the manufacturer's protocol. RT-PCR was performed using the isolated total RNA as the initial template, and the Taqman One-Step RT-PCR Master Mix Reagent (Applied Biosystems, Foster City, CA). Fluorescently tagged primer/probe mixtures were purchased from Applied Biosystems (Foster City, CA) for human glyceraldehyde-3-phosphate dehydrogenase (GAPDH), Hdm2, PUMA (p53 upregulated modulator of apoptosis), p53, p21^{waf1/cip1} and PIG-3 (p53 inducible gene-3) and were designed to span an intron/exon boundary to discount genomic DNA contamination. All samples were normalized to GAPDH and were quantified using the standard curve method. Reactions were carried out in an ABI Prism 7000 (Applied Biosystems, Foster City, CA).

Early mRNA induction was observed for p21 ^{wafl/cipl} and Hdm2 peaking after 4-hours post-exposure to TDP521252, with increases in PUMAα and PIG-3 following at later time points (Figure 3). The time course of early and late induction of these genes is consistent with previously reported results of promoter occupancy (Kaeser, 2002, *Proc Natl Acad Sci U S A* 99, 95-100). In contrast, no induction of p21^{wafl/cipl} was observed following treatment of the cells with either DMSO or the inactive enantiomer, TDP536356. A similar profile was observed in JAR choriocarcinoma cells (data not shown).

Dose-dependent increases in the amount of the corresponding proteins were also observed (data not shown). At the highest dose of TDP521252 tested (40 µM), the greatest induction of protein relative to untreated controls was observed with PUMAα (84x) followed by p21 wafl/cipl(67x) and Hdm2 (45x). Furthermore, the level of p53 protein was approximately 3-fold higher after 24 hours in the presence of 40 µM TDP521252.

These data demonstrate that stabilization of p53 by TDP521252 leads to the increased expression of p53-regulated genes.

### EXAMPLE 122

### Benzodiazapinediones Induces Gene Expression of p21 ^{wafl/cipl} in vivo

To determine whether Benzodiazapinediones could induce p53 activation *in vivo,* a pharmacodynamic assay was performed to measure induction of p21^{waf1/cip1}mRNA from various tissues after treating mice with benzodiazepinediones. Doxorubicin has been previously shown to activate p53 and was therefore used as a positive control (Yeh et al., 2004, *Oncogene* 23, 3580-3588).

Female Balb/c nu/nu mice were administered TDP665759 (25 or 50 mg/kg) twice daily for a total of 7 doses intraperitoneally (12 hours apart) or doxorubicin (10 mg/kg) once intravenously 24 hours prior to sacrifice and tissue collection. In these experiments, the compound was well tolerated at the 25 mg/kg dose, however, the 50 mg/kg group experienced some body weight loss (16%) by the end of the four day study. Six hours after the final dose of the compound, the mice were sacrificed and livers were harvested and flash frozen on dry ice in tubes containing Rneasy. Total RNA was isolated as described above. The amount of p21 ^{wafl/cipl} mRNA in the isolated total RNA was measured by RT-PCR as described above.

The benzodiazepinediones and doxorubicin increased p21 ^{waf1/cip1} mRNA to the greatest extent in liver, with lesser but measurable induction in thymus and spleen, respectively (data not shown). The highest sensitivity of the liver to these agents is consistent with p21 ^{wafl/cipl} induced by gamma irradiation in wt p53 transgenic mice (Fei et al., 2002, *Cancer Res* 62, 7316-7327). Administration of 25 mg/kg of TDP665759 produced a 30-fold induction of p21^{wafl/cipl} relative to vehicle control; this was similar to doxorubicin treatment (Fig. 4). At the 50 mg/kg dose, induction was greater then 150-fold relative to vehicle control. The exponential-like effect of p21^{wafl/lipl} mRNA induction suggests the possibility of regulatory pathways or positive feedback loops and has been observed with other compounds in this chemical series (data not shown).

### EXAMPLE 123

### Combination of Benzodiazapinediones with Other Chemotherapy Resulted in Enhanced Antitumor Activity

This Example tested the effectiveness of a combined therapy involving a chemotherapeutic and a small molecule inhibitor of the Hdm2: p53 interaction.

A375 melanoma cells (ATCC CRL-1619, Manassas, VA) were seeded at 2000 cells per well in 96-well, white walled plates and allowed to adhere overnight. TDP665759 and chemotherapeutic agents were added at the same time in a fixed ratio format (Chou et al., 1984, *Adv Enzyme Regul* 22, 27-55). Cells were exposed to compound for 48 hours and viability of cells was determined using Cell-Titer-Glo (Promega, Madison, WI). Calculations of combination effects were performed according to the method of Chou and Talalay *(Trends in Pharm. Sciences,* 4:450-454, 1983) using CaluSyn software (Biosoft, Cambridge, Great Britain). Combination Indices of less than 0.9 are synergistic, between 0.9 and 1.1 are additive and greater than 1.1 are antagonistic.

It was found that TDP665759 synergized with doxorubicin, 5-fluorouracil and irinotecan (Table 3). In contrast, combination of TDP665759 and cisplatin resulted in an antagonistic effect.

Doxorubicin was further tested in combination with TDP665759 in an A375 xenograft model. The A375 xenograft study was performed at Piedmont Research Center, Inc. (Morrisveille, NC) in female nude mice (Harlan, Indianapolis, IN). After tumors reached 80-120 mg in size, mice were randomized into treatment groups and treatment with vehicle (20.25% hydroxypropyl-β-cyclodextran, bid x 10), 100 mg/kg TDP665759 (po, bid x 10), 1.5 or 3 mg/kg doxorubicin (Qd x 5) or a combination of 100 mg/kg TDP665759 (bid x 10) + 1.5 mg/kg doxorubicin (Qd x 5) was initiated. Mice were monitored daily for general health and tumor size was measured every third or fourth day. Mice were euthanized when tumor volume exceeded 2 grams.

As shown in Figure 5, 100 mg/kg TDP665759 or low dose of doxorubicin (1.5 mg/kg = 0.5 MTD) elicited only a modest effect on A375 tumor growth control as compared with that of a vehicle. However, combining these two agents resulted in tumor growth control similar to that resulting from treatment with the higher dose of doxorubicin (3 mg/kg = MTD). These data demonstrated the synergy between Doxorubicin and a small molecule inhibitor of the p53: hdm2 interaction in vivo.

Having now fully described this invention, it will be understood by those of ordinary skill in the art that the same can be performed within a wide and equivalent range of conditions, formulations and other parameters without affecting the scope of the invention or any embodiment thereof. All patents and publications cited herein are fully incorporated by reference herein in their entirety.

**Table 1. Benzodiazepinedione Inhibitors of The Hdm2:p53 Interaction**

| **Structure** | **Compound** | **FP IC**_{**50**} **(µM)** |
|---|---|---|
| 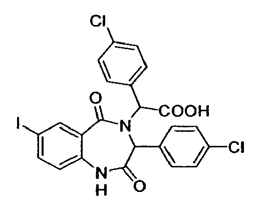 | **TDP222669 (S,S) (R,R) racemate** | **0.567±0.055** |
| | **TDP258625 (S,S) active enantiomer** | **0.28 + 0.029** |
| | **TDP258626 (R,R) inactive enantiomer** | **15.4 ± 0.796** |
| 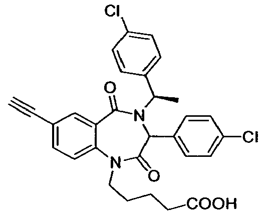 | **TDP521252 (3S) active enantiomer** | **0.708 + 0.002** |
| | **TDP536356 (3R) inactive enantiomer** | **28.5+2.55** |
| 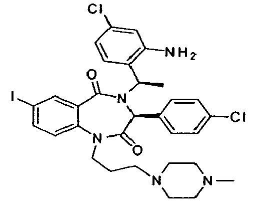 | **TDP665759** | **0.704 ± 0.06** |

**Table 2. Benzodiazepinediones Suppress Tumor Cell Proliferation**

| P53 within The Cell | Cell Name | TDP222669 | TDP521252 |
|---|---|---|---|
| Wild Type p53 | MCF7 | 33.3 + 17.5 | 10.0 + 2.0 |
| | JAR | 33.8 + 3.6 | 19.0 + 3.7 |
| | HepG2 | 45.5 + 10.8 | 11.0 + 3. |
| | A498 | 47.7 + 10.8 | 13.0 + 1.9 |
| | A375 | 46.5 + 12.0 | 12.0 + 3.5 |
| | Hct116 | 58.0 + 3.0 | 14.8 + 1.5 |
| | LNCaP | ND | 4.7 + 1.5 |
| | JEG3 | ND | 46.5 + 6.1 |
| | SJSA | ND | 13.8 + 2.6 |
| | ZR75-1 | ND | 19.0 + 6.0 |
| Mutant p53 | MDA MB 231 | 132.4 + 16.7 | 69.7 + 16.2 |
| | Panc1 | > 200 | > 200 |
| | SK-BR-3 | ND | 30.5 + 3.8 |
| | DU145 | ND | 66.5 + 22.4 |
| | MiaPaCa | > 200 | 141.7 + 7.6 |
| | A431 | > 200 | 123.8 + 37.9 |
| Null p53 | PC3 | ND | 41.5 + 9.3 |
| | SK-OV-3 | ND | 69.8 + 6.2 |
| | H1299 | 183.3 + 28.9 | 101.3 + 62.4 |

**Table 3. Combination Effects of TDP665759 with Various Chemotherapeutic Agents in A375 Melanoma Cells**

| **Chemotherapeutic Agent** | **Combination Index** |
|---|---|
| 5-Fluorouracil | 0.59 |
| Irinotecan | 0.63 |
| Doxorubicin | 0.62 |
| Cisplatin | 1.43 |

## Claims

1. A method of inhibiting the growth of a cell, comprising the steps of:
a. administering to the cell an antineoplastic agent; and
b. administering to the cell a small molecule that inhibits the binding between proteins MDM2 and p53.

2. The method of claim 1, wherein the step (a) comprising administering to the cell a compound selected from the group consisting of doxorubicin, 5-fluorouracil and irinotecan.

3. The method of claim 1, wherein the step (a) comprising administering to the cell doxorubicin.

4. The method of claim 1, wherein the step (b) comprising administering to the cell a compound of Formula (I). 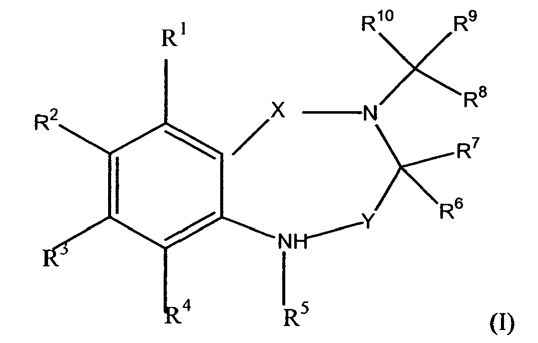 or a solvate, hydrate or pharmaceutically acceptable salt thereof; wherein:
X and Y are independently -C(O)-, -CH₂- or -C(S)-;
R¹, R², R³, and R⁴ are independently hydrogen, halo, alkyl, alkenyl, alkynyl, cycloalkyl, optionally substituted aryl, optionally substituted aralkyl, optionally substituted heteroaryl, optionally substituted heteroaralkyl, alkoxy, optionally substituted aryloxy, optionally substituted heteroaryloxy, cyano, amino, alkanoylamino, nitro, hydroxy, carboxy, or alkoxycarbonyl;
or R¹ and R¹, or R² and R³, or R³ and R⁴ are taken together to form - (CH₂)ᵤ-, where u is 3-6, -CH=CH-CH=CH- or -CH₂CH=CHCH₂-;
R⁵ is hydrogen, alkyl, cycloalkyl, optionally substituted aryl, optionally substituted heteroaryl, optionally substituted aralkyl, optionally substituted heteroaralkyl, carboxyalkyl, alkoxycarbonyl, alkoxycarbonylalkyl, aminocarbonyl, aminocarbonylalkyl, alkylaminocarbonyl or alkylaminocarbonylalkyl;
R⁶ is cycloalkyl, aryl, heteroaryl, cycloalkylalkyl, aralkyl, heteroarylalkyl, or a saturated or partially unsaturated heterocycle, each of which is optionally substituted;
R⁷ and R⁸ are independently hydrogen or alkyl;
R⁹ is cycloalkyl, aryl, heteroaryl, a saturated or partially unsaturated heterocycle, cycloalkyl(alkyl), aralkyl or heteroarylalkyl, each of which is optionally substituted; and
R¹⁰ is -(CH₂)ₙ-CO₂R^{b}, -(CH₂)ₘ-CO₂M, -(CH₂)ᵢ-OH or -(CH₂)ⱼ₋CONR^{c}R^{d}, where
R^{b} is hydrogen, alkyl, optionally substituted cycloalkyl, or optionally substituted, saturated or partially unsaturated heterocycle;
M is a cation;
R^{c} and R^{d} are independently hydrogen, alkyl, hydroxyalkyl, carboxyalkyl, aminoalkyl, optionally substituted cycloalkyl, optionally substituted aryl, optionally substituted aralkyl, optionally substituted heteroaryl, optionally substituted heteroarylalkyl, and an optionally substituted, saturated or partially unsaturated heterocycle; and
n is 0-8, m is 0-8, i is 1-8 and j is 0-8.

5. The method of claim 1, wherein the step (b) comprising administering to the cell a compound of Formula (II). 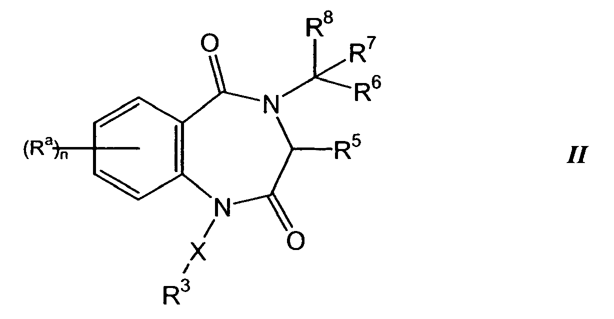 or a solvate, hydrate or pharmaceutically acceptable salt thereof; wherein:
each instance of R^{a} is independently halo, C₁₋₆ alkyl, C₂₋₆ alkenyl, C₂₋₆ alkynyl, cyano, C₃₋₈ cycloalkyl, hydroxy, C₁₋₆ alkoxy, carboxy, (C₁₋₆ alkoxy)carbonyl, C₁₋₆ acyl, carbamoyl, (C₁₋₆ alkyl)aminocarbonyl, alkylthio, amino or nitro;
n is 0; or n is 1 and R^{a} occurs at the 7- or 8-position; or n is 2 and R^{a} occurs at the 7- and 8-positions;
X is a bivalent radical of: a C₁₋₆ alkane, an optionally-substituted C₆₋₁₀ arene, an optionally-substituted 5- to 7-membered heteroarene wherein 1 or 2 ring atoms are heteroatoms, an optionally-substituted (C₆₋₁₀ aryl)C₁₋₆ alkane, or an optionally-substituted heteroaryl(C₁₋₆) alkane in which the heteroaryl portion contains 5 to 7 ring atoms and wherein 1 or 2 of the ring atoms are heteroatoms;
R³ is -CO₂R^{d} or -CO₂M, where R^{d} is hydrogen, C₁₋₆ alkyl or optionally-substituted C₃₋₈ cycloalkyl, and M is a cation;
R⁵ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁶ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁷ is hydrogen, C₁₋₆ alkyl, C₃₋₈ cycloalkyl or (C₃₋₈ cycloalkyl) alkyl; and
R⁸ is hydrogen or C₁₋₆ alkyl.

6. The method of claim 1, wherein the step (b) comprising administering to the cell a compound of Formula (III): 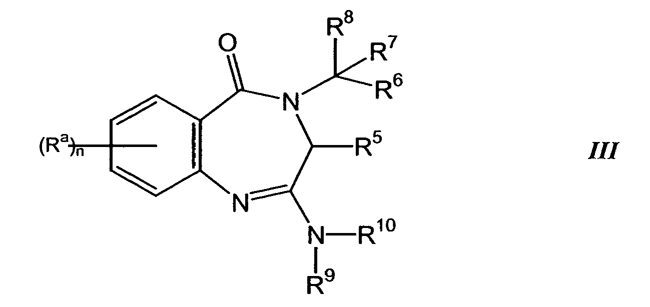 or a solvate, hydrate or pharmaceutically acceptable salt thereof; wherein:
each instance of R^{a} is independently halo, C₁₋₆ alkyl, C₂₋₆ alkenyl, C₂₋₆ alkynyl, cyano, C₃₋₈ cycloalkyl, hydroxy, C₁₋₆ alkoxy, carboxy, (C₁₋₆ alkoxy)carbonyl, C₁₋₆ acyl, carbamoyl, (C₁₋₆ alkyl)aminocarbonyl, amino, alkylthio or nitro;
n is 0; or n is 1 and R^{a} occurs at the 7- or 8-position; or n is 2 and R^{a} occurs at the 7- and 8-positions;
R⁵ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁶ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁷ is hydrogen, C₁₋₆ alkyl, C₃₋₈ cycloalkyl or (C₃₋₈ cycloalkyl)alkyl;
R⁸ is hydrogen or C₁₋₆ alkyl;
R⁹ is hydrogen, C₁₋₆ alkyl, C₃₋₆ cycloalkyl, hydroxy(C₁₋₆) alkyl, amino(C₁₋₆) alkyl, carboxy(C₁₋₆) alkyl, (C₁₋₆ alkoxy)carbonyl, (C₁₋₆ alkoxy)carbonyl(C₁₋₆) alkyl, carbamoyl, carbamoyl(C₁₋₆) alkyl, (C₁₋₆ alkylamino)carbonyl or (C₁₋₆ alkylamino)carbonyl(C₁₋₆) alkyl; and
R¹⁰ is hydrogen or C₁₋₆ alkyl.

7. The method of claim 1, wherein the step (b) comprising administering to the cell a compound selected from the group consisting of synthetic chalcones, norbornane derivatives, cis-imidazoline derivatives (Nutlins), a pyrazolidinedione sulfonamide, 1,4-benzodiazepine-2,5-diones, and tryptophan derivatives.

8. A method of inhibiting the growth of a cell, comprising the steps of:
a. exposing the cell to a radiation treatment; and
b. administering to the cell a small molecule that inhibits the binding between proteins MDM2 and p53.

9. The method of claim 8, wherein the step (b) comprising administering to the cell a compound of Formula (I). 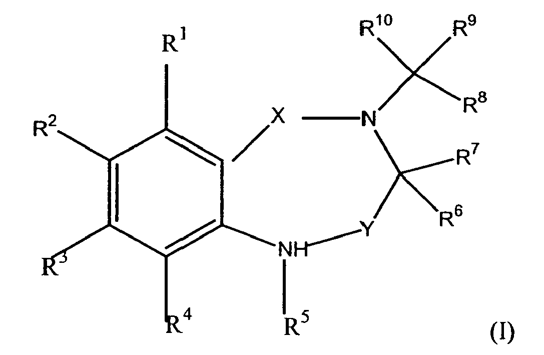 or a solvate, hydrate or pharmaceutically acceptable salt thereof; wherein:
X and Y are independently -C(O)-, -CH₂- or -C(S)-;
R¹, R², R³, and R⁴ are independently hydrogen, halo, alkyl, alkenyl, alkynyl, cycloalkyl, optionally substituted aryl, optionally substituted aralkyl, optionally substituted heteroaryl, optionally substituted heteroaralkyl, alkoxy, optionally substituted aryloxy, optionally substituted heteroaryloxy, cyano, amino, alkanoylamino, nitro, hydroxy, carboxy, or alkoxycarbonyl;
or R¹ and R², or R² and R³, or R³ and R⁴ are taken together to form - (CH₂)ᵤ-, where u is 3-6, -CH=CH-CH=CH- or -CH₂CH=CHCH₂-;
R⁵ is hydrogen, alkyl, cycloalkyl, optionally substituted aryl, optionally substituted heteroaryl, optionally substituted aralkyl, optionally substituted heteroaralkyl, carboxyalkyl, alkoxycarbonyl, alkoxycarbonylalkyl, aminocarbonyl, aminocarbonylalkyl, alkylaminocarbonyl or alkylaminocarbonylalkyl;
R⁶ is cycloalkyl, aryl, heteroaryl, cycloalkylalkyl, aralkyl, heteroarylalkyl, or a saturated or partially unsaturated heterocycle, each of which is optionally substituted;
R⁷ and R⁸ are independently hydrogen or alkyl;
R⁹ is cycloalkyl, aryl, heteroaryl, a saturated or partially unsaturated heterocycle, cycloalkyl(alkyl), aralkyl or heteroarylalkyl, each of which is optionally substituted; and
R¹⁰ is -(CH₂)ₙ-CO₂R^{b}, -(CH₂)ₘ-CO₂M, -(CH₂)ᵢ-OH or -(CH₂)ⱼ₋CONR^{c}R^{d}, where
R^{b} is hydrogen, alkyl, optionally substituted cycloalkyl, or optionally substituted, saturated or partially unsaturated heterocycle;
M is a cation;
R^{c} and R^{d} are independently hydrogen, alkyl, hydroxyalkyl, carboxyalkyl, aminoalkyl, optionally substituted cycloalkyl, optionally substituted aryl, optionally substituted aralkyl, optionally substituted heteroaryl, optionally substituted heteroarylalkyl, and an optionally substituted, saturated or partially unsaturated heterocycle; and
n is 0-8, m is 0-8, i is 1-8 and j is 0-8.

10. The method of claim 8, wherein the step (b) comprising administering to the cell a compound of Formula (II). 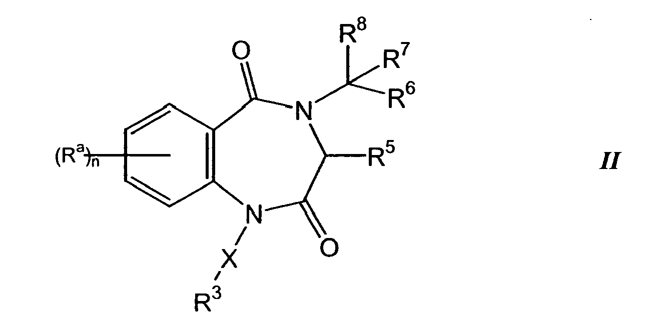 or a solvate, hydrate or pharmaceutically acceptable salt thereof; wherein:
each instance of R^{a} is independently halo, C₁₋₆ alkyl, C₂₋₆ alkenyl, C₂₋₆ alkynyl, cyano, C₃₋₈ cycloalkyl, hydroxy, C₁₋₆ alkoxy, carboxy, (C₁₋₆ alkoxy)carbonyl, C₁₋₆ acyl, carbamoyl, (C₁₋₆ alkyl)aminocarbonyl, alkylthio, amino or nitro;
n is 0; or n is 1 and R^{a} occurs at the 7- or 8-position; or n is 2 and R^{a} occurs at the 7- and 8-positions;
X is a bivalent radical of: a C₁₋₆ alkane, an optionally-substituted C₆₋₁₀ arene, an optionally-substituted 5- to 7-membered heteroarene wherein 1 or 2 ring atoms are heteroatoms, an optionally-substituted (C₆₋₁₀ aryl)C₁₋₆ alkane, or an optionally-substituted heteroaryl(C₁₋₆) alkane in which the heteroaryl portion contains 5 to 7 ring atoms and wherein 1 or 2 of the ring atoms are heteroatoms;
R³ is -CO₂R^{d} or -CO₂M, where R^{d} is hydrogen, C₁₋₆ alkyl or optionally-substituted C₃₋₈ cycloalkyl, and M is a cation;
R⁵ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁶ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁷ is hydrogen, C₁₋₆ alkyl, C₃₋₈ cycloalkyl or (C₃₋₈ cycloalkyl) alkyl; and
R⁸ is hydrogen or C₁₋₆ alkyl.

11. The method of claim 8, wherein the step (b) comprising administering to the cell a compound of Formula (III). 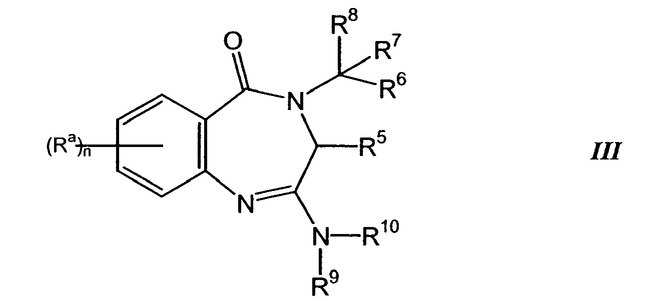 or a solvate, hydrate or pharmaceutically acceptable salt thereof; wherein:
each instance of R^{a} is independently halo, C₁₋₆ alkyl, C₂₋₆ alkenyl, C₂₋₆ alkynyl, cyano, C₃₋₈ cycloalkyl, hydroxy, C₁₋₆ alkoxy, carboxy, (C₁₋₆ alkoxy)carbonyl, C₁₋₆ acyl, carbamoyl, (C₁₋₆ alkyl)aminocarbonyl, amino, alkylthio or nitro;
n is 0; or n is 1 and R^{a} occurs at the 7- or 8-position; or n is 2 and R^{a} occurs at the 7- and 8-positions;
R⁵ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁶ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁷ is hydrogen, C₁₋₆ alkyl, C₃₋₈ cycloalkyl or (C₃₋₈ cycloalkyl)alkyl;
R⁸ is hydrogen or C₁₋₆ alkyl;
R⁹ is hydrogen, C₁₋₆ alkyl, C₃₋₆ cycloalkyl, hydroxy(C₁₋₆) alkyl, amino(C₁₋₆) alkyl, carboxy(C₁₋₆) alkyl, (C₁₋₆ alkoxy)carbonyl, (C₁₋₆ alkoxy)carbonyl(C₁₋₆) alkyl, carbamoyl, carbamoyl(C₁₋₆) alkyl, (C₁₋₆ alkylamino)carbonyl or (C₁₋₆ alkylamino)carbonyl(C₁₋₆) alkyl; and
R¹⁰ is hydrogen or C₁₋₆ alkyl.

12. The method of claim 8, wherein the step (b) comprising administering to the cell a compound selected from the group consisting of synthetic chalcones, norbornane derivatives, cis-imidazoline derivatives (Nutlins), a pyrazolidinedione sulfonamide, 1,4-benzodiazepine-2,5-diones, and tryptophan derivatives.

13. A method of monitoring the effect of a small molecule that inhibits the binding between proteins MDM2 and p53, comprising the steps of:
a. obtaining a biological sample from a subjected who has been administered the small molecule that inhibits the binding between proteins MDM2 and p53; and
b. measuring the level of gene expression of a gene whose transcription is regulated by p53.

14. The method of claim 13, wherein the small molecule that inhibits the binding between proteins MDM2 and p53 is a compound of Formula (I). 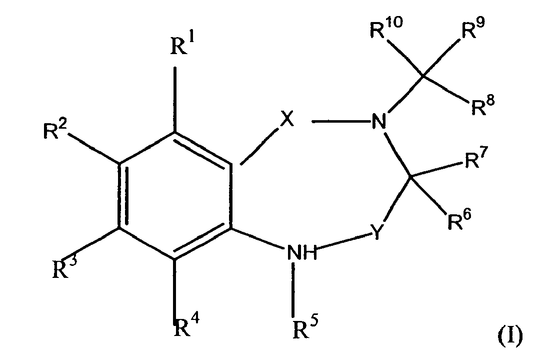 or a solvate, hydrate or pharmaceutically acceptable salt thereof; wherein:
X and Y are independently -C(O)-, -CH₂- or -C(S)-;
R¹, R², R³, and R⁴ are independently hydrogen, halo, alkyl, alkenyl, alkynyl, cycloalkyl, optionally substituted aryl, optionally substituted aralkyl, optionally substituted heteroaryl, optionally substituted heteroaralkyl, alkoxy, optionally substituted aryloxy, optionally substituted heteroaryloxy, cyano, amino, alkanoylamino, nitro, hydroxy, carboxy, or alkoxycarbonyl;
or R¹ and R², or R² and R,or 3 R³ and R⁴ are taken together to form - (CH₂)ᵤ-, where u is 3-6, -CH=CH-CH=CH- or -CH₂CH=CHCH₂-;
R⁵ is hydrogen, alkyl, cycloalkyl, optionally substituted aryl, optionally substituted heteroaryl, optionally substituted aralkyl, optionally substituted heteroaralkyl, carboxyalkyl, alkoxycarbonyl, alkoxycarbonylalkyl, aminocarbonyl, aminocarbonylalkyl, alkylaminocarbonyl or alkylaminocarbonylalkyl;
R⁶ is cycloalkyl, aryl, heteroaryl, cycloalkylalkyl, aralkyl, heteroarylalkyl, or a saturated or partially unsaturated heterocycle, each of which is optionally substituted;
R⁷ and R⁸ are independently hydrogen or alkyl;
R⁹ is cycloalkyl, aryl, heteroaryl, a saturated or partially unsaturated heterocycle, cycloalkyl(alkyl), aralkyl or heteroarylalkyl, each of which is optionally substituted; and
R¹⁰ is -(CH₂)ₙ-CO₂R^{b}, -(CH₂)ₘ-CO₂M, -(CH₂)ᵢ-OH or -(CH₂)ⱼ₋CONR^{c}R^{d}, where
R^{b} is hydrogen, alkyl, optionally substituted cycloalkyl, or optionally substituted, saturated or partially unsaturated heterocycle;
M is a cation;
R^{c} and R^{d} are independently hydrogen, alkyl, hydroxyalkyl, carboxyalkyl, aminoalkyl, optionally substituted cycloalkyl, optionally substituted aryl, optionally substituted aralkyl, optionally substituted heteroaryl, optionally substituted heteroarylalkyl, and an optionally substituted, saturated or partially unsaturated heterocycle; and
n is 0-8, m is 0-8, i is 1-8 and j is 0-8.

15. The method of claim 13, wherein the small molecule that inhibits the binding between proteins MDM2 and p53 is a compound of Formula (II), 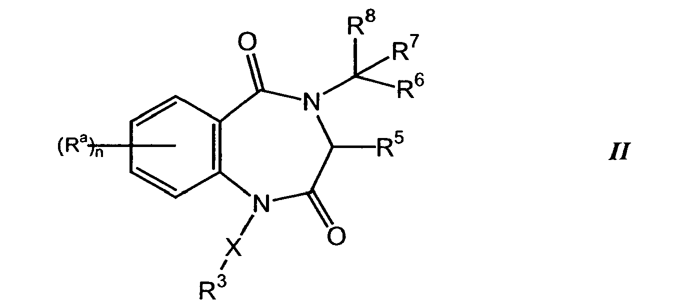 or a solvate, hydrate or pharmaceutically acceptable salt thereof; wherein:
each instance of R^{a} is independently halo, C₁₋₆ alkyl, C₂₋₆ alkenyl, C₂₋₆ alkynyl, cyano, C₃₋₈ cycloalkyl, hydroxy, C₁₋₆ alkoxy, carboxy, (C₁₋₆ alkoxy)carbonyl, C₁₋₆ acyl, carbamoyl, (C₁₋₆ alkyl)aminocarbonyl, alkylthio, amino or nitro;
n is 0; or n is 1 and R^{a} occurs at the 7- or 8-position; or n is 2 and R^{a} occurs at the 7- and 8-positions;
X is a bivalent radical of: a C₁₋₆ alkane, an optionally-substituted C₆₋₁₀ arene, an optionally-substituted 5- to 7-membered heteroarene wherein 1 or 2 ring atoms are heteroatoms, an optionally-substituted (C₆₋₁₀ aryl)C₁₋₆ alkane, or an optionally-substituted heteroaryl(C₁₋₆) alkane in which the heteroaryl portion contains 5 to 7 ring atoms and wherein 1 or 2 of the ring atoms are heteroatoms;
R³ is -CO₂R^{d} or -CO₂M, where R^{d} is hydrogen, C₁₋₆ alkyl or optionally-substituted C₃₋₈ cycloalkyl, and M is a cation;
R⁵ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁶ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁷ is hydrogen, C₁₋₆ alkyl, C₃₋₈ cycloalkyl or (C₃₋₈ cycloalkyl) alkyl; and
R⁸ is hydrogen or C₁₋₆ alkyl.

16. The method of claim 13, wherein the small molecule that inhibits the binding between proteins MDM2 and p53 is a compound of Formula (III): 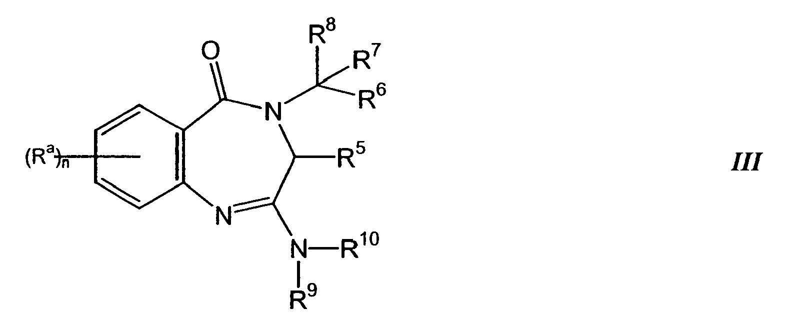 or a solvate, hydrate or pharmaceutically acceptable salt thereof; wherein:
each instance of R^{a} is independently halo, C₁₋₆ alkyl, C₂₋₆ alkenyl, C₂₋₆ alkynyl, cyano, C₃₋₈ cycloalkyl, hydroxy, C₁₋₆ alkoxy, carboxy, (C₁₋₆ alkoxy)carbonyl, C₁₋₆ acyl, carbamoyl, (C₁₋₆ alkyl)aminocarbonyl, amino, alkylthio or nitro;
n is 0; or n is 1 and R^{a} occurs at the 7- or 8-position; or n is 2 and R^{a} occurs at the 7- and 8-positions;
R⁵ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁶ is C₃₋₈ cycloalkyl, C₆₋₁₀ aryl, 5- to 7-membered heteroaryl wherein 1 or 2 of the ring atoms are heteroatoms, (C₃₋₈ cycloalkyl)alkyl, (C₆₋₁₀ aryl)alkyl, (heteroaryl)alkyl in which the heteroaryl portion contains 5 to 7 ring atoms and wherein 1 or 2 of the ring atoms are heteroatoms, or 5- to 7-membered saturated or partially unsaturated heterocycle wherein 1 or 2 of the ring atoms are heteroatoms, in which each of the preceding groups is optionally substituted;
R⁷ is hydrogen, C₁₋₆ alkyl, C₃₋₈ cycloalkyl or (C₃₋₈ cycloalkyl)alkyl;
R⁸ is hydrogen or C₁₋₆ alkyl;
R⁹ is hydrogen, C₁₋₆ alkyl, C₃₋₆ cycloalkyl, hydroxy(C₁₋₆) alkyl, amino(C₁₋₆) alkyl, carboxy(C₁₋₆) alkyl, (C₁₋₆ alkoxy)carbonyl, (C₁₋₆ alkoxy)carbonyl(C₁₋₆) alkyl, carbamoyl, carbamoyl(C₁₋₆) alkyl, (C₁₋₆ alkylamino)carbonyl or (C₁₋₆ alkylamino)carbonyl(C₁₋₆) alkyl; and
R¹⁰ is hydrogen or C₁₋₆ alkyl.

17. The method of claim 13, wherein the step (b) comprising measuring the level of gene expression of the gene p21^{wafl/cipl}.

18. A product containing an antineoplastic agent and a small molecule that inhibits the binding between proteins MDM2 and p53, as a combined preparation for simultaneous, separate or sequential use for inhibiting the growth of a cell.

19. The use of an antineoplastic agent and a small molecule that inhibits the binding between proteins MDM2 and p53 for the manufacture of a medicament for inhibiting the growth of a cell.

20. The product of claim 18 or use of claim 19 wherein the antineoplastic agent is selected from the group consisting of doxorubicin, 5-fluorouracil and irinotecan.

21. The product of claim 18 or use of claim 19 wherein the antineoplastic agent is doxorubicin.

22. The use of a small molecule that inhibits the binding between proteins MDM2 and p53 for the manufacture of a medicament for inhibiting the growth of a cell, wherein the cell is exposed to a radiation treatment.

23. The product of any of claims 18, 20 or 21 or use of any of claims 19-22 wherein the small molecule is of formula (I) as defined in claim 4.

24. The product of any of claims 18, 20 or 21 or use of any of claims 19-22 wherein the small molecule is of formula (II) as defined in claim 5.

25. The product of any of claims 18, 20 or 21 or use of any of claims 19-22 wherein the small molecule is of formula (III) as defined in claim 6.

26. A compound of formula (I) as defined in claim 4 for use in therapy.

27. A compound of formula (II) as defined in claim 5 for use in therapy.

28. A compound of formula (III) as defined in claim 6 for use in therapy.

29. The product of any of claims 18, 20, 21 or 23-25, use of any of claims 19-25, or compound of any of claims 26-29 for the treatment of cancer.
